# EUROPEAN PATENT APPLICATION

(11) **EP 3 778 892 A1**
(43) Date of publication of application: **17.02.2021**
(21) Application number: 19784428.5
(22) Date of filing: 10.04.2019
(51) Int. Cl.: C12N 15/113, A61K 31/713, A61P 35/00

(54) **NOVEL SMALL ACTIVATING RNA**

(30) Priority: 10.04.2018 CN 201810317366
(71) Applicant: Ractigen Therapeutics, Nantong, Jiangsu 226400 (CN)
(72) Inventor: LI, Longcheng, Nantong, Jiangsu 226400 (CN); KANG, Moorim, Nantong, Jiangsu 226400 (CN); PLACE, Robert F., Nantong, Jiangsu 226400 (CN); WU, Jiancheng, Nantong, Jiangsu 226400 (CN)
(74) Representative: Høiberg P/S
(86) International application number: PCT/CN2019/082149
(87) International publication number: WO 2019/196887

(57) **Abstract**

saRNAs are provided in the present invention. The saRNAs are composed of a first oligonucleotide strand containing 17 to 30 nucleotides and a second oligonucleotide strand containing 17 to 30 nucleotides. Sequences of at least 15 nucleotides in length are complementary in the two oligonucleotide strands, and the unpaired terminal nucleotides form overhangs. The first oligonucleotide strand or the second oligonucleotide strand has more than 75% homology or complementarity with any continuous fragment of 16 to 35 nucleotides in length in the promoter of the target gene. The second oligonucleotide strand has an overhang composed of 1 to 4 nucleotides at 3' end. saRNAs of the present invention can upregulate target gene expression more effectively while reducing off-target effects.

## Description

### FIELD OF THE INVENTION

The present invention relates to the field of molecular biology, and in particular to up-regulating gene expression with a double-stranded small RNA targeting a gene promoter.

### BACKGROUND OF THE INVENTION

Double-stranded small nucleic acid molecules including chemically synthesized oligoribonucleotides (such as small activating RNA (saRNA)) and naturally occurring oligoribonucleotides (such as micro ribonucleic acid (miRNA)) have been proved to be capable of targeting regulatory sequences (such as promoter sequences) of protein-coding genes in a sequence-specific manner to up-regulate gene expression at the transcriptional and epigenetic level, a phenomenon known as RNA activation (RNAa) (Li, Okino et al. (2006) Proc Natl Acad Sci U S A 103:17337-17342; Janowski, Younger et al. (2007) Nat Chem Biol 3:166-173; Place, Li et al. (2008) Proc Natl Acad Sci U S A 105:1608-1613; Huang, Place et al. (2012) Nucleic Acids Res 40:1695-1707; Li (2017) Adv Exp Med Biol 983:1-20). Studies have shown that RNAa is an endogenous molecular mechanism evolutionarily conserved from caenorhabditis elegans to the human being (Huang, Qin et al. (2010) PLoS One 5:e8848; Seth, Shirayama et al. (2013) Dev Cell 27:656-663; Turner, Jiao et al. (2014) Cell Cycle 13:772-781).

Safe strategies to selectively enhance gene and/or protein production remain a challenge in gene therapy. Viral-based systems have inherent drawbacks including adverse effects on host genome integrity and immunological consequences. RNAa, with the advantages of being able to activate endogenous genes without the risk of altering the genome, represents a new strategy to stimulate target gene expression.

Cyclin-dependent kinase (CDK) inhibitor p21WAF1/CIP1 (p21) is a mediator of several anti-growth pathways and considered a potent tumor suppressor gene in cancer cells (Harper, Adami et al. (1993) Cell 75:805-816). In fact, overexpression of p21 by ectopic vectors or stimulation of endogenous transcription inhibits tumor growth both *in vitro* and *in vivo* (Harper, Adami et al. (1993) Cell 75:805-816; Eastham, Hall et al. (1995) Cancer Res 55:5151-5155; Wu, Bellas et al. (1998) J Exp Med 187:1671-1679; Harrington, Spitzweg et al. (2001) J Urol 166:1220-1233). As such, selective activation of p21 can possess therapeutic application for regulating cell growth and treatment of disease (e.g. cancer).

### SUMMARY OF THE INVENTION

The present invention provides a type of oligonucleotide molecules and a method that can upregulate gene expression in a cell in a targeted manner. By introducing into cells, the oligonucleotide molecule targeting the promoter sequence of a gene, the method can effectively upregulate the expression of the targeted gene and produce an effective biological effect. The oligonucleotides of the present invention are structurally-optimized double-stranded ribonucleic acid molecules, which is also known as small activating RNA (saRNA).

The present invention provides a saRNA, comprising a first oligonucleotide strand of 17 to 30 nucleotides in length and a second oligonucleotide strand of 17 to 30 nucleotides in length, and a region of at least 15 nucleotides in length in the first oligonucleotide strand that is complementary to the second oligonucleotide strand. The first oligonucleotide strand or the second oligonucleotide strand has more than 75% (such as more than 80%, more than 85%, more than 90%, more than 95%, more than 99%, or 100%) sequence homology or complementarity with any continuous fragment of 15 to 30 nucleotides in length in the promoter of a target gene. One end of a duplex formed by the first oligonucleotide strand and the second oligonucleotide strand is a blunt end, i.e. having no overhang structure, and the other end of the duplex can have an overhang consisting of 1 to 4 nucleotides (such as 1, 2, 3 or 4 nucleotides) formed at the terminus of the first oligonucleotide strand or the second oligonucleotide strand. In a specific embodiment, one end of the duplex formed by the first oligonucleotide strand and the second oligonucleotide strand in the aforementioned saRNA is a blunt end, and the other end can have an overhang of 2 or 3 nucleotides formed at the terminus of the first oligonucleotide strand or the second oligonucleotide strand.

In the aforementioned saRNA, nucleotides of the overhang can be selected from T (thymine), U (uracil), or a "natural" nucleotide, for example, the overhang is selected from dTdTdT, dTdT, UUU, UU, or 2 or 3 continuous natural nucleotides (such as A, T, G and C). The natural nucleotide overhang in the present invention means that nucleotides overhanging at the terminus of the first oligonucleotide strand or the second oligonucleotide strand are homologous to or complementary with nucleotides at the corresponding position in a target sequence.

In the aforementioned saRNA, the 5' terminus of the first oligonucleotide strand or the second oligonucleotide strand is at the blunt end of the duplex, and there are 1 to 3 nucleotides of the first to the third nucleotide from the 5' terminus in the first or second oligonucleotide strand mispaired with nucleotides at the corresponding position in the other strand. Preferably, the mispairing involves mispaired cytosines. There are at least two patterns for the cytosine mispairing described herein. In one pattern, the nucleotides on one strand are cytosine nucleotides (C), while the guanine nucleotides (G) to be paired with the cytosine nucleotides by Watson-Crick base-pairing are replaced by non-guanine nucleotides (such as A, T or U). In the other pattern, the nucleotides on one strand are non-guanine nucleotides, such as A, U, or T, and the original nucleotides on the other strand to be paired with A, U, or T by Watson-Crick base-pairing are replaced with cytosine nucleotides (C).

In the saRNA provided in the invention, the length of the duplex formed by the first oligonucleotide strand and the second oligonucleotide strand, excluding the overhang, is 17 to 24 nucleotides, such as 17, 18, 19, 20, 21, 22, 23, or 24 nucleotides; and preferably, the length of the duplex formed by the first oligonucleotide strand and the second oligonucleotide strand, excluding the overhang, is 18 to 20 nucleotides.

The present invention further provides the use of the aforementioned saRNA in the preparation of a formulation, such as a drug, for activating or upregulating the expression of a target gene in a cell. The saRNA can be introduced into the cell directly. The cell can be a mammal cell, and preferably a human cell. The human cell can be present in a human body, or can be cultured *in vitro,* such as an isolated cell line, a cell strain, or a primary cell.

The human body can suffer from a disease caused by the defect and/or deficiency of expression of a target gene. Administration of an effective amount of an saRNA of the invention can, for example, treat the disease.

The target gene for the saRNA, in one case, is human p21. In other cases, the target gene can be other pathogenic genes resulting in diseases due to a gene expression defect and/or deficiency, such as KLF4, NKX3-1, and VEGFA.

If the target gene is p21 or one of the other genes, the saRNA can activate or upregulate the expression of the target gene by at least 10%, such as more than 10%, more than 20%, more than 30%, more than 40%, more than 50%, more than 60%, more than 70%, more than 80%, more than 90%, or more than 100%.

The present invention also provides a composition containing the aforementioned saRNA and a pharmaceutically acceptable carrier. The pharmaceutically acceptable carrier is a liposome, a macromolecular polymer, or a polypeptide.

The present invention further provides the use of the saRNA or the composition in the preparation of a formulation for activating or upregulating the expression of the target gene. Preferably, the use of the saRNA or the composition in the preparation of a formulation is for treating a tumor or a benign proliferative lesion. Preferably, the tumor is a bladder cancer, a prostate cancer, a hepatocellular carcinoma, or a colorectal cancer.

### BRIEF DESCRIPTION OF THE DRAWINGS

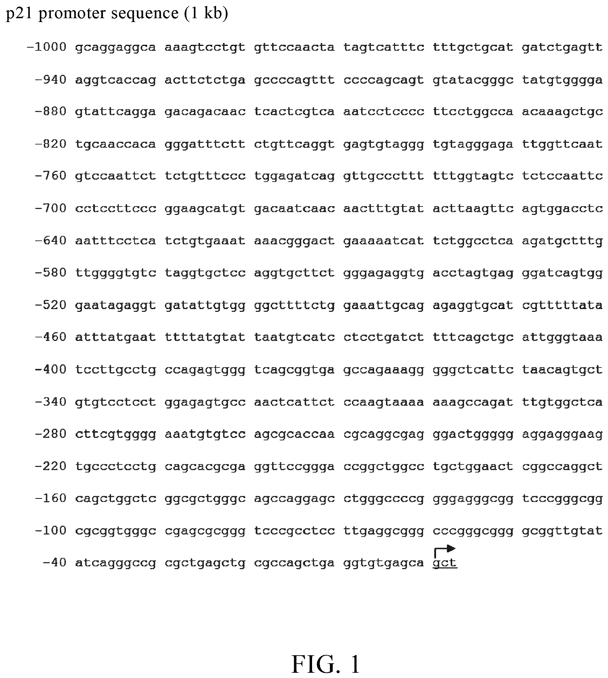

Fig. 1 shows the sequence of the p21 gene promoter (SEQ ID NO: 601), from -1000 bp upstream of the transcription start site (TSS) to +3 bp downstream of the TSS. The TSS is denoted by a curved arrow.

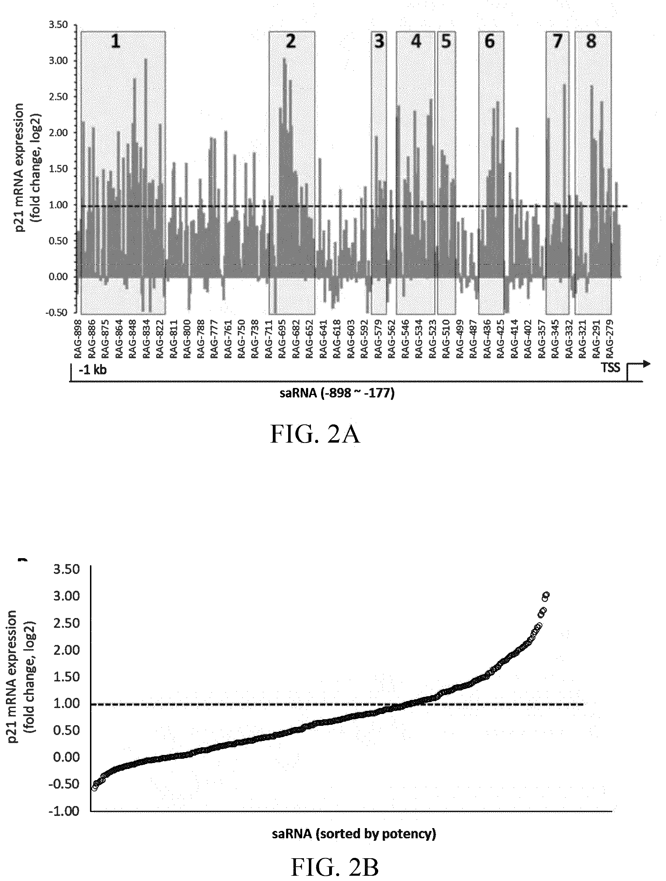

Figs. 2A and 2B show saRNA hotspot regions in the p21 promoter as revealed by screening. Four hundred and thirty-nine (439) saRNAs were designed on the sequence of the p21 promoter shown in Fig. 1 and chemically synthesized. The resultant saRNAs were individually transfected into PC3 human prostate cancer cells. 72 hours after transfection, the mRNA levels of p21 were determined by using the QuantiGene 2.0 assay. FIG. 2A shows the fold change (Y axis) of p21 mRNA levels caused by each of the 439 saRNAs (X axis) relative to the control treatment (Mock). The saRNAs on the X axis are sorted according to their positions relative to the TSS of p21 (from the most upstream RAG-898 to the most downstream RAG1-177). Eight hotspot regions are identified and shown (grayish rectangular boxes). FIG. 2B shows the data of FIG. 2A by sorting the saRNAs by their activity in activating p21 mRNA expression in an ascending order. The dotted lines in FIG. 2A and FIG. 2B represent the position of a two-fold induction.

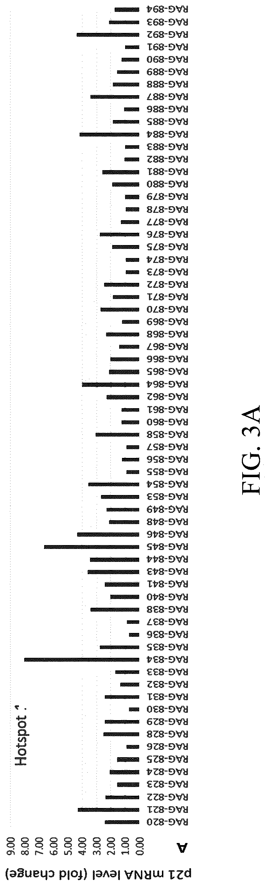

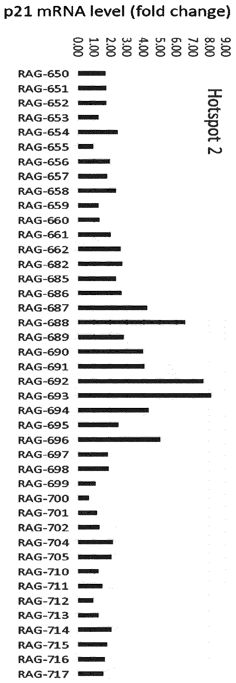

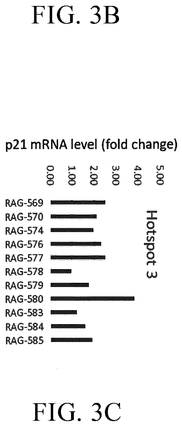

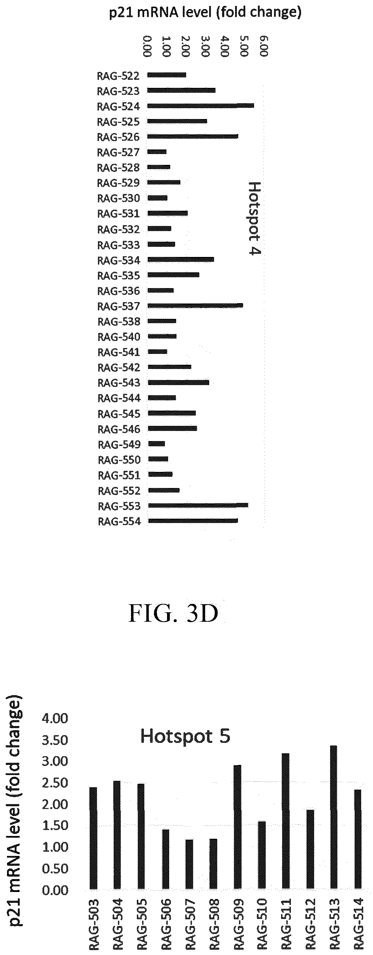

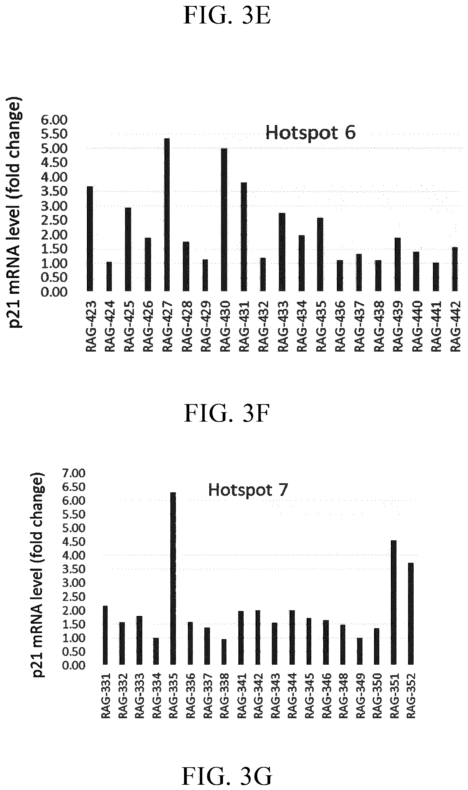

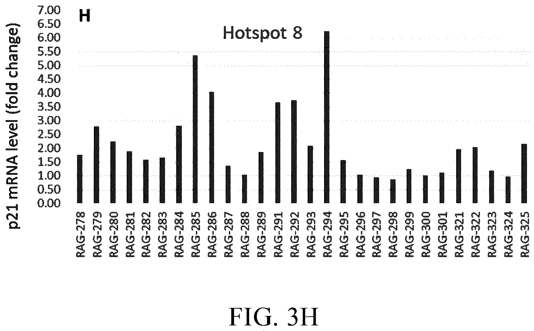

Figs. 3A-3H show the activating effects of the saRNAs targeting the hotspot regions 1 to 8 on the p21 promoter (FIG. 3A: hotspot region 1; FIG. 3B: hotspot region 2; FIG. 3C: hotspot region 3; FIG. 3D: hotspot region 4; FIG. 3E: hotspot region 5; FIG. 3F: hotspot region 6; FIG. 3G: hotspot region 7; FIG. 3H: hotspot region 8).

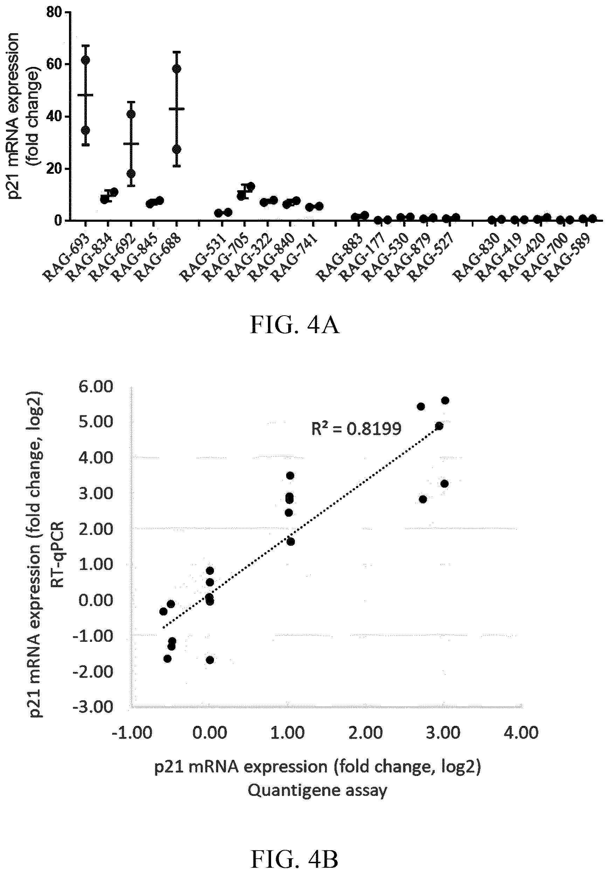

Figs. 4A and 4B show the mRNA levels of p21 assessed by RT-qPCR for verifying the result obtained from the QuantiGene 2.0 assay. FIG. 4A shows the mRNA level of p21 determined by RT-qPCR. The 439 saRNAs were divided into four bins according to their activities in inducing p21 mRNA expression from the highest to the lowest, and 5 saRNAs were randomly selected from each bin and individually transfected into PC3 cells at a 10 nM concentration. 72 hours after transfection, mRNA was extracted for reverse transcription to obtain cDNA, and then the mRNA level of p21 was determined by RT-qPCR. FIG. 4B shows the correlation of relative p21 mRNA level induced by the saRNAs determined by QuantiGene 2.0 (X axis) and by RT-qPCR (Y axis).

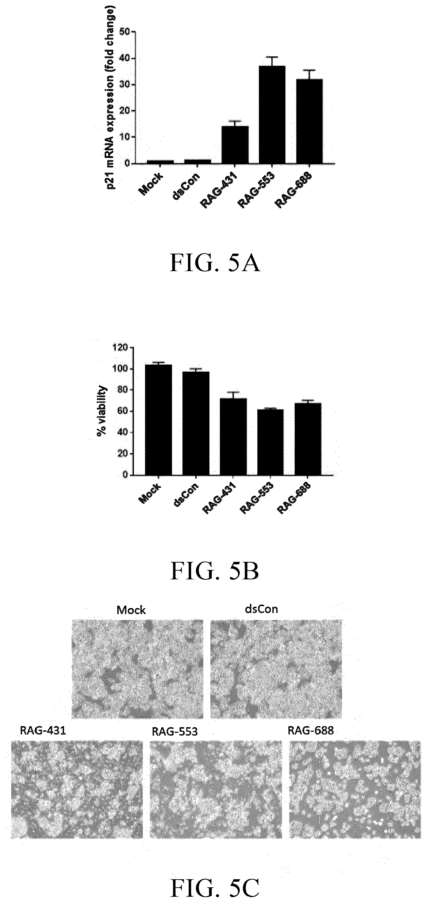

Figs. 5A-5C show the effect of saRNAs in inducing p21 mRNA expression and suppressing the proliferation of KU-7-Luc2-GFP cells. KU-7-Luc2-GFP cells were transfected with each of three saRNAs (RAG-431, RAG-553 and RAG-688) at a concentration of 10 nM for 72 hours. Mock samples were transfected in the absence of an oligonucleotide. Non-specific duplex (dsCon) served as a negative control. FIG. 5A shows the mRNA expression levels of p21 determined by RT-qPCR. FIG. 5B shows viability of saRNA treated cells as evaluated by the CCK-8 assay and plotted as percentages relative to the cell viability for control treatment (Mock). FIG. 5C shows representative cell images (100 ×) at the end of transfection.

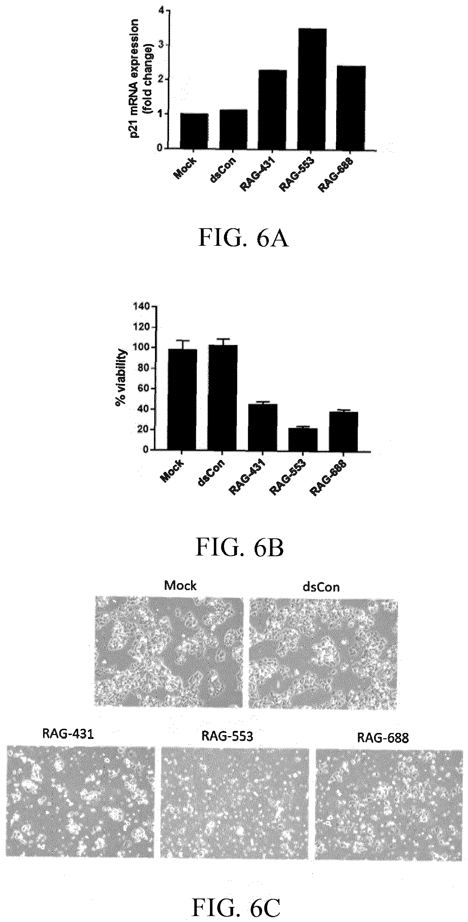

Figs. 6A-6C show the effect of the saRNAs in inducing p21 mRNA expression and suppressing the proliferation of HCT116 cells. HCT116 cells were transfected with each of three saRNAs (RAG-431, RAG-553 and RAG-688) at a concentration of 10 nM for 72 hours. FIG. 6A shows the mRNA expression levels of p21 determined by RT-qPCR. FIG. 6B shows the cell viability evaluated by the CCK-8 assay, and cell viability of saRNA-treated cells is plotted as percentages relative to the cell viability of the control treatment (Mock). FIG. 6C shows representative phase contrast cell images (100 ×) at the end of transfection.

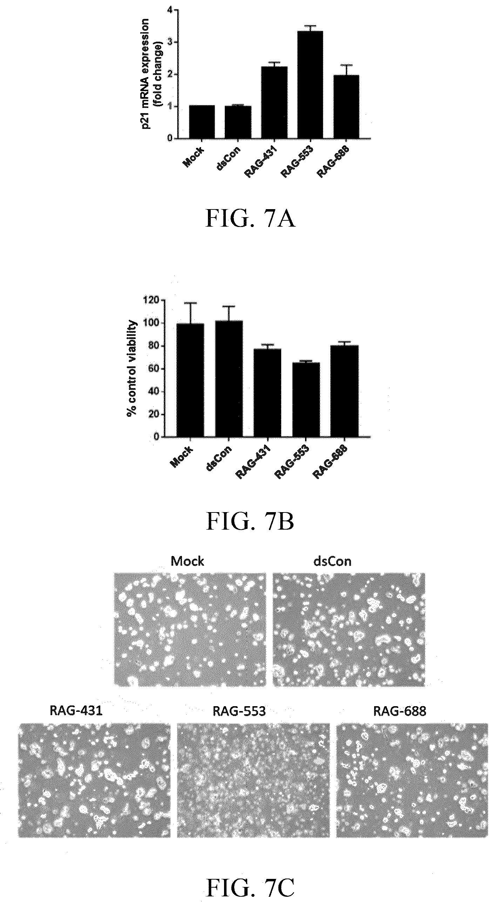

Figs. 7A-7C show the effect of the saRNAs in inducing p21 mRNA expression and suppressing the proliferation of HepG2 cells. HepG2 cells were transfected with each of three saRNAs (RAG-431, RAG-553 and RAG-688) at a concentration of 10 nM for 72 hours. FIG. 7A shows the mRNA expression levels of p21 determined by RT-qPCR. FIG. 7B shows the cell viability evaluated by the CCK-8 assay, and cell viability of saRNA-treated cells is plotted as percentages relative to the cell viability of the control treatment (Mock). FIG. 7C shows representative phase contrast cell images (100 ×) at the end of transfection.

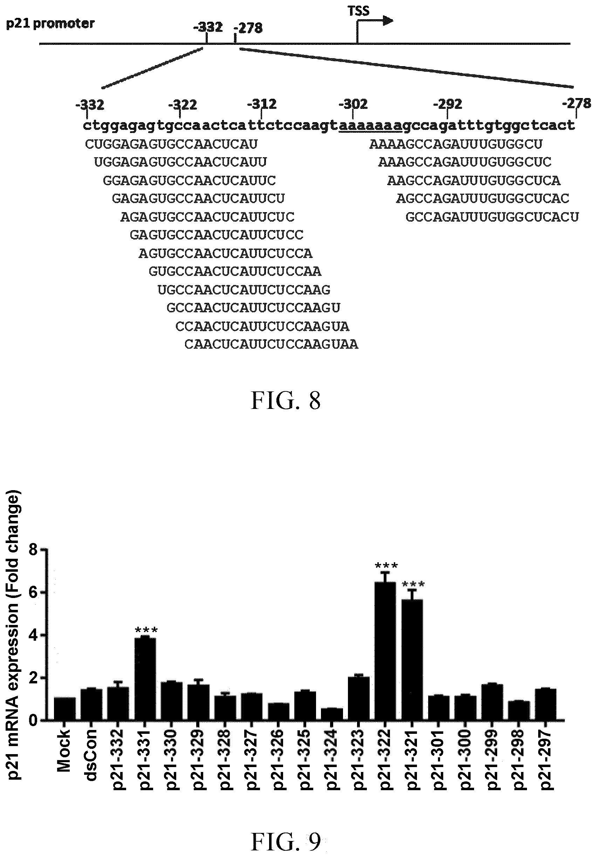

Fig. 8 is a schematic diagram illustrating a p21 promoter region and a target sequence for saRNAs therein. A 40 bp region spanning nucleotides -332 to -292 relative to the TSS in the p21 promoter was targeted by a series of saRNA duplexes for gene activation. The sequences shown correspond to the sites targeted by their cognate duplexes. Note the defined promoter region contains 17 overlapping targeted sites (SEQ ID NOs: 584-600) that shift by a single nucleotide each time skipping a short stretch of a polyadenosine repeat (underlined).
Fig. 9 shows the gene induction activities of saRNAs targeting the p21 promoter. KU-7-luc2-GFP cells were transfected at 10 nM concentrations of the indicated saRNAs for 72 hours. Mock samples were transfected in the absence of an oligonucleotide. Non-specific duplex (dsCon) served as a negative control. Relative expression was determined by RT-qPCR (mean ± SD). Expression levels of p21 were normalized to that of GAPDH. Statistical significance was determined by one-way ANOVA and Tukey's multiple comparisons test (^{∗∗∗} p < 0.001 versus mock).

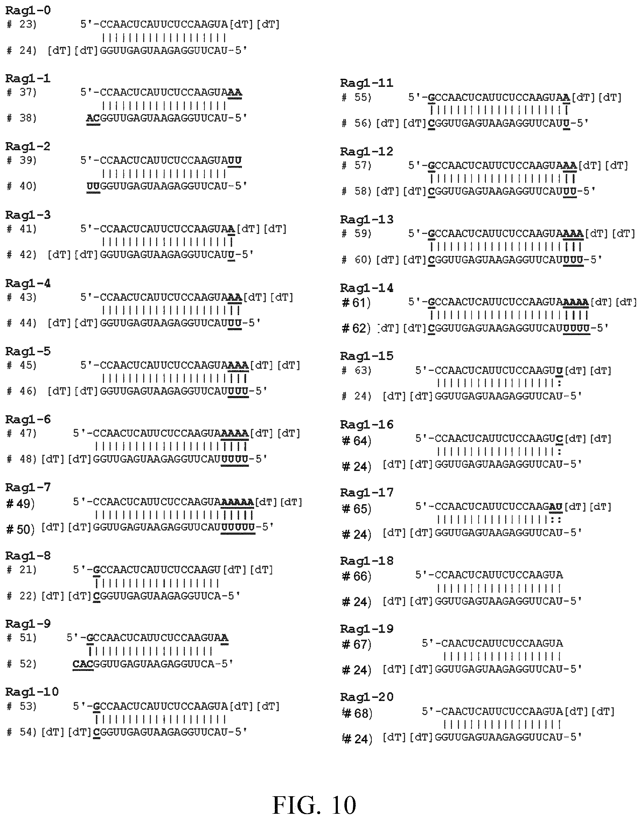

Fig. 10 is a schematic diagram showing sequences and structures of saRNA variants (from Rag1-0 to Rag1-20) (SEQ ID NOs:21-24,37-68). Mispaired bases are represented by colons. Boldfaces represent mutated nucleotides in saRNA variants compared to the original Rag1-0.

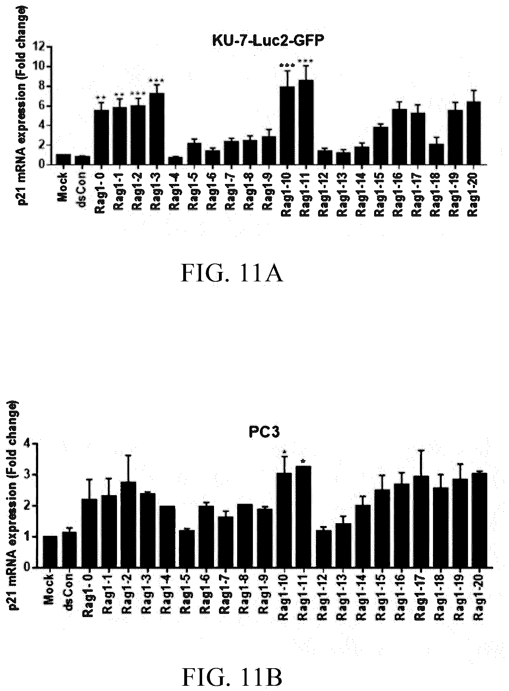

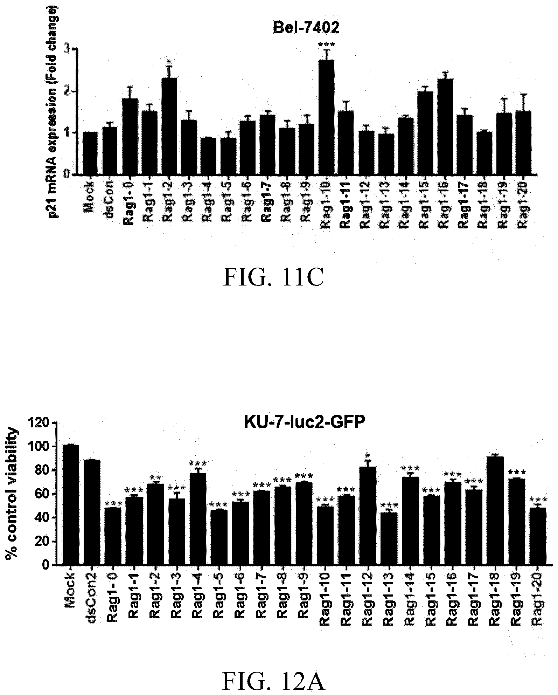

Figs. 11A-11C show the activity of the Rag1 variants (Rag1-0 to Rag1-20) in inducing p21 expression in human cancer cell lines. KU-7-Luc2-GFP (FIG. 11A), PC3 (FIG. 11B), and Bel-7402 (FIG. 11C) cells were transfected with 10 nM of the indicated Rag1 duplex variants for 72 hours. Mock samples were transfected in the absence of an oligonucleotide. Non-specific duplex (dsCon) served as a negative control. Relative p21 mRNA expression was determined by RT-qPCR (mean ± SD). Expression levels of p21 were normalized to that of GAPDH. Statistical significance was determined by one-way ANOVA and Tukey's multiple comparisons test (^{∗}P<0.5; ^{∗∗}P<0.01; ^{∗∗∗} p < 0.001 versus Mock).
Figs. 12A-12C show the suppressing effect of the saRNAs (Rag1-0 to Rag1-20) on the proliferation of human cancer cell lines. Cell viabilities were quantitatively assayed by the CCK-8 method. Statistical significance was determined by one-way ANOVA and Tukey's multiple comparisons test (^{∗}P<0.5; ^{∗∗}P<0.01; ^{∗∗∗} p < 0.001 versus Mock).

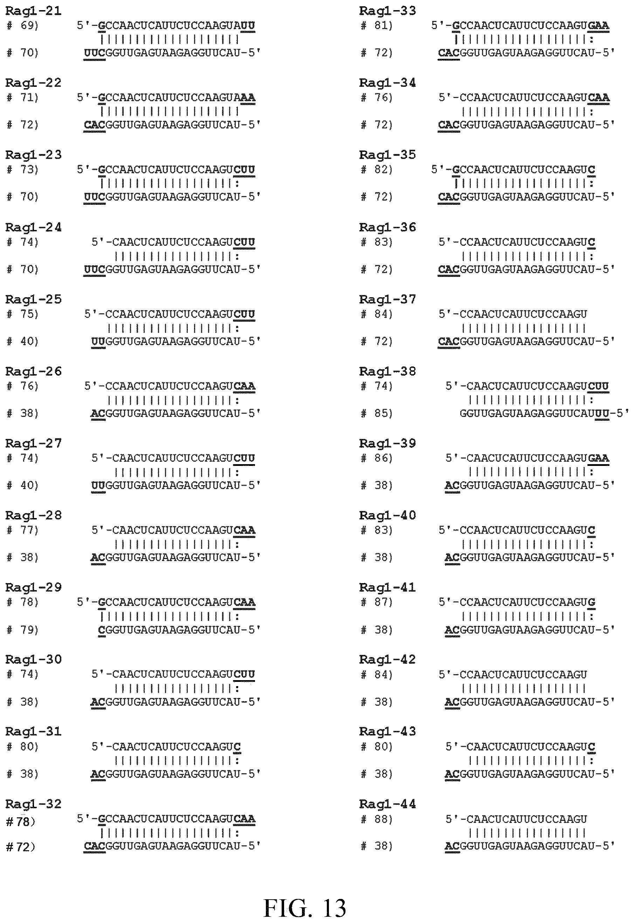

Fig. 13 is a schematic diagram for the sequences and structures of duplex saRNAs Rag1-21 to Rag1-44 (SEQ ID NOs:38, 40, 69-88). Mispaired bases in duplexes are represented by colons, and boldfaces represent altered nucleotides or structure in the variants.

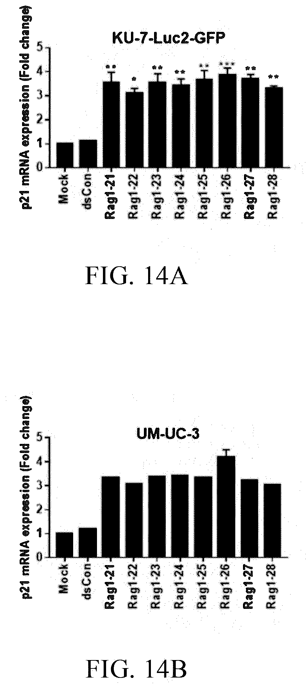

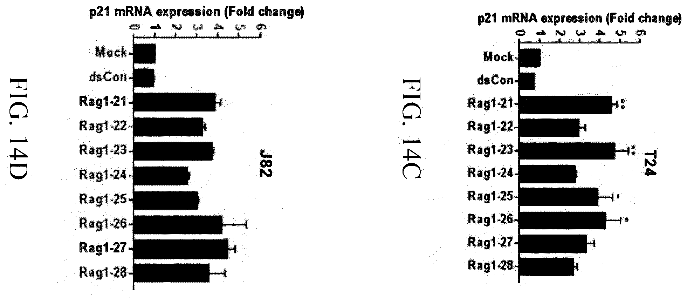

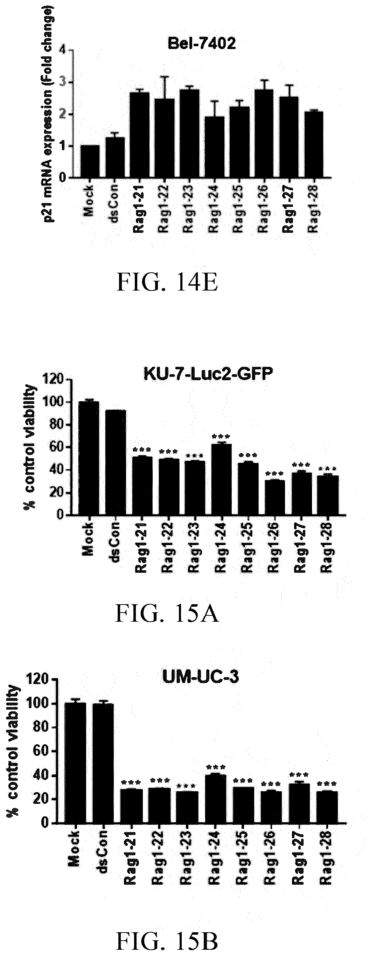

Figs. 14A-14E show the activities of the saRNAs (Rag1-21 to Rag1-28) in inducing p21 expression in human cancer cell lines. KU-7-Luc2-GFP (FIG. 14A), UM-UC-3 (FIG. 14B), T24 (FIG. 14C), J82 (FIG. 14D) and Bel-7402 (FIG. 14E) cells were transfected with 10 nM of the indicated saRNA variants for 72 hours. Mock samples were transfected in the absence of an oligonucleotide. Non-specific duplex (dsCon) served as a negative control. Relative p21 mRNA expression was determined by RT-qPCR (mean ± SD). Expression levels of p21 were normalized to that of GAPDH. Statistical significance was determined by one-way ANOVA and Tukey's multiple comparisons test (^{∗}P<0.5; ^{∗∗}P<0.01; ^{∗∗∗} p < 0.001 versus Mock).

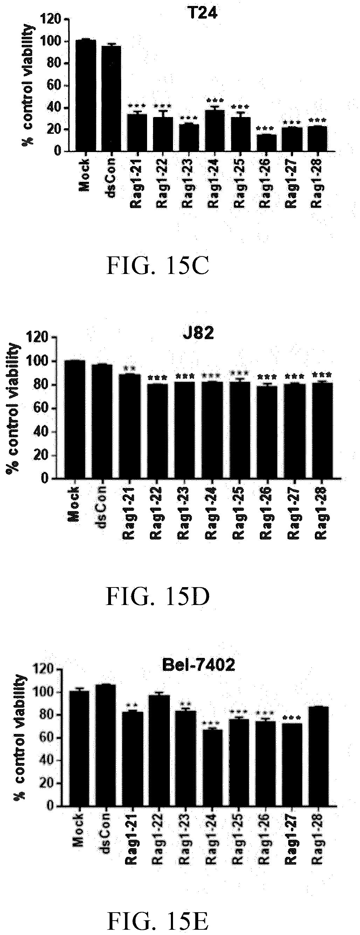

Figs. 15A-15E show the suppressing effect of the saRNAs (Rag1-21 to Rag1-28) on the proliferation of human cancer cell lines. KU-7-Luc2-GFP (FIG. 15A), UM-UC-3 (FIG. 15B), T24 (FIG. 15C), J82 (FIG. 15D) and Bel-7402 (FIG. 15E) cells were transfected with 10 nM of the indicated saRNA variants for 72 hours. Mock samples were transfected in the absence of an oligonucleotide. Non-specific duplex (dsCon) served as a negative control. Cell viabilities were quantitatively assayed by the CCK-8 method. Statistical significance was determined by one-way ANOVA and Tukey's multiple comparisons test (^{∗}P<0.5; ^{∗∗}P<0.01; ^{∗∗∗} p < 0.001 versus Mock).

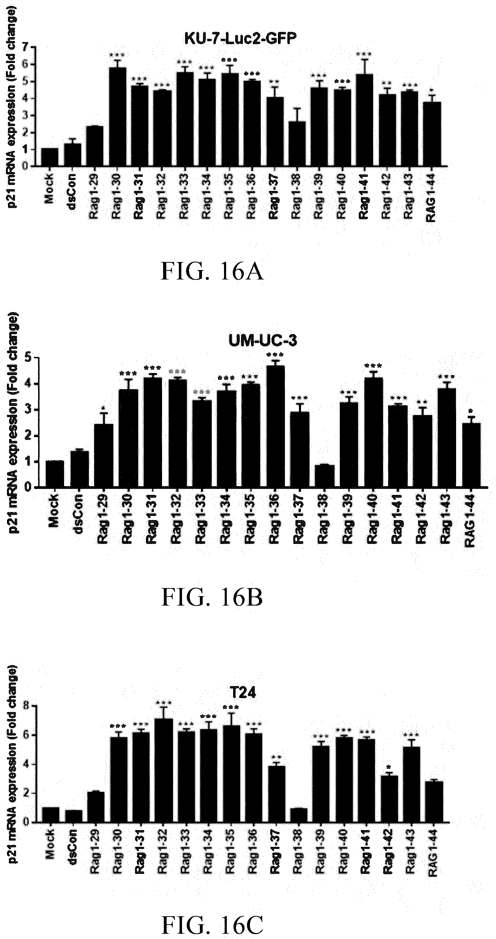

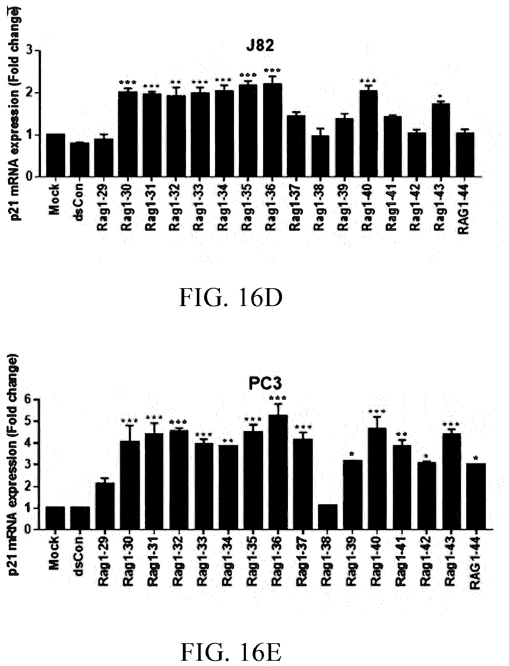

Figs. 16A-16E show the activity of saRNAs (Rag1-29 to Rag1-44) in inducing p21 expression in human cancer cell lines. KU-7-Luc2-GFP (FIG. 16A), UM-UC-3 (FIG. 16B), T24 (FIG. 16C), J82 (FIG. 16D), and PC3 (FIG. 16E) cells were transfected with 10 nM of the indicated saRNA variants for 72 hours. Mock samples were transfected in the absence of an oligonucleotide. Non-specific duplex (dsCon) served as a negative control. Relative p21 mRNA expression was determined by RT-qPCR (mean ± SD). Expression levels of p21 were normalized to that of GAPDH. Statistical significance was determined by one-way ANOVA and Tukey's multiple comparisons test (^{∗}P<0.5; ^{∗∗}P<0.01; ^{∗∗∗} p < 0.001 versus Mock).

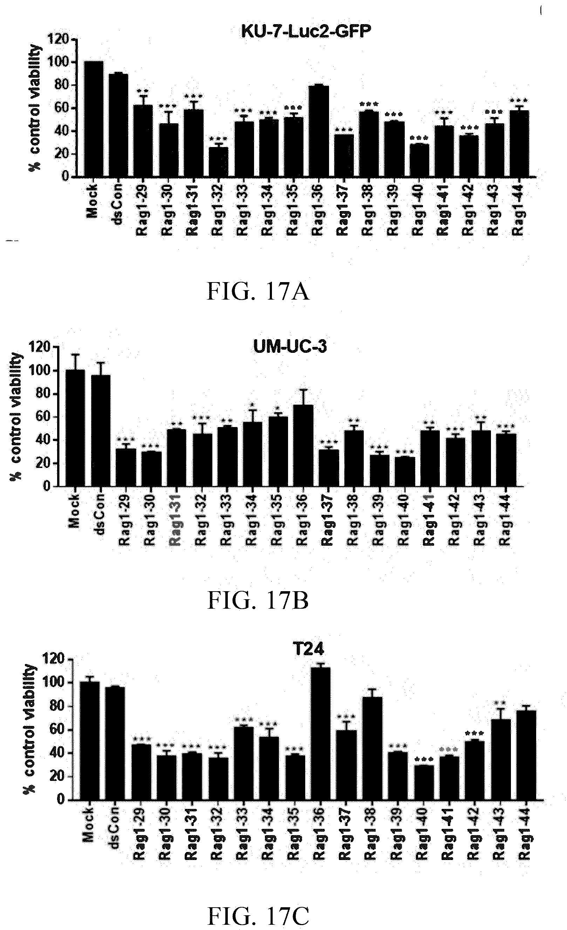

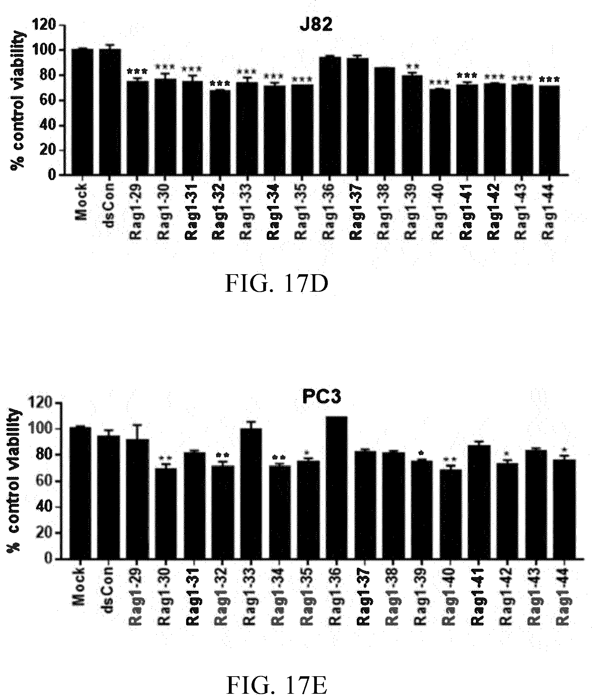

Figs. 17A-17E show the suppressing effect of the saRNAs (Rag1-29 to Rag1-44) on the proliferation of human cancer cell lines. KU-7-Luc2-GFP (FIG. 17A), UM-UC-3 (FIG. 17B), T24 (FIG. 17C), J82 (FIG. 17D), and PC3 (FIG. 17E) cells were transfected with 10 nM of the indicated saRNA variants for 72 hours. Mock samples were transfected in the absence of an oligonucleotide. Non-specific duplex (dsCon) served as a negative control. Cell viability was quantitatively assayed by the CCK-8 method. Statistical significance was determined by one-way ANOVA and Tukey's multiple comparisons test (^{∗}P<0.5; ^{∗∗}P<0.01; ^{∗∗∗} p < 0.001 versus Mock).

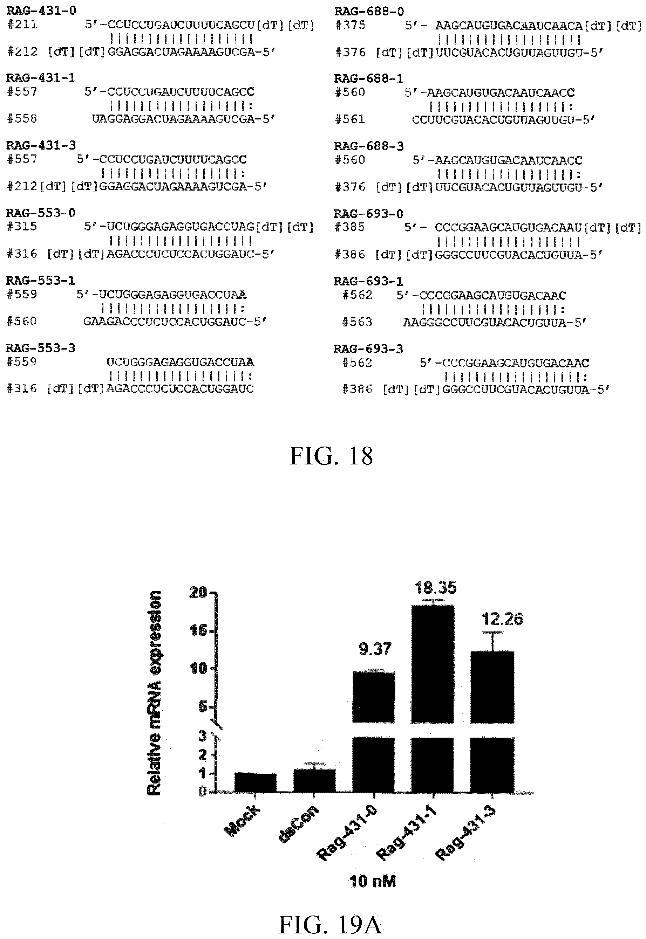

Fig. 18 is a schematic diagram for p21 saRNA duplex sequences (SEQ ID NOs: 211, 212, 315, 316, 375, 376, 385, 386, 557-563). Mispaired bases are represented by colons, and boldfaces represent altered nucleotides or structure in saRNA variants.
Figs. 19A-19D show gene induction activity of saRNAs targeting the p21 promoter. KU-7-Luc2-GFP cells were transfected with 10 nM of the indicated saRNA variants for 72 hours. Mock samples were transfected in the absence of an oligonucleotide. Non-specific duplex (dsCon) served as a negative control. Relative p21 mRNA expression was determined by RT-qPCR (mean ± SD). Expression levels of p21 were normalized to that of GAPDH.

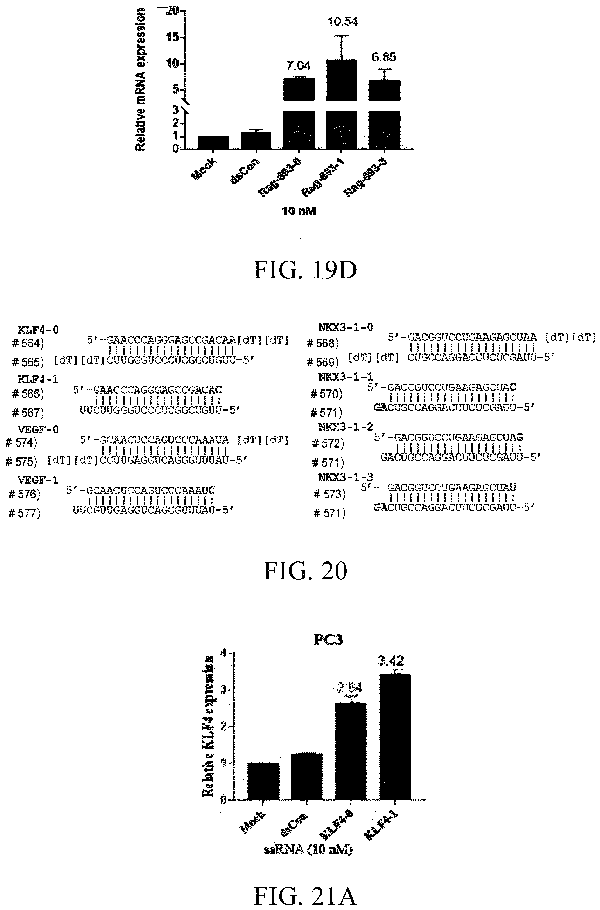

Fig. 20 is a schematic diagram of saRNA duplex sequences targeting the promoter of KLF4, NKX3-1, and VEGFA genes (SEQ ID NOs: 564-577). Mispaired bases are represented by colons, and boldfaces represent altered nucleotides or structures in saRNA variants.

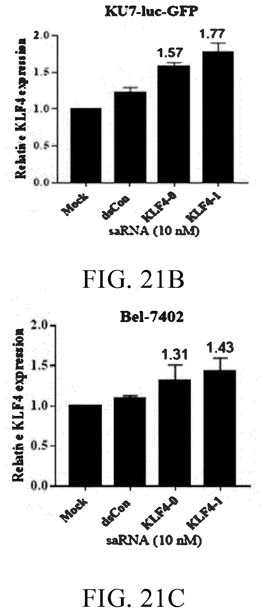

Figs. 21A-21C show the gene induction activities of saRNAs targeting the KLF4 promoter. PC3 (FIG. 21A), KU-7-Luc2-GFP (FIG. 21B), and Bel-7402 (FIG. 21C) cells were transfected with the indicated saRNAs individually at the indicated concentration for 72 hours. Transfected samples containing no oligonucleotide were adopted as blank controls (Mock), and non-specific duplex (dsCon) transfected samples were adopted as negative controls. Relative KLF4 mRNA expression was determined by RT-qPCR (mean ± SD). Expression levels of KLF4 were normalized to that of GAPDH.

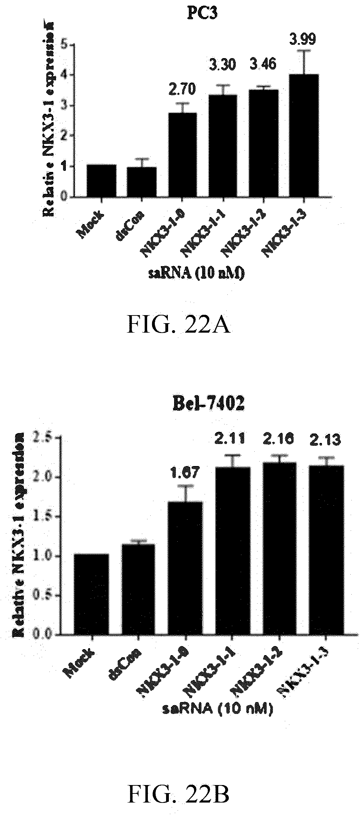

Figs. 22A and 22B show the gene induction activities of saRNAs targeting the NKX3-1 promoter. PC3 (FIG. 22A) and Bel-7402 (FIG. 22B) cells were transfected with the indicated saRNAs individually at the indicated concentration for 72 hours. Transfected samples containing no oligonucleotide were adopted as blank controls (Mock), and non-specific duplex (dsCon) transfected samples were adopted as negative controls. Relative NKX3-1 mRNA expression was determined by RT-qPCR (mean ± SD). Expression levels of NKX3-1 were normalized to that of GAPDH.

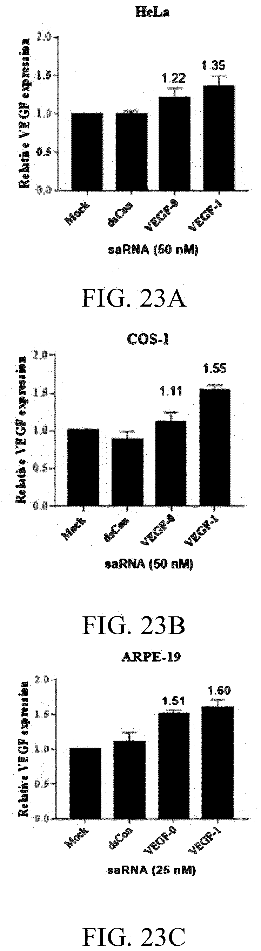

Figs. 23A-23C show gene induction activity of saRNAs targeting the VEGFA promoter. HeLa (FIG. 23A), COS-1 (FIG. 23B), and ARPE-19 (FIG. 23C) cells were transfected with the indicated saRNAs individually at the indicated concentration for 72 hours. Transfected samples containing no oligonucleotide were adopted as blank controls (Mock), and non-specific duplex (dsCon) transfected samples were adopted as negative controls. Relative VEGFA mRNA expression was determined by RT-qPCR (mean ± SD). Expression levels of VEGFA were normalized to that of GAPDH.

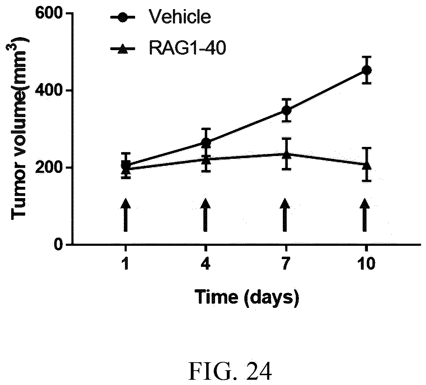

Fig. 24 shows the suppression of *in vivo* tumor growth by p21 saRNA. The indicated saRNA was intratumorally injected at a dose of 1 mg/kg, and the volume of tumor was recorded. The arrows indicate the time points of injections.

### DETAILED DESCRIPTION OF THE INVENTION

The present invention is further described hereinafter by specific description.

Unless otherwise defined, all the technological and scientific terms used therein have the same meanings as those generally understood by those of ordinary skill in the art covering the present invention.

In the present application, singular forms, such as "a" and "this", include plural objects, unless otherwise specified clearly in the context.

### Definition

The term "complementary" as used herein refers to the capability of forming base pairs between two oligonucleotide strands. The base pairs are generally formed by hydrogen bonds between nucleotide units in the antiparallel oligonucleotide strands. The bases of the complementary oligonucleotide strands can be paired in the Watson-Crick manner (such as A to T, A to U, and C to G) or in any other manners allowing the formation of a duplex (such as Hoogsteen or reverse Hoogsteen base pair). "100% pairing" refers to 100% complementarity, that is, all the nucleotide units of the two strands are bound by hydrogen bonds.

"Complete complementarity" or "100% complementarity" means that each nucleotide unit from the first oligonucleotide strand can form a hydrogen bond with the second oligonucleotide strand in the double-stranded region of the double-stranded oligonucleotide molecule, with no base pair being mispaired. "Incomplete complementarity" means that not all the nucleotide units of the two strands are bound with each other by hydrogen bonds. For example, for two oligonucleotide strands each of 20 nucleotides in length in the double-stranded region, if only two base pairs in this double-stranded region can be formed through hydrogen bonds, the oligonucleotide strands have a complementarity of 10%. In the same example, if 18 base pairs in this double-stranded region can be formed through hydrogen bonds, the oligonucleotide strands have a complementarity of 90%. "Substantial complementarity" refers to more than about 79%, about 80%, about 85%, about 90%, or about 95% complementarity.

The term "oligonucleotide" as used herein refers to polymers of nucleotides, and includes, but is not limited to, single-stranded or double-stranded molecules of DNA, RNA, or DNA/RNA hybrid, oligonucleotide strands containing regularly and irregularly alternating deoxyribonucleotide portions and ribonucleotide portions, as well as modified and naturally or unnaturally existed frameworks for such oligonucleotides. The oligonucleotide for activating target gene transcription described in the present invention is a saRNA.

The term "oligoribonucleotide" as used herein refers to an oligonucleotide containing two or more modified or unmodified ribonucleotides and/or analogues thereof.

The terms "oligonucleotide strand" and "oligonucleotide sequence" as used herein can be used interchangeably, referring to a generic term for short nucleotide sequences having less than 30 bases (including deoxyribonucleic acid (DNA) or ribonucleic acid (RNA)). In the present invention, the length of an oligonucleotide strand can be any length from 17 to 30 nucleotides.

The term "gene" as used herein refers to all nucleotide sequences required to code a polypeptide chain or to transcribe a functional RNA. "Gene" can be an endogenous or fully or partially recombinant gene for a host cell (for example, because an exogenous oligonucleotide and a coding sequence for coding a promoter are introduced into a host cell, or a heterogeneous promoter adjacent to an endogenous coding sequence is introduced into a host cell). For example, the term "gene" includes a nucleic acid sequence composed of exons and introns. Protein-coding sequences are, for example, sequences contained within exons in an open reading frame between an initiation codon and a termination codon, and as used herein, "gene" can comprise a gene regulatory sequence, such as a promoter, an enhancer, and all other sequences known in the art for controlling the transcription, expression or activity of another gene, no matter whether the gene contains a coding sequence or a non-coding sequence. In one case, for example, "gene" can be used to describe a functional nucleic acid containing a regulatory sequence such as a promoter or an enhancer. The expression of a recombinant gene can be controlled by one or more types of heterogeneous regulatory sequences.

The term "target gene" as used herein can refer to nucleic acid sequences, transgenes, viral or bacterial sequences, chromosomes or extrachromosomal genes that are naturally present in organisms, and/or can be transiently or stably transfected or incorporated into cells and/or chromatins thereof. The target gene can be a protein-coding gene or a non-protein-coding gene (such as a microRNA gene and a long non-coding RNA gene). The target gene generally contains a promoter sequence, and the positive regulation for the target gene can be achieved by designing a saRNA having sequence homology with the promoter sequence, characterized as the upregulation of expression of the target gene. "Sequence of a target gene promoter" refers to a non-coding sequence of the target gene, and the reference of the sequence of a target gene promoter in the phrase "complementary with the sequence of a target gene promoter" of the present invention means a coding strand of the sequence, also known as a non-template strand, i.e. a nucleic acid sequence having the same sequence as the coding sequence of the gene. "Target sequence" refers to a sequence fragment in the target gene promoter, which is homologous or complementary with a sense oligonucleotide strand or an antisense oligonucleotide strand of a saRNA.

As used herein, the term "first oligonucleotide strand" can be a sense strand or an antisense strand. The sense strand of a saRNA refers to an oligonucleotide strand having homology with the coding strand of the promoter DNA sequence of the target gene in the saRNA duplex. The antisense strand refers to an oligonucleotide strand complementary with the sense strand in the saRNA duplex.

As used herein, the term "second oligonucleotide strand" can also be a sense strand or an antisense strand. If the first oligonucleotide strand is a sense strand, the second oligonucleotide strand is an antisense strand; and if the first oligonucleotide strand is an antisense strand, the second oligonucleotide strand is a sense strand.

The term "coding strand" as used herein refers to a DNA strand in the target gene which is not used for transcription of the gene, and the nucleotide sequence of this strand is the same as that of a RNA produced from transcription (in the RNA, T in DNA is replaced by U). The coding strand of the double-stranded DNA sequence of the target gene promoter described in the present invention refers to a promoter sequence on the same DNA strand as the DNA coding strand of the target gene.

The term "template strand" as used herein refers to the other strand complementary with the coding strand in the double-stranded DNA of the target gene, i.e. the strand that, as a template, can be transcribed into RNA, and this strand is complementary with the transcribed RNA (A to U and G to C). In the process of transcription, RNA polymerase is bound with the template strand, moves along the 3'→5' direction of the template strand, and catalyzes the synthesis of the RNA in the 5'→3' direction. The template strand of the double-stranded DNA sequence of the target gene promoter described in the present invention refers to a promoter sequence on the same DNA strand as the DNA template strand of the target gene.

The term "promoter" as used herein refers to a nucleic acid sequence, which encodes no proteins and plays a regulatory role for the transcription of a protein-coding or RNA-coding nucleic acid sequence by associating with them spatially. Generally, a eukaryotic promoter contains 100 to 5,000 base pairs, although this length range is not intended to limit the term of "promoter" as used herein. Although the promoter sequence is generally located at the 5' terminus of a protein-coding or RNA-coding sequence, it also exists in exon and intron sequences.

The term "transcription start site" as used herein refers to a nucleotide marking the transcription start on the template strand of a gene. The transcription start site can appear on the template strand of the promoter region. A gene can have more than one transcription start sites.

The term "sequence identity" or "sequence homology" as used herein means that one oligonucleotide strand (sense or antisense) of a saRNA has at least 80% similarity with a region on the coding strand or template strand of the promoter sequence of a target gene.

The term "overhang" as used herein refers to non-base-paired nucleotides at the terminus (5' or 3') of an oligonucleotide strand, which is formed by one strand extending out of the other strand in a duplex oligonucleotide. A single-stranded region extending out of the 3' terminus and/or 5' terminus of a duplex is referred to as an overhang.

The terms "natural overhang" and "natural nucleotide overhang" as used herein can be used interchangeably, and refer to an overhang, at the 5' or 3' terminus of the sense or antisense strand of an saRNA, that is derived from or homologous to its target sequence.

As used herein, the terms "gene activation" or "activating gene expression" can be used interchangeably, and means an increase or upregulation in transcription, translation, expression or activity of a certain nucleic acid as determined by measuring the transcription level, mRNA level, protein level, enzymatic activity, methylation state, chromatin state or configuration, translation level or the activity or state in a cell or biological system of a gene. These activities or states can be determined directly or indirectly. In addition, "gene activation" or "activating gene expression" refers to an increase in activity associated with a nucleic acid sequence, regardless the mechanism of such activation. For example, gene activation occurs at the transcriptional level to increase transcription into RNA and the RNA is translated into a protein, thereby increasing the expression of the protein.

As used herein, the terms "small activating RNA" and "saRNA" can be used interchangeably and refer to a ribonucleic acid molecule that can upregulate target gene expression. It can be a double-stranded nucleic acid molecule composed of a first nucleic acid strand containing a ribonucleotide sequence with sequence homology with the non-coding nucleic acid sequence (such as a promoter and an enhancer) of a target gene and a second nucleic acid strand containing a nucleotide sequence complementary with the first strand. The saRNA can also be comprised of a synthesized or vector-expressed single-stranded RNA molecule that can form a hairpin structure by two complementary regions within the molecule, wherein the first region contains a ribonucleotide sequence having sequence homology with the target sequence of a promoter of a gene, and a ribonucleotide sequence contained in the second region is complementary with the first region. The length of the duplex region of the saRNA molecule is typically about 10 to about 50, about 12 to about 48, about 14 to about 46, about 16 to about 44, about 18 to about 42, about 20 to about 40, about 22 to about 38, about 24 to about 36, about 26 to about 34, and about 28 to about 32 base pairs, and typically about 10, about 15, about 20, about 25, about 30, about 35, about 40, about 45 or about 50 base pairs. In addition, the terms "small activating RNA" and "saRNA" also contain nucleic acids other than the ribonucleotide, including, but not limited to, modified nucleotides or analogues.

As used herein, the term "hotspot" refers to a promoter region of a gene where targets for functional saRNAs are enriched. A hotspot is defined as a sequence region containing targets for at least 10 saRNAs, wherein at least 60% of the saRNAs can induce a 1.5-fold or more change in the mRNA expression of the gene.

As used herein, the term "synthesis" refers to the method for synthesis of an oligonucleotide, including any method allowing RNA synthesis, such as chemical synthesis, *in vitro* transcription, and/or vector-based expression.

As used herein, the term "p21" refers to the p21^{WAF1/CIP1} gene, also known as the CDKN1A gene. As a cyclin-dependent kinase (CDK) inhibitor, p21 is an important tumor suppressor gene. Overexpression of p21 or activation of endogenous p21 transcription has been shown to inhibit the growth of cultured tumor cells and tumors *in vivo.*

### Materials and methods

### Cell culture and transfection

Cell lines RT4, KU-7, T24, and HT-1197 were cultured in the modified McCoy's 5A medium (Gibco); cell lines J82, TCCSUP, and UM-UC-3 were cultured in the MEM medium (Gibco); and cell lines 5637, PC3, and Bel-7402 were cultured in the RPMI-1640 medium (Gibco). All the media contained 10% bovine calf serum (Sigma-Aldrich) and 1% penicillin/streptomycin (Gibco). The cells were cultured at 5% CO₂ and 37°C. saRNAs were transfected into cells using RNAiMax (Invitrogen, Carlsbad, CA) according to the instructions provided by the manufacturer at the concentration of 10 nM (unless otherwise specified). All the saRNA sequences are listed in Table 1.

**Table 1: Strand sequences and duplex compositions**

| Title | Sequence No. | Sequence (5'-3') | Length |
|---|---|---|---|
| dsCon | SEQ ID NO 1 | ACUACUGAGUGACAGUAGA[dT] [dT] | 21 nt |
| | SEQ ID NO 2 | UCUACUGUCACUCAGUAGU[dT] [dT] | 21 nt |
| P21-332 | SEQ ID NO 3 | CUGGAGAGUGCCAACUCAU[dT] [dT] | 21 nt |
| | SEQ ID NO 4 | AUGAGUUGGCACUCUCCAG[dT] [dT] | 21 nt |
| P21-331 | SEQ ID NO 5 | UGGAGAGUGCCAACUCAUU[dT][dT] | 21 nt |
| | SEQ ID NO 6 | AAUGAGUUGGCACUCUCCA[dT] [dT] | 21 nt |
| P21-330 | SEQ ID NO 7 | GGAGAGUGCCAACUCAUUC[dT] [dT] | 21 nt |
| | SEQ ID NO 8 | GAAUGAGUUGGCACUCUCC[dT][dT] | 21 nt |
| P21-329 | SEQ ID NO 9 | GAGAGUGCCAACUCAUUCU[dT][dT] | 21 nt |
| | SEQ ID NO 10 | AGAAUGAGUUGGCACUCUC[dT] [dT] | 21 nt |
| P21-328 | SEQ ID NO 11 | AGAGUGCCAACUCAUUCUC[dT] [dT] | 21 nt |
| | SEQ ID NO 12 | GAGAAUGAGUUGGCACUCU[dT][dT] | 21 nt |
| P21-327 | SEQ ID NO 13 | GAGUGCCAACUCAUUCUCC[dT] [dT] | 21 nt |
| | SEQ ID NO 14 | GGAGAAUGAGUUGGCACUC[dT] [dT] | 21 nt |
| P21-326 | SEQ ID NO 15 | AGUGCCAACUCAUUCUCCA[dT] [dT] | 21 nt |
| | SEQ ID NO 16 | UGGAGAAUGAGUUGGCACU[dT] [dT] | 21 nt |
| P21-325 | SEQ ID NO 17 | GUGCCAACUCAUUCUCCAA[dT] [dT] | 21 nt |
| | SEQ ID NO 18 | UUGGAGAAUGAGUUGGCAC[dT] [dT] | 21 nt |
| P21-324 | SEQ ID NO 19 | UGCCAACUCAUUCUCCAAG[dT] [dT] | 21 nt |
| | SEQ ID NO 20 | CUUGGAGAAUGAGUUGGCA[dT] [dT] | 21 nt |
| P21-323 | SEQ ID NO 21 | GCCAACUCAUUCUCCAAGU[dT] [dT] | 21 nt |
| | SEQ ID NO 22 | ACUUGGAGAAUGAGUUGGC[dT] [dT] | 21 nt |
| P21-322 | SEQ ID NO 23 | CCAACUCAUUCUCCAAGUA[dT] [dT] | 21 nt |
| | SEQ ID NO 24 | UACUUGGAGAAUGAGUUGG[dT] [dT] | 21 nt |
| P21-321 | SEQ ID NO 25 | CAACUCAUUCUCCAAGUAA[dT] [dT] | 21 nt |
| | SEQ ID NO 26 | UUACUUGGAGAAUGAGUUG[dT] [dT] | 21 nt |
| P21-301 | SEQ ID NO 27 | AAAAGCCAGAUUU GUGGCU[dT] [dT] | 21 nt |
| | SEQ ID NO 28 | AGCCACAAAUCUGGCUUUU[dT] [dT] | 21 nt |
| P21-300 | SEQ ID NO 29 | AAAGCCAGAUUUGUGGCUC[dT] [dT] | 21 nt |
| | SEQ ID NO 30 | GAGCCACAAAU CUGGCUUU [dT] [dT] | 21 nt |
| P21-299 | SEQ ID NO 31 | AAGCCAGAUUUGUGGCUCA[dT] [dT] | 21 nt |
| | SEQ ID NO 32 | UGAGCCACAAAUCUGGCUU[dT] [dT] | 21 nt |
| P21-298 | SEQ ID NO 33 | AGCCAGAUUUGUGGCUCAC[dT] [dT] | 21 nt |
| | SEQ ID NO 34 | GUGAGCCACAAAUCUGGCU[dT] [dT] | 21 nt |
| P21-297 | SEQ ID NO 35 | GCCAGAUUUGUGGCUCACU[dT] [dT] | 21 nt |
| | SEQ ID NO 36 | AGUGAGCCACAAAUCUGGC[dT] [dT] | 21 nt |
| Rag1-0 | SEQ ID NO 23 | CCAACUCAUUCUCCAAGUA[dT] [dT] | 21 nt |
| | SEQ ID NO 24 | UACUUGGAGAAUGAGUUGG[dT] [dT] | 21 nt |
| Rag1-1 | SEQ ID NO 37 | CCAACUCAUUCUCCAAGUAAA | 21 nt |
| | SEQ ID NO 38 | UACUUGGAGAAUGAGUUGGCA | 21 nt |
| Rag1-2 | SEQ ID NO 39 | CCAACUCAUUCUCCAAGUAUU | 21 nt |
| | SEQ ID NO 40 | UACUUGGAGAAUGAGUUGGUU | 21 nt |
| Rag1-3 | SEQ ID NO 41 | CCAACUCAUUCUCCAAGUAA[dT] [dT] | 22 nt |
| | SEQ ID NO 42 | UUACUUGGAGAAUGAGUUGG[dT] [dT] | 22 nt |
| Rag1-4 | SEQ ID NO 43 | CCAACUCAUUCUCCAAGUAAA[dT] [dT] | 23 nt |
| | SEQ ID NO 44 | UUUACUUGGAGAAUGAGUUGG[dT] [dT] | 23 nt |
| Rag1-5 | SEQ ID NO 45 | CCAACUCAUUCUCCAAGUAAAA[dT] [dT] | 24 nt |
| | SEQ ID NO 46 | UUUUACUUGGAGAAUGAGUUGG[dT] [dT] | 24 nt |
| Rag1-6 | SEQ ID NO 47 | CCAACUCAUUCUCCAAGUAAAAA[dT] [dT] | 25 nt |
| | SEQ ID NO 48 | UUUUUACUUGGAGAAUGAGUUGG[dT] [dT] | 25 nt |
| Rag1-7 | SEQ ID NO 49 | CCAACUCAUUCUCCAAGUAAAAAA[dT] [dT] | 26 nt |
| | SEQ ID NO 50 | UUUUUUACUUGGAGAAUGAGUUGG[dT] [dT] | 26 nt |
| Rag1-8 | SEQ ID NO 21 | GCCAACUCAUUCUCCAAGU[dT] [dT] | 21 nt |
| | SEQ ID NO 22 | ACUUGGAGAAUGAGUUGGC[dT] [dT] | 21 nt |
| Rag1-9 | SEQ ID NO 51 | GCCAACUCAUUCUCCAAGUAA | 21 nt |
| | SEQ ID NO 52 | ACUUGGAGAAUGAGUUGGCAC | 21 nt |
| Rag1-10 | SEQ ID NO 53 | GCCAACUCAUUCUCCAAGUA[dT] [dT] | 22 nt |
| | SEQ ID NO 54 | UACUUGGAGAAUGAGUUGGC[dT] [dT] | 22 nt |
| Rag1-11 | SEQ ID NO 55 | GCCAACUCAUUCUCCAAGUAA[dT] [dT] | 23 nt |
| | SEQ ID NO 56 | UUACUUGGAGAAUGAGUUGGC[dT] [dT] | 23 nt |
| Rag1-12 | SEQ ID NO 57 | GCCAACUCAUUCUCCAAGUAAA[dT] [dT] | 24 nt |
| | SEQ ID NO 58 | UUUACUUGGAGAAUGAGUUGGC[dT] [dT] | 24 nt |
| Rag1-13 | SEQ ID NO 59 | GCCAACUCAUUCUCCAAGUAAAA[dT] [dT] | 25 nt |
| | SEQ ID NO 60 | UUUUACUUGGAGAAUGAGUUGGC[dT] [dT] | 25 nt |
| Rag1-14 | SEQ ID NO 61 | GCCAACUCAUUCUCCAAGUAAAAA[dT] [dT] | 26 nt |
| | SEQ ID NO 62 | UUUUUACUUGGAGAAUGAGUUGGC[dT] [dT] | 26 nt |
| Rag1-15 | SEQ ID NO 63 | CCAACUCAUUCUCCAAGUU[dT] [dT] | 21 nt |
| | SEQ ID NO 24 | UACUUGGAGAAUGAGUUGG[dT] [dT] | 21 nt |
| Rag1-16 | SEQ ID NO 64 | CCAACUCAUUCUCCAAGUC[dT] [dT] | 21 nt |
| | SEQ ID NO 24 | UACUUGGAGAAUGAGUUGG[dT] [dT] | 21 nt |
| Rag1-17 | SEQ ID NO 65 | CCAACUCAUUCUCCAAGAU[dT] [dT] | 21 nt |
| | SEQ ID NO 24 | UACUUGGAGAAUGAGUUGG[dT] [dT] | 21 nt |
| Rag1-18 | SEQ ID NO 66 | CCAACUCAUUCUCCAAGUA | 19 nt |
| | SEQ ID NO 24 | UACUUGGAGAAUGAGUUGG[dT] [dT] | 21 nt |
| Rag1-19 | SEQ ID NO 67 | CAACUCAUUCUCCAAGUA | 18 nt |
| | SEQ ID NO 24 | UACUUGGAGAAUGAGUUGG[dT] [dT] | 21 nt |
| Rag1-20 | SEQ ID NO 68 | CAACUCAUUCUCCAAGUA[dT] [dT] | 20 nt |
| | SEQ ID NO 24 | UACUUGGAGAAUGAGUUGG[dT] [dT] | 21 nt |
| Rag1-21 | SEQ ID NO 69 | GCCAACUCAUUCUCCAAGUAUU | 22 nt |
| | SEQ ID NO 70 | UACUUGGAGAAUGAGUUGGCUU | 22 nt |
| Rag1-22 | SEQ ID NO 71 | GCCAACUCAUUCUCCAAGUAAA | 22 nt |
| | SEQ ID NO 72 | UACUUGGAGAAUGAGUUGGCAC | 22 nt |
| Rag1-23 | SEQ ID NO 73 | GCCAACUCAUUCUCCAAGUCUU | 22 nt |
| | SEQ ID NO 70 | UACUUGGAGAAUGAGUUGGCUU | 22 nt |
| Rag1-24 | SEQ ID NO 74 | CAACUCAUUCUCCAAGUCUU | 20 nt |
| | SEQ ID NO 70 | UACUUGGAGAAUGAGUUGGCUU | 22 nt |
| Rag1-25 | SEQ ID NO 75 | CCAACUCAUUCUCCAAGUCUU | 21 nt |
| | SEQ ID NO 40 | UACUUGGAGAAUGAGUUGGUU | 21 nt |
| Rag1-26 | SEQ ID NO 76 | CCAACUCAUUCUCCAAGUCAA | 21 nt |
| | SEQ ID NO 38 | UACUUGGAGAAUGAGUUGGCA | 21 nt |
| Rag1-27 | SEQ ID NO 74 | CAACUCAUUCUCCAAGUCUU | 20 nt |
| | SEQ ID NO 40 | UACUUGGAGAAUGAGUUGGUU | 21 nt |
| Rag1-28 | SEQ ID NO 77 | CAACUCAUUCUCCAAGUCAA | 20 nt |
| | SEQ ID NO 38 | UACUUGGAGAAUGAGUUGGCA | 21 nt |
| Rag1-29 | SEQ ID NO 78 | GCCAACUCAUUCUCCAAGUCAA | 22 nt |
| | SEQ ID NO 79 | UACUUGGAGAAUGAGUUGGC | 20 nt |
| Rag1-30 | SEQ ID NO 74 | CAACUCAUUCUCCAAGUCUU | 20 nt |
| | SEQ ID NO 38 | UACUUGGAGAAUGAGUUGGCA | 21 nt |
| Rag1-31 | SEQ ID NO 80 | CAACUCAUUCUCCAAGUC | 18 nt |
| | SEQ ID NO 38 | UACUUGGAGAAUGAGUUGGCA | 21 nt |
| Rag1-32 | SEQ ID NO 78 | GCCAACUCAUUCUCCAAGUCAA | 22 nt |
| | SEQ ID NO 72 | UACUUGGAGAAUGAGUUGGCAC | 22 nt |
| Rag1-33 | SEQ ID NO 81 | GCCAACUCAUUCUCCAAGUGAA | 22 nt |
| | SEQ ID NO 72 | UACUUGGAGAAUGAGUUGGCAC | 22 nt |
| Rag1-34 | SEQ ID NO 76 | CCAACUCAUUCUCCAAGUCAA | 21 nt |
| | SEQ ID NO 72 | UACUUGGAGAAUGAGUUGGCAC | 22 nt |
| Rag1-35 | SEQ ID NO 82 | GCCAACUCAUUCUCCAAGUC | 20 nt |
| | SEQ ID NO 72 | UACUUGGAGAAUGAGUUGGCAC | 22 nt |
| Rag1-36 | SEQ ID NO 83 | CCAACUCAUUCUCCAAGUC | 19 nt |
| | SEQ ID NO 72 | UACUUGGAGAAUGAGUUGGCAC | 22 nt |
| Rag1-37 | SEQ ID NO 84 | CCAACUCAUUCUCCAAGU | 18 nt |
| | SEQ ID NO 72 | UACUUGGAGAAUGAGUUGGCAC | 22 nt |
| Rag1-38 | SEQ ID NO 74 | CAACUCAUUCUCCAAGUCUU | 20 nt |
| | SEQ ID NO 85 | UUUACUUGGAGAAUGAGUUGG | 21 nt |
| Rag1-39 | SEQ ID NO 86 | CAACUCAUUCUCCAAGUGAA | 20 nt |
| | SEQ ID NO 38 | UACUUGGAGAAUGAGUUGGCA | 21 nt |
| Rag 1-40 | SEQ ID NO 83 | CCAACUCAUUCUCCAAGUC | 19 nt |
| | SEQ ID NO 38 | UACUUGGAGAAUGAGUUGGCA | 21 nt |
| Rag1-41 | SEQ ID NO 87 | CCAACUCAUUCUCCAAGUG | 19 nt |
| | SEQ ID NO 38 | UACUUGGAGAAUGAGUUGGCA | 21 nt |
| Rag1-42 | SEQ ID NO 84 | CCAACUCAUUCUCCAAGU | 18 nt |
| | SEQ ID NO 38 | UACUUGGAGAAUGAGUUGGCA | 21 nt |
| Rag1-43 | SEQ ID NO 80 | CAACUCAUUCUCCAAGUC | 18 nt |
| | SEQ ID NO 38 | UACUUGGAGAAUGAGUUGGCA | 21 nt |
| Rag 1-44 | SEQ ID NO 88 | CAACUCAUUCUCCAAGU | 17 nt |
| | SEQ ID NO 38 | UACUUGGAGAAUGAGUUGGCA | 21 nt |
| Rag-431-0 | SEQ ID NO 211 | CCUCCUGAUCUUUUCAGCU[dT] [dT] | 21 nt |
| | SEQ ID NO 212 | AGCUGAAAAGAUCAGGAGG[dT] [dT] | 21 nt |
| Rag-431-1 | SEQ ID NO 557 | CCUCCUGAUCUUUUCAGCC | 19 nt |
| | SEQ ID NO 558 | AGCUGAAAAGAUCAGGAGGAU | 21 nt |
| Rag-431-3 | SEQ ID NO 557 | CCUCCUGAUCUUUUCAGCC | 19 nt |
| | SEQ ID NO 212 | AGCUGAAAAGAUCAGGAGG[dT] [dT] | 21 nt |
| Rag-553-0 | SEQ ID NO 315 | UCUGGGAGAGGUGACCUAG[dT] [dT] | 21 nt |
| | SEQ ID NO 316 | CUAGGUCACCUCUCCCAGA[dT] [dT] | 21 nt |
| Rag-553-1 | SEQ ID NO 559 | UCUGGGAGAGGUGACCUAA | 19 nt |
| | SEQ ID NO 560 | CUAGGUCACCUCUCCCAGAAG | 21 nt |
| Rag-553-3 | SEQ ID NO 559 | UCUGGGAGAGGUGACCUAA | 19 nt |
| | SEQ ID NO 316 | CUAGGUCACCUCUCCCAGA[dT] [dT] | 21 nt |
| Rag-688-0 | SEQ ID NO: 375 | AAGCAUGUGACAAUCAACA[dT] [dT] | 21 nt |
| | SEQ ID NO: 376 | UGUUGAUUGUCACAUGCUU[dT] [dT] | 21 nt |
| Rag-688-1 | SEQ ID NO 560 | AAGCAUGUGACAAUCAACC | 19 nt |
| | SEQ ID NO 561 | UGUUGAUUGUCACAUGCUUCC | 21 nt |
| Rag-688-3 | SEQ ID NO 560 | AAGCAUGUGACAAUCAACC | 19 nt |
| | SEQ ID NO: 376 | UGUUGAUUGUCACAUGCUU[dT] [dT] | 21 nt |
| Rag-693-0 | SEQ ID NO: 385 | CCCGGAAGCAUGUGACAAU[dT] [dT] | 21 nt |
| | SEQ ID NO: 386 | AUUGUCACAUGCUUCCGGG[dT] [dT] | 21 nt |
| Rag-693-1 | SEQ ID NO 562 | CCCGGAAGCAUGUGACAAC | 19 nt |
| | SEQ ID NO 563 | AUUGUCACAUGCUUCCGGGAA | 21 nt |
| Rag-693-3 | SEQ ID NO 562 | CCCGGAAGCAUGUGACAAC | 19 nt |
| | SEQ ID NO: 386 | AUUGUCACAUGCUUCCGGG[dT] [dT] | 21 nt |
| KLF4-0 | SEQ ID NO 564 | GAACCCAGGGAGCCGACAA[dT] [dT] | 21 nt |
| | SEQ ID NO 565 | UUGUCGGCUCCCUGGGUUC[dT] [dT] | 21 nt |
| KLF4-1 | SEQ ID NO 566 | GAACCCAGGGAGCCGACAC | 19 nt |
| | SEQ ID NO 567 | UUGUCGGCUCCCUGGGUUCUU | 21 nt |
| NKX3-1-0 | SEQ ID NO 568 | GACGGUCCUGAAGAGCUAA[dT] [dT] | 21 nt |
| | SEQ ID NO 569 | UUAGCUCUUCAGGACCGUC[dT] [dT] | 21 nt |
| NKX3-1-1 | SEQ ID NO 570 | GACGGUCCUGAAGAGCUAC | 19 nt |
| | SEQ ID NO 571 | UUAGCUCUUCAGGACCGUCAG | 21 nt |
| Nkx3-1-2 | SEQ ID NO 572 | GACGGUCCUGAAGAGCUAG | 19 nt |
| | SEQ ID NO 571 | UUAGCUCUUCAGGACCGUCAG | 21 nt |
| NKX3-1-3 | SEQ ID NO 573 | GACGGUCCUGAAGAGCUAU | 19 nt |
| | SEQ ID NO 571 | UUAGCUCUUCAGGACCGUCAG | 21 nt |
| VEGF-0 | SEQ ID NO 574 | GCAACUCCAGUCCCAAAUA[dT] [dT] | 21 nt |
| | SEQ ID NO 575 | UAUUUGGGACUGGAGUUGC[dT] [dT] | 20 nt |
| VEGF-1 | SEQ ID NO 576 | GCAACUCCAGUCCCAAAUC | 19 nt |
| | SEQ ID NO 577 | UAUUUGGGACUGGAGUUGCUU | 21 nt |

### RNA Isolation and reverse transcription-polymerase chain reaction (RT-PCR)

Cells were seeded into 6-well plates with 2-3 × 10⁵ cells/well and were reverse transfected with oligonucleotide duplexes. At the end of the transfection, total cellular RNA was isolated using an RNeasy Plus Mini kit (Qiagen; Hilden, Germany) according to its manual. The resultant RNA (1 µg) was reverse transcribed into cDNA by using a PrimeScript RT kit containing gDNA Eraser (Takara, Shiga, Japan). The resultant cDNA was amplified in an ABI 7500 Fast Real-time PCR System (Applied Biosystems; Foster City, CA) using SYBR Premix Ex Taq II (Takara, Shiga, Japan) reagents and target gene specific primers. The reaction conditions were: 95°C for 3 seconds, 60°C for 30 seconds, and 40 cycles. Amplification of GAPDH served as an internal control. All primer sequences are listed in Table 2.

**Table 2: Primer sequences for RT-qPCR assay**

| **Primer Title** | **Sequence No.** | **Sequence (5'-3')** |
|---|---|---|
| GAPDH F | SEQ ID NO 89 | ATCACCATCTTCCAGGAGCGA |
| GAPDH R | SEQ ID NO 90 | TTCTCCATGGTGGTGAAGACG |
| CDKN1A F | SEQ ID NO 91 | GGAAGACCATGTGGACCTGT |
| CDKN1A R | SEQ ID NO 92 | GGATTAGGGCTTCCTCTTGG |
| KLF4 F | SEQ ID NO 578 | TTCCCATCTCAAGGCACACC |
| KLF4 R | SEQ ID NO 579 | GCGAATTTCCATCCACAGCC |
| NKX3-1 F | SEQ ID NO 580 | AGGCTTCCCCAAACCCCTAA |
| NKX3-1 R | SEQ ID NO 581 | TCCGTGAGCTTGAGGTTCTT |
| VEGF F | SEQ ID NO 582 | CTTTCTGCTGTCTTGGGTGC |
| VEGF R | SEQ ID NO 583 | TTCGTGATGATTCTGCCCTCC |

### Assay of cell proliferation

Cells were plated into a 96-well plate with 2-4 × 10³ cells/well, cultured overnight, and transfected with oligonucleotide duplexes. Three days after transfection, the CCK-8 reagent (Dojindo; Rockville, MD) was used to assess cell proliferation according to its manual. Briefly, 10 µL of CCK8 reagent was added into each well on the plate which was then incubated at 37°C for 1 hour. Absorbance for each well on the plate was measured at 450 nm by a microplate reader.

### QuantiGene 2.0 assay

Cells were plated into a 96-well plate and transfected with oligonucleotide duplexes. 72 hours after transfection, the mRNA levels of target genes were quantitatively assayed with a QuantiGene 2.0 kit (AffyMetrix; Santa Clara, CA). QuantiGene 2.0 assay is based on hybridization technology, wherein mRNA levels were directly quantified with gene-specific probes. The experimental procedure is briefly described as follows: a lysis solution was added to lyse the transfected cells, and the cell lysates were added into a capture well plate coated with probe for CDKN1A (p21) and HPRT1 which serves as a housekeeping gene for hybridizing overnight at 55°C. In order to enhance the hybridization signal, hybridizations with 2.0 PreAMP Probe, 2.0 AMP Probe, and 2.0 Label Probe were conducted sequentially in 100 µL of a corresponding buffer solution (provided by Quantigene 2.0 kit). All the hybridizations were conducted with shaking at 50-55°C for 1 hour. After the last wash step, 2.0 Substrate was added to the solution and incubated at room temperature for 5 minutes. Optical signals were detected with an Infinite 200 PRO plate reader (Tecan, Switzerland).

### Statistical analysis

Results were represented as mean ± standard deviation. One-way analysis of variance was carried out with GraphPad Prism software (GraphPad Software), and then a Tukey's t test was conducted for pairwise comparisons. A statistical significance was set as ^{∗} p < 0.05, ^{∗∗} p < 0.01 and ^{∗∗∗} p < 0.001.

### Examples

The present invention is further illustrated by the following examples. These examples are provided merely for illustration purposes and shall not be interpreted to limit the scope or content of the present invention in any way.

For the convenience of description, the structure of small activating saRNAs (saRNA for short hereinafter) are described by the following formulas.

Description for a single-stranded oligonucleotide:

[50Hₙ] + Nₙ+ [30Hₙ] (formula 1)

50Hₙ and 30Hₙ represents an overhang at the 5' and 3' terminus respectively, which can be a natural nucleotide overhang (natural overhang, NO), a deoxythymine nucleotide (T) overhang or a uracil nucleotide (U) overhang, wherein n represents the number of overhanging nucleotides; and Nₙ represents a region capable of forming a double-stranded structure with another oligonucleotide strand, wherein N can be S (sense strand) or AS (antisense strand), and n represents the number of nucleotides (excluding the overhang) for forming a double-stranded structure with another complementary strand. For example, 5NO₁ + AS₁₉ + 3T₂ represents an antisense strand that can form a double-stranded region of 19 nucleotides in length with another complementary strand, wherein there is an overhang having one natural nucleotide at the 5' terminus and an overhang having two deoxythymine nucleotides at the 3' terminus, and the total length is 22 nucleotides (i.e., 1+19+2). Square brackets represent an option.

Description for a double-stranded oligonucleotide:

[B₅] + [B₃] + [MM_{(N'nZ: X,Y)}] + [OH_{N5'}] (formula 2)

B₅ and B₃ represent a 5' and 3' blunt end respectively relative to the duplex or to the 5' terminus of the sense strand; MM represents mismatches between the two strands; Further, in the expression of (N'n Z: X, Y), n represents the position of a mispaired nucleotide in the duplex counted either from N' terminus of the duplex, wherein N' can be 5' or 3' terminus, X represents an unmutated nucleotide identical or complementary with a target sequence, Y represents a mutated nucleotide, Z represents the strand where the mutated nucleotide is located and can be S (for sense) or AS (for antisense). For example, "3' 1S:A, C" means that "A" (adenine nucleotide) of the sense strand at the first position counted from the 3' terminus of the duplex is mutated into "C" (cytosine nucleotide). OH_{N5'} represents an unconventional 5' overhang, and N represents S (sense) or AS (antisense) strand. For example, OH_{AS5'} means that a 5' overhang exists in the AS strand. A duplex containing symmetric 3' overhangs is regarded as a conventional structure and will not be specifically described with formula. Square brackets represent an option.

### Example 1: Screening of functional saRNAs targeting the promoter region of p21 gene

The coding strand (Fig. 1) for the 1 kb promoter sequence of p21 (SEQ ID NO: 601) was retrieved from the UCSC Genome database to screen for functional saRNAs capable of activating p21 gene expression. A total of 982 target sequences were obtained by selecting a target with a size of 19 bp starting from the -1 kb position upstream of the TSS and moving toward the TSS one base pair (bp) at a time. The target sequences were filtered to remove those that have a GC content higher than 65% or lower than 35% and those contain 5 or more consecutive nucleotides. After filtration of the target sequences, 439 target sequences remained and were used as candidates for screening. A RNA sequence of 19 nucleotides in size with 100% sequence homology with each of the candidate target sequence was synthesized chemically, and a dTdT dinucleotide was added to its 3' terminus to obtain a sense strand with a structural formula as S₁₉ + [3T₂]. Meanwhile, a RNA sequence of 19 nucleotides in size with 100% complementarity with the same target sequence was synthesized, and a dTdT dinucleotide was added to its 3' terminus to obtain an antisense strand with a structural formula as AS₁₉ + [3T₂]. The sense and the antisense RNA strands were mixed in the same mole number and annealed to obtain a double-stranded saRNA.

The aforementioned double-stranded saRNA was transfected into Ku-7-luc2-GFP cells at the final concentration of 10 nM, and 72 hours after transfection, the mRNA level of p21 was detected with QuantiGene 2.0 kit. The fold change of p21 mRNA level induced by each saRNA relative to the blank control was calculated and plotted in Fig. 2. In this study, the fold changes of p21mRNA induced by all saRNAs ranged from 0.66 (suppression) to 8.12 (induction) (Fig. 2B). 361 (82.2%) saRNAs induced a 1.01-fold to 8.12-fold change of p21 mRNA expression, 74 (16.9%) saRNAs exhibited a suppressing effect (0.99-fold to 0.66-fold change), and 4 (0.9%) saRNAs did not change p21 mRNA level (1.0-fold).

Among the 439 screened saRNAs, 132 saRNAs (30.1%) could induce at least a 2-fold change, and 229 saRNAs (52.4%) could induce at least a 1.5-fold change in p21 mRNA level. The targets for these functional saRNAs were scattered in the entire p21 promoter region. However, 8 discrete regions showed enrichment for saRNA targets and these regions are considered as saRNA "hotspots". The hotspot is defined as a region containing targets at least for 10 corresponding saRNAs, wherein at least 60% of them can induce a 1.5-fold or more change in the mRNA expression of p21 (Fig. 2A and Fig. 3). The target sequences for hotspots 1 to hotspot 8 and their corresponding saRNA sequences are listed in Table 3 and Table 4 respectively.

**Table 3: Target sequences of hotspot regions of p21 promoter**

| **Hotspot** | **Sequence No.** | **DNA Sequence (5'-3')** | **Range** | **Length (bp)** |
|---|---|---|---|---|
| Hotspot 1 | SEQ ID NO: 93 | ggctatgtggggagtattcaggagacagacaactcact cgtcaaatcctccccttcctggccaacaaagctgctgca accacagggatttct | -893∼-801 | 92 |
| Hotspot 2 | SEQ ID NO: 94 | ggtagtctctccaattccctccttcccggaagcatgtgac aatcaacaactttgtatacttaagttcagtggacctcaattt cctc | -717∼-632 | 86 |
| Hotspot 3 | SEQ ID NO: 95 | ctttgttggggtgtctaggtgctccaggtgcttct | -585∼-551 | 35 |
| Hotspot 4 | SEQ ID NO: 96 | ttctgggagaggtgacctagtgagggatcagtgggaat agaggtgatattg | -554∼-504 | 51 |
| Hotspot 5 | SEQ ID NO: 97 | aggtgatattgtggggcttttctggaaatt | -514∼-485 | 30 |
| Hotspot 6 | SEQ ID NO: 98 | attaatgtcatcctcctgatcttttcagctgcattggg | -442∼-405 | 38 |
| Hotspot 7 | SEQ ID NO: 99 | tctaacagtgctgtgtcctcctggagagtgccaactcatt | -352∼-313 | 40 |
| Hotspot 8 | SEQ ID NO: 100 | gtgccaactcattctccaagtaaaaaaagccagatttgtg gctcacttcgtggggaaatgtgtcca | -325∼-260 | 66 |

**Table 4: functional saRNAs located in hotspot regions of p21 promoter**

| **Title** | **Sequence No.** | **Sequence (5'-3')** | **Length** |
|---|---|---|---|
| **Hotspot 8** | | | |
| RAG-278 | SEQ ID NO: 101 | UCGUGGGGAAAUGUGUCCA[dT] [dT] | 21 nt |
| | SEQ ID NO: 102 | UGGACACAUUUCCCCACGA[dT] [dT] | 21 nt |
| RAG-279 | SEQ ID NO: 103 | UUCGUGGGGAAAUGUGUCC[dT] [dT] | 21 nt |
| | SEQ ID NO: 104 | GGACACAUUUCCCCACGAA[dT] [dT] | 21 nt |
| RAG-280 | SEQ ID NO: 105 | CUUCGUGGGGAAAUGUGUC[dT][dT] | 21 nt |
| | SEQ ID NO: 106 | GACACAUUUCCCCACGAAG[dT][dT] | 21 nt |
| RAG-281 | SEQ ID NO: 107 | ACUUCGUGGGGAAAUGUGU[dT] [dT] | 21 nt |
| | SEQ ID NO: 108 | ACACAUUUCCCCACGAAGU[dT] [dT] | 21 nt |
| RAG-282 | SEQ ID NO: 109 | CACUUCGUGGGGAAAUGUG[dT] [dT] | 21 nt |
| | SEQ ID NO: 110 | CACAUUUCCCCACGAAGUG[dT] [dT] | 21 nt |
| RAG-283 | SEQ ID NO: 111 | UCACUUCGUGGGGAAAUGU[dT] [dT] | 21 nt |
| | SEQ ID NO: 112 | ACAUUUCCCCACGAAGUGA[dT] [dT] | 21 nt |
| RAG-284 | SEQ ID NO: 113 | CUCACUUCGUGGGGAAAUG[dT] [dT] | 21 nt |
| | SEQ ID NO: 114 | CAUUUCCCCACGAAGUGAG[dT] [dT] | 21 nt |
| RAG-285 | SEQ ID NO: 115 | GCUCACUUCGUGGGGAAAU[dT] [dT] | 21 nt |
| | SEQ ID NO: 116 | AUUUCCCCACGAAGUGAGC[dT] [dT] | 21 nt |
| RAG-286 | SEQ ID NO: 117 | GGCUCACUUCGUGGGGAAA[dT] [dT] | 21 nt |
| | SEQ ID NO: 118 | UUUCCCCACGAAGUGAGCC[dT] [dT] | 21 nt |
| RAG-287 | SEQ ID NO: 119 | UGGCUCACUUCGUGGGGAA[dT] [dT] | 21 nt |
| | SEQ ID NO: 120 | UUCCCCACGAAGUGAGCCA[dT] [dT] | 21 nt |
| RAG-288 | SEQ ID NO: 121 | GUGGCUCACUUCGUGGGGA[dT] [dT] | 21 nt |
| | SEQ ID NO: 122 | UCCCCACGAAGUGAGCCAC[dT] [dT] | 21 nt |
| RAG-289 | SEQ ID NO: 123 | UGUGGCUCACUUCGUGGGG[dT][dT] | 21 nt |
| | SEQ ID NO: 124 | CCCCACGAAGUGAGCCACA[dT][dT] | 21 nt |
| RAG-291 | SEQ ID NO: 125 | UUUGUGGCUCACUUCGUGG[dT] [dT] | 21 nt |
| | SEQ ID NO: 126 | CCACGAAGUGAGCCACAAA[dT] [dT] | 21 nt |
| RAG-292 | SEQ ID NO: 127 | AUUUGUGGCUCACUUCGUG[dT] [dT] | 21 nt |
| | SEQ ID NO: 128 | CACGAAGUGAGCCACAAAU [dT] [dT] | 21 nt |
| RAG-293 | SEQ ID NO: 129 | GAUUUGUGGCUCACUUCGU[dT][dT] | 21 nt |
| | SEQ ID NO: 130 | ACGAAGUGAGCCACAAAUC[dT][dT] | 21 nt |
| RAG-294 | SEQ ID NO: 131 | AGAUUUGUGGCUCACUUCG[dT] [dT] | 21 nt |
| | SEQ ID NO: 132 | CGAAGUGAGCCACAAAUCU [dT] [dT] | 21 nt |
| RAG-295 | SEQ ID NO: 133 | CAGAUUUGUGGCUCACUUC[dT] [dT] | 21 nt |
| | SEQ ID NO: 134 | GAAGUGAGCCACAAAUCUG[dT] [dT] | 21 nt |
| RAG-296 | SEQ ID NO: 135 | CCAGAUUUGUGGCUCACUU[dT][dT] | 21 nt |
| | SEQ ID NO: 136 | AAGUGAGCCACAAAUCUGG[dT][dT] | 21 nt |
| RAG-297 | SEQ ID NO: 137 | GCCAGAUUUGUGGCUCACU[dT][dT] | 21 nt |
| | SEQ ID NO: 138 | AGUGAGCCACAAAUCUGGC[dT][dT] | 21 nt |
| RAG-298 | SEQ ID NO: 139 | AGCCAGAUUUGUGGCUCAC[dT] [dT] | 21 nt |
| | SEQ ID NO: 140 | GUGAGCCACAAAUCUGGCU[dT] [dT] | 21 nt |
| RAG-299 | SEQ ID NO: 141 | AAGCCAGAUUUGUGGCUCA[dT] [dT] | 21 nt |
| | SEQ ID NO: 142 | UGAGCCACAAAUCUGGCUU[dT][dT] | 21 nt |
| RAG-300 | SEQ ID NO: 143 | AAAGCCAGAUUUGUGGCUC[DT] [dT] | 21 nt |
| | SEQ ID NO: 144 | GAGCCACAAAUCUGGCUUU[dT] [dT] | 21 nt |
| RAG-301 | SEQ ID NO: 145 | AAAAGCCAGAUUUGUGGCU[dT][dT] | 21 nt |
| | SEQ ID NO: 146 | AGCCACAAAUCUGGCUUUU[dT][dT] | 21 nt |
| RAG-321 | SEQ ID NO: 147 | CAACUCAUUCUCCAAGUAA[dT] [dT] | 21 nt |
| | SEQ ID NO: 148 | UUACUUGGAGAAUGAGUUG[dT] [dT] | 21 nt |
| RAG-322 | SEQ ID NO: 149 | CCAACUCAUUCUCCAAGUA[dT][dT] | 21 nt |
| | SEQ ID NO: 150 | UACUUGGAGAAUGAGUUGG[dT][dT] | 21 nt |
| RAG-323 | SEQ ID NO: 151 | GCCAACUCAUUCUCCAAGU[dT][dT] | 21 nt |
| | SEQ ID NO: 152 | ACUUGGAGAAUGAGUUGGC[dT][dT] | 21 nt |
| RAG-324 | SEQ ID NO: 153 | UGCCAACUCAUUCUCCAAG[dT] [dT] | 21 nt |
| | SEQ ID NO: 154 | CUUGGAGAAUGAGUUGGCA[dT] [dT] | 21 nt |
| RAG-325 | SEQ ID NO: 155 | GUGCCAACUCAUUCUCCAA[dT][dT] | 21 nt |
| | SEQ ID NO: 156 | UUGGAGAAUGAGUUGGCAC[dT][dT] | 21 nt |

| **Hotspot 7** | | | |
|---|---|---|---|
| RAG-331 | SEQ ID NO: 157 | UGGAGAGUGCCAACUCAUU[dT] [dT] | 21 nt |
| | SEQ ID NO: 158 | AAUGAGUUGGCACUCUCCA[dT] [dT] | 21 nt |
| RAG-332 | SEQ ID NO: 159 | CUGGAGAGUGCCAACUCAU[dT] [dT] | 21 nt |
| | SEQ ID NO: 160 | AUGAGUUGGCACUCUCCAG[dT] [dT] | 21 nt |
| RAG-333 | SEQ ID NO: 161 | CCUGGAGAGUGCCAACUCA[dT] [dT] | 21 nt |
| | SEQ ID NO: 162 | UGAGUUGGCACUCUCCAGG[dT] [dT] | 21 nt |
| RAG-334 | SEQ ID NO: 163 | UCCUGGAGAGUGCCAACUC[dT][dT] | 21 nt |
| | SEQ ID NO: 164 | GAGUUGGCACUCUCCAGGA[dT][dT] | 21 nt |
| RAG-335 | SEQ ID NO: 165 | CUCCUGGAGAGUGCCAACU[dT] [dT] | 21 nt |
| | SEQ ID NO: 166 | AGUUGGCACUCUCCAGGAG[dT] [dT] | 21 nt |
| RAG-336 | SEQ ID NO: 167 | CCUCCUGGAGAGUGCCAAC[dT] [dT] | 21 nt |
| | SEQ ID NO: 168 | GUUGGCACUCUCCAGGAGG[dT] [dT] | 21 nt |
| RAG-337 | SEQ ID NO: 169 | UCCUCCUGGAGAGUGCCAA[dT] [dT] | 21 nt |
| | SEQ ID NO: 170 | UUGGCACUCUCCAGGAGGA[dT] [dT] | 21 nt |
| RAG-338 | SEQ ID NO: 171 | GUCCUCCUGGAGAGUGCCA[dT][dT] | 21 nt |
| | SEQ ID NO: 172 | UGGCACUCUCCAGGAGGAC[dT] [dT] | 21 nt |
| RAG-341 | SEQ ID NO: 173 | UGUGUCCUCCUGGAGAGUG[dT][dT] | 21 nt |
| | SEQ ID NO: 174 | CACUCUCCAGGAGGACACA[dT][dT] | 21 nt |
| RAG-342 | SEQ ID NO: 175 | CUGUGUCCUCCUGGAGAGU[dT][dT] | 21 nt |
| | SEQ ID NO: 176 | ACUCUCCAGGAGGACACAG[dT] [dT] | 21 nt |
| RAG-343 | SEQ ID NO: 177 | GCUGUGUCCUCCUGGAGAG[dT][dT] | 21 nt |
| | SEQ ID NO: 178 | CUCUCCAGGAGGACACAGC[dT][dT] | 21 nt |
| RAG-344 | SEQ ID NO: 179 | UGCUGUGUCCUCCUGGAGA[dT] [dT] | 21 nt |
| | SEQ ID NO: 180 | UCUCCAGGAGGACACAGCA[dT] [dT] | 21 nt |
| RAG-345 | SEQ ID NO: 181 | GUGCUGUGUCCUCCUGGAG[dT][dT] | 21 nt |
| | SEQ ID NO: 182 | CUCCAGGAGGACACAGCAC[dT][dT] | 21 nt |
| RAG-346 | SEQ ID NO: 183 | AGUGCUGUGUCCUCCUGGA[dT] [dT] | 21 nt |
| | SEQ ID NO: 184 | UCCAGGAGGACACAGCACU[dT][dT] | 21 nt |
| RAG-348 | SEQ ID NO: 185 | ACAGUGCUGUGUCCUCCUG[dT] [dT] | 21 nt |
| | SEQ ID NO: 186 | CAGGAGGACACAGCACUGU [dT][dT] | 21 nt |
| RAG-349 | SEQ ID NO: 187 | AACAGUGCUGUGUCCUCCU[dT][dT] | 21 nt |
| | SEQ ID NO: 188 | AGGAGGACACAGCACUGUU[dT][dT] | 21 nt |
| RAG-350 | SEQ ID NO: 189 | U A AC AGUGCU GU GU CCUCC [dT] [dT] | 21 nt |
| | SEQ ID NO: 190 | GGAGGACACAGCACUGUU A[dT] [dT] | 21 nt |
| RAG-351 | SEQ ID NO: 191 | CUAACAGUGCUGUGUCCUC[dT][dT] | 21 nt |
| | SEQ ID NO: 192 | GAGGACACAGCACUGUUAG[dT] [dT] | 21 nt |
| RAG-352 | SEQ ID NO: 193 | UCUAACAGUGCUGUGUCCU[dT][dT] | 21 nt |
| | SEQ ID NO: 194 | AGGACACAGCACUGUUAGA[dT][dT] | 21 nt |

| **Hotspot 6** | | | |
|---|---|---|---|
| RAG-423 | SEQ ID NO: 195 | UCUUUUCAGCUGCAUUGGG[dT] [dT] | 21 nt |
| | SEQ ID NO: 196 | CCCAAU GCAGCU GAAAAGA[dT] [dT] | 21 nt |
| RAG-424 | SEQ ID NO: 197 | AUCUUUUCAGCUGCAUUGG[dT] [dT] | 21 nt |
| | SEQ ID NO: 198 | CCAAUGCAGCUGAAAAGAU[dT][dT] | 21 nt |
| RAG-425 | SEQ ID NO: 199 | GAUCUUUUCAGCUGCAUUG[dT][dT] | 21 nt |
| | SEQ ID NO: 200 | CAAUGCAGCUGAAAAGAUC[dT][dT] | 21 nt |
| RAG-426 | SEQ ID NO: 201 | UGAUCUUUUCAGCUGCAUU[dT] [dT] | 21 nt |
| | SEQ ID NO: 202 | AAUGCAGCUGAAAAGAUCA[dT] [dT] | 21 nt |
| RAG-427 | SEQ ID NO: 203 | CUGAUCUUUUCAGCUGCAU[dT][dT] | 21 nt |
| | SEQ ID NO: 204 | AUGCAGCUGAAAAGAUCAG[dT] [dT] | 21 nt |
| RAG-428 | SEQ ID NO: 205 | CCUGAUCUUUUCAGCUGCA[dT][dT] | 21 nt |
| | SEQ ID NO: 206 | UGCAGCUGAAAAGAUCAGG[dT] [dT] | 21 nt |
| RAG-429 | SEQ ID NO: 207 | UCCUGAUCUUUUCAGCUGC[dT] [dT] | 21 nt |
| | SEQ ID NO: 208 | GCAGCUGAAAAGAUCAGGA[dT][dT] | 21 nt |
| RAG-430 | SEQ ID NO: 209 | CUCCUGAUCUUUUCAGCUG[dT][dT] | 21 nt |
| | SEQ ID NO: 210 | CAGCUGAAAAGAUCAGGAG[dT] [dT] | 21 nt |
| RAG-431 | SEQ ID NO: 211 | CCUCCUGAUCUUUUCAGCU[dT][dT] | 21 nt |
| | SEQ ID NO: 212 | AGCUGAAAAGAUCAGGAGG[dT] [dT] | 21 nt |
| RAG-432 | SEQ ID NO: 213 | UCCUCCUGAUCUUUUCAGC[dT] [dT] | 21 nt |
| | SEQ ID NO: 214 | GCUGAAAAGAUCAGGAGGA[dT] [dT] | 21 nt |
| RAG-433 | SEQ ID NO: 215 | AUCCUCCUGAUCUUUUCAG[dT][dT] | 21 nt |
| | SEQ ID NO: 216 | CUGAAAAGAUCAGGAGGAU[dT][dT] | 21 nt |
| RAG-434 | SEQ ID NO: 217 | CAUCCUCCUGAUCUUUUCA[dT][dT] | 21 nt |
| | SEQ ID NO: 218 | UGAAAAGAUCAGGAGGAUG[dT][dT] | 21 nt |
| RAG-435 | SEQ ID NO: 219 | UCAUCCUCCUGAUCUUUUC[dT][dT] | 21 nt |
| | SEQ ID NO: 220 | GAAAAGAUCAGGAGGAUGA[dT][dT] | 21 nt |
| RAG-436 | SEQ ID NO: 221 | GUCAUCCUCCUGAUCUUUU[dT][dT] | 21 nt |
| | SEQ ID NO: 222 | AAAAGAUCAGGAGGAUGAC[dT][dT] | 21 nt |
| RAG-437 | SEQ ID NO: 223 | UGUCAUCCUCCUGAUCUUU[dT][dT] | 21 nt |
| | SEQ ID NO: 224 | AAAGAUCAGGAGGAUGACA[dT][dT] | 21 nt |
| RAG-438 | SEQ ID NO: 225 | AUGUCAUCCUCCUGAUCUU[dT][dT] | 21 nt |
| | SEQ ID NO: 226 | AAGAUCAGGAGGAUGACAU[dT][dT] | 21 nt |
| RAG-439 | SEQ ID NO: 227 | AAUGUCAUCCUCCUGAUCU[dT][dT] | 21 nt |
| | SEQ ID NO: 228 | AGAUCAGGAGGAUGACAUU[dT][dT] | 21 nt |
| RAG-440 | SEQ ID NO: 229 | UAAUGUCAUCCUCCUGAUC[dT][dT] | 21 nt |
| | SEQ ID NO: 230 | GAUCAGGAGGAUGACAUU A[dT] [dT] | 21 nt |
| RAG-441 | SEQ ID NO: 231 | UUAAUGUCAUCCUCCUGAU[dT] [dT] | 21 nt |
| | SEQ ID NO: 232 | AUCAGGAGGAUGACAUUAA[dT] [dT] | 21 nt |
| RAG-442 | SEQ ID NO: 233 | AUUAAUGUCAUCCUCCUGA[dT][dT] | 21 nt |
| | SEQ ID NO: 234 | UCAGGAGGAUGACAUUAAU[dT][dT] | 21 nt |

| **Hotspot 5** | | | |
|---|---|---|---|
| RAG-503 | SEQ ID NO: 235 | UGGGGCUUUUCUGGAAAUU[dT] [dT] | 21 nt |
| | SEQ ID NO: 236 | AAUUUCCAGAAAAGCCCCA[dT] [dT] | 21 nt |
| RAG-504 | SEQ ID NO: 237 | GUGGGGCUUUUCUGGAAAU[dT][dT] | 21 nt |
| | SEQ ID NO: 238 | AUUUCCAGAAAAGCCCCAC[dT][dT] | 21 nt |
| RAG-505 | SEQ ID NO: 239 | UGUGGGGCUUUUCUGGAAA[dT] [dT] | 21 nt |
| | SEQ ID NO: 240 | UUUCCAGAAAAGCCCCACA[dT] [dT] | 21 nt |
| RAG-506 | SEQ ID NO: 241 | UUGUGGGGCUUUUCUGGAA[dT] [dT] | 21 nt |
| | SEQ ID NO: 242 | UUCCAGAAAAGCCCCACAA[dT] [dT] | 21 nt |
| RAG-507 | SEQ ID NO: 243 | AUUGUGGGGCUUUUCUGGA[dT] [dT] | 21 nt |
| | SEQ ID NO: 244 | UCCAGAAAAGCCCCACAAU[dT] [dT] | 21 nt |
| RAG-508 | SEQ ID NO: 245 | UAUUGUGGGGCUUUUCUGG[dT][dT] | 21 nt |
| | SEQ ID NO: 246 | CCAGAAAAGCCCCACAAUA[dT][dT] | 21 nt |
| RAG-509 | SEQ ID NO: 247 | AUAUUGUGGGGCUUUUCUG[dT] [dT] | 21 nt |
| | SEQ ID NO: 248 | CAGAAAAGCCCCACAAUAU [dT] [dT] | 21 nt |
| RAG-510 | SEQ ID NO: 249 | GAUAUUGUGGGGCUUUUCU[dT][dT] | 21 nt |
| | SEQ ID NO: 250 | AGAAAAGCCCCACAAUAUC[dT][dT] | 21 nt |
| RAG-511 | SEQ ID NO: 251 | UGAUAUUGUGGGGCUUUUC[dT][dT] | 21 nt |
| | SEQ ID NO: 252 | GAAAAGCCCCACAAUAUCA[dT][dT] | 21 nt |
| RAG-512 | SEQ ID NO: 253 | GUGAUAUUGUGGGGCUUUU[dT][dT] | 21 nt |
| | SEQ ID NO: 254 | AAAAGCCCCACAAUAUCAC[dT] [dT] | 21 nt |
| RAG-513 | SEQ ID NO: 255 | GGUGAUAUUGUGGGGCUUU[dT][dT] | 21 nt |
| | SEQ ID NO: 256 | AAAGCCCCACAAUAUCACC[dT][dT] | 21 nt |
| RAG-514 | SEQ ID NO: 257 | AGGUGAUAUUGUGGGGCUU[dT] [dT] | 21 nt |
| | SEQ ID NO: 258 | AAGCCCCACAAUAUCACCU[dT] [dT] | 21 nt |

| **Title** | **Sequence No.** | **Sequence (5'-3')** | **Length** |
|---|---|---|---|
| **Hotspot 4** | | | |
| RAG-522 | SEQ ID NO: 259 | GGGAAUAGAGGUGAUAUUG[dT][dT] | 21 nt |
| | SEQ ID NO: 260 | CAAUAUCACCUCUAUUCCC[dT][dT] | 21 nt |
| RAG-523 | SEQ ID NO: 261 | UGGGAAUAGAGGUGAUAUU[dT][dT] | 21 nt |
| | SEQ ID NO: 262 | AAUAUCACCUCUAUUCCCA[dT][dT] | 21 nt |
| RAG-524 | SEQ ID NO: 263 | GUGGGAAUAGAGGUGAUAU[dT][dT] | 21 nt |
| | SEQ ID NO: 264 | AUAUCACCUCUAUUCCCAC[dT][dT] | 21 nt |
| RAG-525 | SEQ ID NO: 265 | AGUGGGAAUAGAGGUGAUA[dT] [dT] | 21 nt |
| | SEQ ID NO: 266 | UAUCACCUCUAUUCCCACU[dT] [dT] | 21 nt |
| RAG-526 | SEQ ID NO: 267 | CAGUGGGAAUAGAGGUGAU[dT][dT] | 21 nt |
| | SEQ ID NO: 268 | AUCACCUCUAUUCCCACUG[dT] [dT] | 21 nt |
| RAG-527 | SEQ ID NO: 269 | UCAGUGGGAAUAGAGGUGA[dT] [dT] | 21 nt |
| | SEQ ID NO: 270 | UCACCUCUAUUCCCACUGA[dT] [dT] | 21 nt |
| RAG-528 | SEQ ID NO: 271 | AUCAGUGGGAAUAGAGGUG[dT][dT] | 21 nt |
| | SEQ ID NO: 272 | CACCUCUAUUCCCACUGAU[dT][dT] | 21 nt |
| RAG-529 | SEQ ID NO: 273 | GAUCAGUGGGAAUAGAGGU[dT][dT] | 21 nt |
| | SEQ ID NO: 274 | ACCUCUAUUCCCACUGAUC[dT][dT] | 21 nt |
| RAG-530 | SEQ ID NO: 275 | GGAUCAGUGGGAAUAGAGG[dT][dT] | 21 nt |
| | SEQ ID NO: 276 | CCUCUAUUCCCACUGAUCC[dT][dT] | 21 nt |
| RAG-531 | SEQ ID NO: 277 | GGGAUCAGUGGGAAUAGAG[dT][dT] | 21 nt |
| | SEQ ID NO: 278 | CUCUAUUCCCACUGAUCCC[dT][dT] | 21 nt |
| RAG-532 | SEQ ID NO: 279 | AGGGAUCAGUGGGAAUAGA[dT][dT] | 21 nt |
| | SEQ ID NO: 280 | UCUAUUCCCACUGAUCCCU[dT][dT] | 21 nt |
| RAG-533 | SEQ ID NO: 281 | GAGGGAUCAGUGGGAAUAG[dT] [dT] | 21 nt |
| | SEQ ID NO: 282 | CUAUUCCCACUGAUCCCUC[dT][dT] | 21 nt |
| RAG-534 | SEQ ID NO: 283 | UGAGGGAUCAGUGGGAAUA[dT][dT] | 21 nt |
| | SEQ ID NO: 284 | UAUUCCCACUGAUCCCUCA[dT][dT] | 21 nt |
| RAG-535 | SEQ ID NO: 285 | GUGAGGGAUCAGUGGGAAU[dT][dT] | 21 nt |
| | SEQ ID NO: 286 | AUUCCCACUGAUCCCUCAC[dT][dT] | 21 nt |
| RAG-536 | SEQ ID NO: 287 | AGUGAGGGAUCAGUGGGAA[dT] [dT] | 21 nt |
| | SEQ ID NO: 288 | UUCCCACUGAUCCCUCACU[dT][dT] | 21 nt |
| RAG-537 | SEQ ID NO: 289 | UAGUGAGGGAUCAGUGGGA[dT][dT] | 21 nt |
| | SEQ ID NO: 290 | UCCCACUGAUCCCUCACUA[dT][dT] | 21 nt |
| RAG-538 | SEQ ID NO: 291 | CUAGUGAGGGAUCAGUGGG[dT][dT] | 21 nt |
| | SEQ ID NO: 292 | CCCACUGAUCCCUCACUAG[dT] [dT] | 21 nt |
| RAG-540 | SEQ ID NO: 293 | ACCUAGUGAGGGAUCAGUG[dT] [dT] | 21 nt |
| | SEQ ID NO: 294 | CACUGAUCCCUCACUAGGU [dT] [dT] | 21 nt |
| RAG-541 | SEQ ID NO: 295 | GACCUAGUGAGGGAUCAGU[dT][dT] | 21 nt |
| | SEQ ID NO: 296 | ACUGAUCCCUCACUAGGUC[dT][dT] | 21 nt |
| RAG-542 | SEQ ID NO: 297 | UGACCUAGUGAGGGAUCAG[dT] [dT] | 21 nt |
| | SEQ ID NO: 298 | CUGAUCCCUCACUAGGUCA[dT][dT] | 21 nt |
| RAG-543 | SEQ ID NO: 299 | GUGACCUAGUGAGGGAUCA[dT][dT] | 21 nt |
| | SEQ ID NO: 300 | UGAUCCCUCACUAGGUCAC[dT][dT] | 21 nt |
| RAG-544 | SEQ ID NO: 301 | GGUGACCUAGUGAGGGAUC[dT][dT] | 21 nt |
| | SEQ ID NO: 302 | GAUCCCUCACUAGGUCACC[dT][dT] | 21 nt |
| RAG-545 | SEQ ID NO: 303 | AGGUGACCUAGUGAGGGAU[dT] [dT] | 21 nt |
| | SEQ ID NO: 304 | AUCCCUCACUAGGUCACCU[dT] [dT] | 21 nt |
| RAG-546 | SEQ ID NO: 305 | GAGGUGACCUAGUGAGGGA[dT][dT] | 21 nt |
| | SEQ ID NO: 306 | UCCCUCACUAGGUCACCUC[dT][dT] | 21 nt |
| RAG-549 | SEQ ID NO: 307 | GGAGAGGUGACCUAGUGAG[dT] [dT] | 21 nt |
| | SEQ ID NO: 308 | CUCACUAGGUCACCUCUCC[dT][dT] | 21 nt |
| RAG-550 | SEQ ID NO: 309 | GGGAGAGGUGACCUAGUGA[dT][dT] | 21 nt |
| | SEQ ID NO: 310 | UCACUAGGUCACCUCUCCC[dT] [dT] | 21 nt |
| RAG-551 | SEQ ID NO: 311 | UGGGAGAGGUGACCUAGUG[dT][dT] | 21 nt |
| | SEQ ID NO: 312 | CACUAGGUCACCUCUCCCA[dT][dT] | 21 nt |
| RAG-552 | SEQ ID NO: 313 | CUGGGAGAGGUGACCUAGU[dT][dT] | 21 nt |
| | SEQ ID NO: 314 | ACUAGGUCACCUCUCCCAG[dT] [dT] | 21 nt |
| RAG-553 | SEQ ID NO: 315 | UCUGGGAGAGGUGACCUAG[dT] [dT] | 21 nt |
| | SEQ ID NO: 316 | CU AGGUCACCUCUCCCAGA[dT] [dT] | 21 nt |
| RAG-554 | SEQ ID NO: 317 | UUCUGGGAGAGGUGACCU A[dT] [dT] | 21 nt |
| | SEQ ID NO: 318 | UAGGUCACCUCUCCCAGAA[dT] [dT] | 21 nt |

| **Hotspot 3** | | | |
|---|---|---|---|
| RAG-569 | SEQ ID NO: 319 | AGGUGCUCCAGGUGCUUCU[dT] [dT] | 21 nt |
| | SEQ ID NO: 320 | AGAAGCACCUGGAGCACCU[dT] [dT] | 21 nt |
| RAG-570 | SEQ ID NO: 321 | UAGGUGCUCCAGGUGCUUC[dT][dT] | 21 nt |
| | SEQ ID NO: 322 | GAAGCACCUGGAGCACCUA[dT][dT] | 21 nt |
| RAG-574 | SEQ ID NO: 323 | UGUCUAGGUGCUCCAGGUG[dT][dT] | 21 nt |
| | SEQ ID NO: 324 | CACCUGGAGCACCUAGACA[dT][dT] | 21 nt |
| RAG-576 | SEQ ID NO: 325 | GGUGUCUAGGUGCUCCAGG[dT][dT] | 21 nt |
| | SEQ ID NO: 326 | CCUGGAGCACCUAGACACC[dT][dT] | 21 nt |
| RAG-577 | SEQ ID NO: 327 | GGGUGUCUAGGUGCUCCAG[dT][dT] | 21 nt |
| | SEQ ID NO: 328 | CUGGAGCACCUAGACACCC[dT][dT] | 21 nt |
| RAG-578 | SEQ ID NO: 329 | GGGGUGUCUAGGUGCUCCA[dT][dT] | 21 nt |
| | SEQ ID NO: 330 | UGGAGCACCUAGACACCCC[dT][dT] | 21 nt |
| RAG-579 | SEQ ID NO: 331 | UGGGGUGUCUAGGUGCUCC[dT] [dT] | 21 nt |
| | SEQ ID NO: 332 | GGAGCACCUAGACACCCCA[dT] [dT] | 21 nt |
| RAG-580 | SEQ ID NO: 333 | UUGGGGUGUCUAGGUGCUC[dT][dT] | 21 nt |
| | SEQ ID NO: 334 | GAGCACCUAGACACCCCAA[dT][dT] | 21 nt |
| RAG-583 | SEQ ID NO: 335 | UUGUUGGGGUGUCUAGGUG[dT][dT] | 21 nt |
| | SEQ ID NO: 336 | CACCUAGACACCCCAACAA[dT] [dT] | 21 nt |
| RAG-584 | SEQ ID NO: 337 | UUUGUUGGGGUGUCUAGGU[dT] [dT] | 21 nt |
| | SEQ ID NO: 338 | ACCUAGACACCCCAACAAA[dT] [dT] | 21 nt |
| RAG-585 | SEQ ID NO: 339 | CUUUGUUGGGGUGUCUAGG[dT][dT] | 21 nt |
| | SEQ ID NO: 340 | CCUAGACACCCCAACAAAG[dT][dT] | 21 nt |

| **Hotspot 2** | | | |
|---|---|---|---|
| RAG-650 | SEQ ID NO: 341 | AGUGGACCUCAAUUUCCUC[dT][dT] | 21 nt |
| | SEQ ID NO: 342 | GAGGAAAUUGAGGUCCACU[dT][dT] | 21 nt |
| RAG-651 | SEQ ID NO: 343 | CAGUGGACCUCAAUUUCCU[dT][dT] | 21 nt |
| | SEQ ID NO: 344 | AGGAAAUUGAGGUCCACUG[dT][dT] | 21 nt |
| RAG-652 | SEQ ID NO: 345 | UCAGUGGACCUCAAUUUCC[dT][dT] | 21 nt |
| | SEQ ID NO: 346 | GGAAAUUGAGGUCCACUGA[dT][dT] | 21 nt |
| RAG-653 | SEQ ID NO: 347 | UUCAGUGGACCUCAAUUUC[dT][dT] | 21 nt |
| | SEQ ID NO: 348 | GAAAUUGAGGUCCACUGAA[dT][dT] | 21 nt |
| RAG-654 | SEQ ID NO: 349 | GUUCAGUGGACCUCAAUUU[dT][dT] | 21 nt |
| | SEQ ID NO: 350 | AAAUUGAGGUCCACUGAAC[dT][dT] | 21 nt |
| RAG-655 | SEQ ID NO: 351 | AGUUCAGUGGACCUCAAUU[dT][dT] | 21 nt |
| | SEQ ID NO: 352 | AAUUGAGGUCCACUGAACU[dT][dT] | 21 nt |
| RAG-656 | SEQ ID NO: 353 | AAGUUCAGUGGACCUCAAU[dT][dT] | 21 nt |
| | SEQ ID NO: 354 | AUUGAGGUCCACUGAACUU[dT][dT] | 21 nt |
| RAG-657 | SEQ ID NO: 355 | UAAGUUCAGUGGACCUCAA[dT][dT] | 21 nt |
| | SEQ ID NO: 356 | UUGAGGUCCACUGAACUUA[dT][dT] | 21 nt |
| RAG-658 | SEQ ID NO: 357 | UUAAGUUCAGUGGACCUCA[dT] [dT] | 21 nt |
| | SEQ ID NO: 358 | UGAGGUCCACUGAACUUAA[dT] [dT] | 21 nt |
| RAG-659 | SEQ ID NO: 359 | CUUAAGUUCAGUGGACCUC[dT] [dT] | 21 nt |
| | SEQ ID NO: 360 | GAGGUCCACUGAACUUAAG[dT] [dT] | 21 nt |
| RAG-660 | SEQ ID NO: 361 | ACUUAAGUUCAGUGGACCU[dT][dT] | 21 nt |
| | SEQ ID NO: 362 | AGGUCCACUGAACUUAAGU[dT][dT] | 21 nt |
| RAG-661 | SEQ ID NO: 363 | UACUUAAGUUCAGUGGACC[dT] [dT] | 21 nt |
| | SEQ ID NO: 364 | GGUCCACUGAACUUAAGUA[dT][dT] | 21 nt |
| RAG-662 | SEQ ID NO: 365 | AUACUUAAGUUCAGUGGAC[dT][dT] | 21 nt |
| | SEQ ID NO: 366 | GUCCACUGAACUUAAGUAU[dT][dT] | 21 nt |
| RAG-682 | SEQ ID NO: 367 | GUGACAAUCAACAACUUUG[dT][dT] | 21 nt |
| | SEQ ID NO: 368 | CAAAGUUGUUGAUUGUCAC[dT][dT] | 21 nt |
| RAG-685 | SEQ ID NO: 369 | CAUGUGACAAUCAACAACU [dT] [dT] | 21 nt |
| | SEQ ID NO: 370 | AGUUGUUGAUUGUCACAUG[dT] [dT] | 21 nt |
| RAG-686 | SEQ ID NO: 371 | GCAUGUGACAAUCAACAAC[dT][dT] | 21 nt |
| | SEQ ID NO: 372 | GUUGUUGAUUGUCACAUGC[dT][dT] | 21 nt |
| RAG-687 | SEQ ID NO: 373 | AGCAUGUGACAAUCAACAA[dT] [dT] | 21 nt |
| | SEQ ID NO: 374 | UUGUUGAUUGUCACAUGCU[dT][dT] | 21 nt |
| RAG-688 | SEQ ID NO: 375 | AAGCAUGUGACAAUCAACA[dT] [dT] | 21 nt |
| | SEQ ID NO: 376 | UGUUGAUUGUCACAUGCUU[dT][dT] | 21 nt |
| RAG-689 | SEQ ID NO: 377 | GAAGCAUGUGACAAUCAAC[dT][dT] | 21 nt |
| | SEQ ID NO: 378 | GUUGAUUGUCACAUGCUUC[dT][dT] | 21 nt |
| RAG-690 | SEQ ID NO: 379 | GGAAGCAUGUGACAAUCAA[dT][dT] | 21 nt |
| | SEQ ID NO: 380 | UUGAUUGUCACAUGCUUCC[dT][dT] | 21 nt |
| RAG-691 | SEQ ID NO: 381 | CGGAAGCAUGUGACAAUCA[dT][dT] | 21 nt |
| | SEQ ID NO: 382 | UGAUUGUCACAUGCUUCCG[dT][dT] | 21 nt |
| RAG-692 | SEQ ID NO: 383 | CCGGAAGCAUGUGACAAUC[dT][dT] | 21 nt |
| | SEQ ID NO: 384 | GAUUGUCACAUGCUUCCGG[dT][dT] | 21 nt |
| RAG-693 | SEQ ID NO: 385 | CCCGGAAGCAUGUGACAAU[dT][dT] | 21 nt |
| | SEQ ID NO: 386 | AUUGUCACAUGCUUCCGGG[dT][dT] | 21 nt |
| RAG-694 | SEQ ID NO: 387 | UCCCGGAAGCAUGUGACAA[dT] [dT] | 21 nt |
| | SEQ ID NO: 388 | UUGUCACAUGCUUCCGGGA[dT] [dT] | 21 nt |
| RAG-695 | SEQ ID NO: 389 | UUCCCGGAAGCAUGUGACA[dT] [dT] | 21 nt |
| | SEQ ID NO: 390 | UGUCACAUGCUUCCGGGAA[dT] [dT] | 21 nt |
| RAG-696 | SEQ ID NO: 391 | CUUCCCGGAAGCAUGUGAC[dT][dT] | 21 nt |
| | SEQ ID NO: 392 | GUCACAUGCUUCCGGGAAG[dT][dT] | 21 nt |
| RAG-697 | SEQ ID NO: 393 | CCUUCCCGGAAGCAUGUGA[dT] [dT] | 21 nt |
| | SEQ ID NO: 394 | UCACAUGCUUCCGGGAAGG[dT] [dT] | 21 nt |
| RAG-698 | SEQ ID NO: 395 | UCCUUCCCGGAAGCAUGUG[dT] [dT] | 21 nt |
| | SEQ ID NO: 396 | CACAUGCUUCCGGGAAGGA[dT] [dT] | 21 nt |
| RAG-699 | SEQ ID NO: 397 | CUCCUUCCCGGAAGCAUGU[dT][dT] | 21 nt |
| | SEQ ID NO: 398 | ACAUGCUUCCGGGAAGGAG[dT] [dT] | 21 nt |
| RAG-700 | SEQ ID NO: 399 | CCUCCUUCCCGGAAGCAUG[dT][dT] | 21 nt |
| | SEQ ID NO: 400 | CAUGCUUCCGGGAAGGAGG[dT][dT] | 21 nt |
| RAG-701 | SEQ ID NO: 401 | CCCUCCUUCCCGGAAGCAU[dT][dT] | 21 nt |
| | SEQ ID NO: 402 | AUGCUUCCGGGAAGGAGGG[dT][dT] | 21 nt |
| RAG-702 | SEQ ID NO: 403 | UCCCUCCUUCCCGGAAGCA[dT][dT] | 21 nt |
| | SEQ ID NO: 404 | UGCUUCCGGGAAGGAGGGA[dT][dT] | 21 nt |
| RAG-704 | SEQ ID NO: 405 | AUUCCCUCCUUCCCGGAAG[dT][dT] | 21 nt |
| | SEQ ID NO: 406 | CUUCCGGGAAGGAGGGAAU[dT][dT] | 21 nt |
| RAG-705 | SEQ ID NO: 407 | AAUUCCCUCCUUCCCGGAA[dT][dT] | 21 nt |
| | SEQ ID NO: 408 | UUCCGGGAAGGAGGGAAUU[dT][dT] | 21 nt |
| RAG-710 | SEQ ID NO: 409 | UCUCCAAUUCCCUCCUUCC[dT] [dT] | 21 nt |
| | SEQ ID NO: 410 | GGAAGGAGGGAAUUGGAGA[dT] [dT] | 21 nt |
| RAG-711 | SEQ ID NO: 411 | CUCUCCAAUUCCCUCCUUC[dT][dT] | 21 nt |
| | SEQ ID NO: 412 | GAAGGAGGGAAUUGGAGAG[dT][dT] | 21 nt |
| RAG-712 | SEQ ID NO: 413 | UCUCUCCAAUUCCCUCCUU[dT][dT] | 21 nt |
| | SEQ ID NO: 414 | AAGGAGGGAAUUGGAGAGA[dT][dT] | 21 nt |
| RAG-713 | SEQ ID NO: 415 | GUCUCUCCAAUUCCCUCCU[dT][dT] | 21 nt |
| | SEQ ID NO: 416 | AGGAGGGAAUUGGAGAGAC[dT][dT] | 21 nt |
| RAG-714 | SEQ ID NO: 417 | AGUCUCUCCAAUUCCCUCC[dT][dT] | 21 nt |
| | SEQ ID NO: 418 | GGAGGGAAUUGGAGAGACU[dT][dT] | 21 nt |
| RAG-715 | SEQ ID NO: 419 | UAGUCUCUCCAAUUCCCUC[dT][dT] | 21 nt |
| | SEQ ID NO: 420 | GAGGGAAUUGGAGAGACU A[dT] [dT] | 21 nt |
| RAG-716 | SEQ ID NO: 421 | GUAGUCUCUCCAAUUCCCU[dT][dT] | 21 nt |
| | SEQ ID NO: 422 | AGGGAAUUGGAGAGACUAC[dT][dT] | 21 nt |
| RAG-717 | SEQ ID NO: 423 | GGUAGUCUCUCCAAUUCCC[dT][dT] | 21 nt |
| | SEQ ID NO: 424 | GGGAAUUGGAGAGACUACC[dT][dT] | 21 nt |

| **Hotspot 1** | | | |
|---|---|---|---|
| RAG-820 | SEQ ID NO: 425 | UGCAACCACAGGGAUUUCU[dT] [dT] | 21 nt |
| | SEQ ID NO: 426 | AGAAAUCCCUGUGGUUGCA[dT] [dT] | 21 nt |
| RAG-821 | SEQ ID NO: 427 | CUGCAACCACAGGGAUUUC[dT][dT] | 21 nt |
| | SEQ ID NO: 428 | GAAAUCCCUGUGGUUGCAG[dT][dT] | 21 nt |
| RAG-822 | SEQ ID NO: 429 | GCUGCAACCACAGGGAUUU[dT][dT] | 21 nt |
| | SEQ ID NO: 430 | AAAUCCCUGUGGUUGCAGC[dT][dT] | 21 nt |
| RAG-823 | SEQ ID NO: 431 | UGCUGCAACCACAGGGAUU[dT][dT] | 21 nt |
| | SEQ ID NO: 432 | AAUCCCUGUGGUUGCAGCA[dT][dT] | 21 nt |
| RAG-824 | SEQ ID NO: 433 | CUGCUGCAACCACAGGGAU[dT][dT] | 21 nt |
| | SEQ ID NO: 434 | AUCCCUGUGGUUGCAGCAG[dT][dT] | 21 nt |
| RAG-825 | SEQ ID NO: 435 | GCUGCUGCAACCACAGGGA[dT][dT] | 21 nt |
| | SEQ ID NO: 436 | UCCCUGUGGUUGCAGCAGC[dT] [dT] | 21 nt |
| RAG-826 | SEQ ID NO: 437 | AGCUGCUGCAACCACAGGG[dT] [dT] | 21 nt |
| | SEQ ID NO: 438 | CCCUGUGGUUGCAGCAGCU[dT][dT] | 21 nt |

| **Title** | **Sequence No.** | **Sequence (5'-3')** | **Length** |
|---|---|---|---|
| RAG-828 | SEQ ID NO: 439 | AAAGCUGCUGCAACCACAG[dT] [dT] | 21 nt |
| | SEQ ID NO: 440 | CUGUGGUUGCAGCAGCUUU[dT][dT] | 21 nt |
| RAG-829 | SEQ ID NO: 441 | CAAAGCUGCUGCAACCACA[dT] [dT] | 21 nt |
| | SEQ ID NO: 442 | UGUGGUUGCAGCAGCUUUG[dT] [dT] | 21 nt |
| RAG-830 | SEQ ID NO: 443 | ACAAAGCUGCUGCAACCAC[dT][dT] | 21 nt |
| | SEQ ID NO: 444 | GUGGUUGCAGCAGCUUUGU[dT][dT] | 21 nt |
| RAG-831 | SEQ ID NO: 445 | AACAAAGCUGCUGCAACCA[dT] [dT] | 21 nt |
| | SEQ ID NO: 446 | UGGUUGCAGCAGCUUUGUU[dT] [dT] | 21 nt |
| RAG-832 | SEQ ID NO: 447 | CAACAAAGCUGCUGCAACC[dT][dT] | 21 nt |
| | SEQ ID NO: 448 | GGUUGCAGCAGCUUUGUUG[dT] [dT] | 21 nt |
| RAG-833 | SEQ ID NO: 449 | CCAACAAAGCUGCUGCAAC[dT][dT] | 21 nt |
| | SEQ ID NO: 450 | GUUGCAGCAGCUUUGUUGG[dT][dT] | 21 nt |
| RAG-834 | SEQ ID NO: 451 | GCCAACAAAGCUGCUGCAA[dT] [dT] | 21 nt |
| | SEQ ID NO: 452 | UUGCAGCAGCUUUGUUGGC[dT] [dT] | 21 nt |
| RAG-835 | SEQ ID NO: 453 | GGCCAACAAAGCUGCUGCA[dT] [dT] | 21 nt |
| | SEQ ID NO: 454 | UGCAGCAGCUUUGUUGGCC[dT][dT] | 21 nt |
| RAG-836 | SEQ ID NO: 455 | UGGCCAACAAAGCUGCUGC[dT][dT] | 21 nt |
| | SEQ ID NO: 456 | GCAGCAGCUUUGUUGGCCA[dT][dT] | 21 nt |
| RAG-837 | SEQ ID NO: 457 | CUGGCCAACAAAGCUGCUG[dT][dT] | 21 nt |
| | SEQ ID NO: 458 | CAGCAGCUUUGUUGGCCAG[dT][dT] | 21 nt |
| RAG-838 | SEQ ID NO: 459 | CCUGGCCAACAAAGCUGCU[dT][dT] | 21 nt |
| | SEQ ID NO: 460 | AGCAGCUUUGUUGGCCAGG[dT] [dT] | 21 nt |
| RAG-840 | SEQ ID NO: 461 | UUCCUGGCCAACAAAGCUG[dT] [dT] | 21 nt |
| | SEQ ID NO: 462 | CAGCUUUGUUGGCCAGGAA[dT][dT] | 21 nt |
| RAG-841 | SEQ ID NO: 463 | CUUCCUGGCCAACAAAGCU[dT][dT] | 21 nt |
| | SEQ ID NO: 464 | AGCUUUGUUGGCCAGGAAG[dT][dT] | 21 nt |
| RAG-843 | SEQ ID NO: 465 | CCCUUCCUGGCCAACAAAG[dT] [dT] | 21 nt |
| | SEQ ID NO: 466 | CUUUGUUGGCCAGGAAGGG[dT] [dT] | 21 nt |
| RAG-844 | SEQ ID NO: 467 | CCCCUUCCUGGCCAACAAA[dT][dT] | 21 nt |
| | SEQ ID NO: 468 | UUUGUUGGCCAGGAAGGGG[dT][dT] | 21 nt |
| RAG-845 | SEQ ID NO: 469 | UCCCCUUCCUGGCCAACAA[dT] [dT] | 21 nt |
| | SEQ ID NO: 470 | UUGUUGGCCAGGAAGGGGA[dT] [dT] | 21 nt |
| RAG-846 | SEQ ID NO: 471 | CUCCCCUUCCUGGCCAACA[dT] [dT] | 21 nt |
| | SEQ ID NO: 472 | UGUUGGCCAGGAAGGGGAG[dT] [dT] | 21 nt |
| RAG-848 | SEQ ID NO: 473 | UCCUCCCCUUCCUGGCCAA[dT] [dT] | 21 nt |
| | SEQ ID NO: 474 | UUGGCCAGGAAGGGGAGGA[dT] [dT] | 21 nt |
| RAG-849 | SEQ ID NO: 475 | AUCCUCCCCUUCCUGGCCA[dT][dT] | 21 nt |
| | SEQ ID NO: 476 | UGGCCAGGAAGGGGAGGAU[dT][dT] | 21 nt |
| RAG-853 | SEQ ID NO: 477 | UCAAAUCCUCCCCUUCCUG[dT] [dT] | 21 nt |
| | SEQ ID NO: 478 | CAGGAAGGGGAGGAUUUGA[dT] [dT] | 21 nt |
| RAG-854 | SEQ ID NO: 479 | GUCAAAUCCUCCCCUUCCU[dT][dT] | 21 nt |
| | SEQ ID NO: 480 | AGGAAGGGGAGGAUUUGAC[dT][dT] | 21 nt |
| RAG-855 | SEQ ID NO: 481 | CGUCAAAUCCUCCCCUUCC[dT][dT] | 21 nt |
| | SEQ ID NO: 482 | GGAAGGGGAGGAUUUGACG[dT] [dT] | 21 nt |
| RAG-856 | SEQ ID NO: 483 | UCGUCAAAUCCUCCCCUUC[dT][dT] | 21 nt |
| | SEQ ID NO: 484 | GAAGGGGAGGAUUUGACGA[dT][dT] | 21 nt |
| RAG-857 | SEQ ID NO: 485 | CUCGUCAAAUCCUCCCCUU[dT][dT] | 21 nt |
| | SEQ ID NO: 486 | AAGGGGAGGAUUUGACGAG[dT] [dT] | 21 nt |
| RAG-858 | SEQ ID NO: 487 | ACUCGUCAAAUCCUCCCCU[dT][dT] | 21 nt |
| | SEQ ID NO: 488 | AGGGGAGGAUUUGACGAGU[dT][dT] | 21 nt |
| RAG-860 | SEQ ID NO: 489 | UCACUCGUCAAAUCCUCCC[dT] [dT] | 21 nt |
| | SEQ ID NO: 490 | GGGAGGAUUUGACGAGUGA[dT] [dT] | 21 nt |
| RAG-861 | SEQ ID NO: 491 | CUCACUCGUCAAAUCCUCC[dT][dT] | 21 nt |
| | SEQ ID NO: 492 | GGAGGAUUUGACGAGUGAG[dT][dT] | 21 nt |
| RAG-862 | SEQ ID NO: 493 | ACUCACUCGUCAAAUCCUC[dT][dT] | 21 nt |
| | SEQ ID NO: 494 | GAGGAUUUGACGAGUGAGU[dT][dT] | 21 nt |
| RAG-864 | SEQ ID NO: 495 | CAACUCACUCGUCAAAUCC[dT][dT] | 21 nt |
| | SEQ ID NO: 496 | GGAUUUGACGAGUGAGUUG[dT] [dT] | 21 nt |
| RAG-865 | SEQ ID NO: 497 | ACAACUCACUCGUCAAAUC[dT][dT] | 21 nt |
| | SEQ ID NO: 498 | GAUUUGACGAGUGAGUUGU[dT][dT] | 21 nt |
| RAG-866 | SEQ ID NO: 499 | GACAACUCACUCGUCAAAU[dT][dT] | 21 nt |
| | SEQ ID NO: 500 | AUUUGACGAGUGAGUUGUC[dT][dT] | 21 nt |
| RAG-867 | SEQ ID NO: 501 | AGACAACUCACUCGUCAAA[dT][dT] | 21 nt |
| | SEQ ID NO: 502 | UUUGACGAGUGAGUUGUCU[dT][dT] | 21 nt |
| RAG-868 | SEQ ID NO: 503 | CAGACAACUCACUCGUCAA[dT][dT] | 21 nt |
| | SEQ ID NO: 504 | UUGACGAGUGAGUUGUCUG[dT] [dT] | 21 nt |
| RAG-869 | SEQ ID NO: 505 | ACAGACAACUCACUCGUCA[dT] [dT] | 21 nt |
| | SEQ ID NO: 506 | UGACGAGUGAGUUGUCUGU[dT] [dT] | 21 nt |
| RAG-870 | SEQ ID NO: 507 | GACAGACAACUCACUCGUC[dT][dT] | 21 nt |
| | SEQ ID NO: 508 | GACGAGUGAGUUGUCUGUC[dT][dT] | 21 nt |
| RAG-871 | SEQ ID NO: 509 | AGACAGACAACUCACUCGU[dT][dT] | 21 nt |
| | SEQ ID NO: 510 | ACGAGUGAGUUGUCUGUCU[dT][dT] | 21 nt |
| RAG-872 | SEQ ID NO: 511 | GAGACAGACAACUCACUCG[dT][dT] | 21 nt |
| | SEQ ID NO: 512 | CGAGUGAGUUGUCUGUCUC[dT][dT] | 21 nt |
| RAG-873 | SEQ ID NO: 513 | GGAGACAGACAACUCACUC[dT][dT] | 21 nt |
| | SEQ ID NO: 514 | GAGUGAGUUGUCUGUCUCC[dT][dT] | 21 nt |
| RAG-874 | SEQ ID NO: 515 | AGGAGACAGACAACUCACU[dT] [dT] | 21 nt |
| | SEQ ID NO: 516 | AGUGAGUUGUCUGUCUCCU[dT][dT] | 21 nt |
| RAG-875 | SEQ ID NO: 517 | CAGGAGACAGACAACUCAC[dT] [dT] | 21 nt |
| | SEQ ID NO: 518 | GUGAGUUGUCUGUCUCCUG[dT][dT] | 21 nt |
| RAG-876 | SEQ ID NO: 519 | UCAGGAGACAGACAACUCA[dT][dT] | 21 nt |
| | SEQ ID NO: 520 | UGAGUUGUCUGUCUCCUGA[dT][dT] | 21 nt |
| RAG-877 | SEQ ID NO: 521 | UUCAGGAGACAGACAACUC[dT][dT] | 21 nt |
| | SEQ ID NO: 522 | GAGUUGUCUGUCUCCUGAA[dT][dT] | 21 nt |
| RAG-878 | SEQ ID NO: 523 | AUUCAGGAGACAGACAACU [dT] [dT] | 21 nt |
| | SEQ ID NO: 524 | AGUUGUCUGUCUCCUGAAU[dT] [dT] | 21 nt |
| RAG-879 | SEQ ID NO: 525 | UAUUCAGGAGACAGACAAC[dT][dT] | 21 nt |
| | SEQ ID NO: 526 | GUUGUCUGUCUCCUGAAU A [dT] [dT] | 21 nt |
| RAG-880 | SEQ ID NO: 527 | GUAUUCAGGAGACAGACAA[dT][dT] | 21 nt |
| | SEQ ID NO: 528 | UUGUCUGUCUCCUGAAUAC[dT][dT] | 21 nt |
| RAG-881 | SEQ ID NO: 529 | AGUAUUCAGGAGACAGACA[dT][dT] | 21 nt |
| | SEQ ID NO: 530 | UGUCUGUCUCCUGAAUACU[dT][dT] | 21 nt |
| RAG-882 | SEQ ID NO: 531 | GAGUAUUCAGGAGACAGAC[dT][dT] | 21 nt |
| | SEQ ID NO: 532 | GUCUGUCUCCUGAAUACUC[dT][dT] | 21 nt |
| RAG-883 | SEQ ID NO: 533 | GGAGUAUUCAGGAGACAGA[dT][dT] | 21 nt |
| | SEQ ID NO: 534 | UCUGUCUCCUGAAUACUCC[dT][dT] | 21 nt |
| RAG-884 | SEQ ID NO: 535 | GGGAGUAUUCAGGAGACAG[dT][dT] | 21 nt |
| | SEQ ID NO: 536 | CUGUCUCCUGAAUACUCCC[dT] [dT] | 21 nt |
| RAG-885 | SEQ ID NO: 537 | GGGGAGUAUUCAGGAGACA[dT] [dT] | 21 nt |
| | SEQ ID NO: 538 | UGUCUCCUGAAUACUCCCC[dT] [dT] | 21 nt |
| RAG-886 | SEQ ID NO: 539 | UGGGGAGUAUUCAGGAGAC[dT][dT] | 21 nt |
| | SEQ ID NO: 540 | GUCUCCUGAAUACUCCCCA[dT][dT] | 21 nt |
| RAG-887 | SEQ ID NO: 541 | GUGGGGAGUAUUCAGGAGA[dT][dT] | 21 nt |
| | SEQ ID NO: 542 | UCUCCUGAAUACUCCCCAC[dT][dT] | 21 nt |
| RAG-888 | SEQ ID NO: 543 | UGUGGGGAGUAUUCAGGAG[dT][dT] | 21 nt |
| | SEQ ID NO: 544 | CUCCUGAAUACUCCCCACA[dT][dT] | 21 nt |
| RAG-889 | SEQ ID NO: 545 | AUGUGGGGAGUAUUCAGGA[dT][dT] | 21 nt |
| | SEQ ID NO: 546 | UCCUGAAUACUCCCCACAU[dT][dT] | 21 nt |
| RAG-890 | SEQ ID NO: 547 | UAUGUGGGGAGUAUUCAGG[dT][dT] | 21 nt |
| | SEQ ID NO: 548 | CCUGAAUACUCCCCACAUA[dT][dT] | 21 nt |
| RAG-891 | SEQ ID NO: 549 | CUAUGUGGGGAGUAUUCAG[dT][dT] | 21 nt |
| | SEQ ID NO: 550 | CUGAAUACUCCCCACAUAG[dT] [dT] | 21 nt |
| RAG-892 | SEQ ID NO: 551 | GCUAUGUGGGGAGUAUUCA[dT][dT] | 21 nt |
| | SEQ ID NO: 552 | UGAAUACUCCCCACAUAGC[dT][dT] | 21 nt |
| RAG-893 | SEQ ID NO: 553 | GGCUAUGUGGGGAGUAUUC[dT][dT] | 21 nt |
| | SEQ ID NO: 554 | GAAUACUCCCCACAUAGCC[dT] [dT] | 21 nt |
| RAG-894 | SEQ ID NO: 555 | GGGCUAUGUGGGGAGUAUU[dT][dT] | 21 nt |
| | SEQ ID NO: 556 | AAUACUCCCCACAUAGCCC[dT][dT] | 21 nt |

These hotspots include: hotspot 1 having a corresponding target sequence from -893 bp to -801 bp in the p21 promoter sequence, shown as SEQ ID NO: 93, wherein 44 functional saRNAs (Table 4, Fig. 3A) were discovered in this region, comprising RAG-834, RAG-845, RAG-892, RAG-846, RAG-821, RAG-884, RAG-864, RAG-843, RAG-854, RAG-844, RAG-887, RAG-838, RAG-858, RAG-835, RAG-876, RAG-870, RAG-853, RAG-881, RAG-828, RAG-872, RAG-841, RAG-831, RAG-829, RAG-820, RAG-822, RAG-868, RAG-849, RAG-862, RAG-865, RAG-893, RAG-848, RAG-824, RAG-866, RAG-840, RAG-875, RAG-880, RAG-871, RAG-888, RAG-885, RAG-894, RAG-833, RAG-825, RAG-889, and RAG-823;
hotspot 2 (Table 4, Fig. 3B) having a corresponding target sequence from -717 bp to -632 bp in the p21 promoter sequence, shown as SEQ ID NO: 94, wherein 31 functional saRNAs were discovered in this region, comprising RAG-693, RAG-692, RAG-688, RAG-696, RAG-694, RAG-687, RAG-691, RAG-690, RAG-689, RAG-682, RAG-686, RAG-662, RAG-695, RAG-654, RAG-658, RAG-685, RAG-704, RAG-714, RAG-705, RAG-661, RAG-656, RAG-698, RAG-697, RAG-657, RAG-715, RAG-652, RAG-651, RAG-650, RAG-716, RAG-717, and RAG-711;
hotspot 3 (table 4, Fig. 3C) having a corresponding target sequence from -585 bp to -551 bp in the p21 promoter sequence, shown as SEQ ID NO: 95, wherein 9 functional saRNAs were discovered in this region, comprising RAG-580, RAG-577, RAG-569, RAG-576, RAG-570, RAG-574, RAG-585, RAG-579, and RAG-584;
hotspot 4 (Table 4, Fig. 3D) having a corresponding target sequence from -554 bp to -505 bp in the p21 promoter sequence, shown as SEQ ID NO: 96, wherein 17 functional saRNAs were discovered in this region, comprising RAG-524, RAG-553, RAG-537, RAG-526, RAG-554, RAG-523, RAG-534, RAG-543, RAG-525, RAG-535, RAG-546, RAG-545, RAG-542, RAG-531, RAG-522, RAG-529, and RAG-552;
hotspot 5 (Table 4, Fig. 3E) having a corresponding target sequence from -514 bp to -485 bp in the p21 promoter sequence, shown as SEQ ID NO: 97, wherein 9 functional saRNAs were discovered in this region, comprising RAG-503, RAG-504, RAG-505, RAG-506, RAG-507, RAG-508, RAG-509, RAG-510, RAG-511, RAG-512, RAG-513, and RAG-514;
hotspot 6 (table 4, Fig. 3F) having a corresponding target sequence from -442 bp to -405 bp in the p21 promoter sequence, shown as SEQ ID NO: 98, wherein 12 functional saRNAs were discovered in this region, comprising RAG-427, RAG-430, RAG-431, RAG-423, RAG-425, RAG-433, RAG-435, RAG-434, RAG-439, RAG-426, RAG-428, and RAG-442;
hotspot 7 (Table 4, Fig. 3G) having a corresponding target sequence from -352 bp to -313 bp in the p21 promoter sequence, shown as SEQ ID NO: 99, wherein 13 functional saRNAs were discovered in this region, comprising RAG-335, RAG-351, RAG-352, RAG-331, RAG-344, RAG-342, RAG-341, RAG-333, RAG-345, RAG-346, RAG-336, RAG-332, and RAG-343; and hotspot 8 (Table 4, Fig. 3H) having a corresponding target sequence from -325 bp to -260 bp in the p21 promoter sequence, shown as SEQ ID NO: 100, wherein 18 functional saRNAs were discovered in this region, comprising RAG-294, RAG-285, RAG-286, RAG-292, RAG-291, RAG-284, RAG-279, RAG-280, RAG-325, RAG-293, RAG-322, RAG-321, RAG-281, RAG-289, RAG-278, RAG-283, RAG-282, and RAG-295.

In order to verify the QuantiGene 2.0 assay results, the 439 saRNAs were divided into four bins according to their activity in inducing p21 mRNA expression, and 5 saRNAs were randomly selected from each bin and transfected into Ku-7-luc2-GFP cells at a concentration of 10 nM. 72 hours after transfection, total cellular RNA was extracted from the transfected cells and reverse transcribed into cDNA which was amplified by RT-qPCR to determine p21 mRNA level. p21 mRNA expression levels for cells transfected with each of the saRNA determined by the two methods showed a significant correlation (R² = 0.82) (Fig. 4). All selected functional saRNAs obtained through the QuantiGene 2.0 method were verified as real functional saRNAs by the RT-qPCR method, and some of them exhibited even a stronger p21 mRNA induction ability by RT-qPCR (Table 5).

**Table 5: Verification for QuantiGene 2.0 method**

| **saRNA group** | **saRNA** | **Relative p21 mRNA level** | |
|---|---|---|---|
| | | Quantigene 2.0 | RT-qPCR |
| Bin 1 | RAG-693 | 8.12 | 48.20 |
| | RAG-834 | 8.07 | 9.64 |
| | RAG-692 | 7.69 | 29.53 |
| | RAG-845 | 6.67 | 7.15 |
| | RAG-688 | 6.55 | 42.91 |
| Bin 2 | RAG-531 | 2.06 | 3.11 |
| | RAG-705 | 2.05 | 11.33 |
| | RAG-322 | 2.04 | 7.53 |
| | RAG-840 | 2.03 | 7.01 |
| | RAG-741 | 2.02 | 5.45 |
| Bin 3 | RAG-883 | 1.00 | 1.76 |
| | RAG-177 | 1.00 | 0.31 |
| | RAG-530 | 1.00 | 1.41 |
| | RAG-879 | 1.00 | 0.98 |
| | RAG-527 | 0.99 | 1.06 |
| Bin 4 | RAG-830 | 0.72 | 0.45 |
| | RAG-419 | 0.71 | 0.41 |
| | RAG-420 | 0.71 | 0.93 |
| | RAG-700 | 0.69 | 0.32 |
| | RAG-589 | 0.66 | 0.80 |

Taken together, the above data indicates that saRNAs can be designed to target selected regions in the p21 promoter to induce p21 expression with some regions being more sensitive and containing higher percentages of functional saRNA targets.

### Example 2: saRNAs induce p21 mRNA expression and inhibit cancer cell proliferation

In order to further evaluate the effect of p21 saRNAs in inducing p21 mRNA expression and suppressing cancer cell proliferation, the saRNAs (RAG1-431, RAG1-553 and RAG1-688) screened by QuantiGene 2.0 were transfected into cancer cell lines including Ku-7-luc2-GFP (bladder cancer), HCT116 (colon cancer) and HepG2 (hepatocellular carcinoma). The result showed that in the aforementioned cell lines, all saRNAs could induce at least a two-fold change in the p21 mRNA expression levels and suppress cell proliferation, indicating functional activation of p21 protein. Specifically, RAG-431, RAG-553, and RAG-688 were individually transfected into Ku-7-luc2-GFP cells, caused a 14.0, a 36.9, and a 31.9-fold change in the mRNA expression of p21, and exhibited a 71.7%, 60.7% and 67.4% cell survival rate respectively relative to blank control (Mock) (Fig. 5). RAG-431, RAG-553 and RAG-688 were transfected into the HCT116 cells, resulted in a 2.3, a 3.5, and a 2.4-fold change in the mRNA expression of p21, and exhibited a survival rate of 45.3%, 22.5% and 38.5% respectively relative to blank control (Mock) (Fig. 6). RAG-431, RAG-553, and RAG-688 were transfected into the HepG2 cells, resulted in a 2.2, 3.3, and a 2.0-fold change in the mRNA expression of p21, and exhibited a survival rate of 76.7%, 64.9%, and 79.9% respectively relative to blank control (Mock) (Fig. 7).

### Example 3: Further screening and optimizing saRNAs in a hotspot region

In order to screen and validate p21 activating saRNAs, a more focused screen was performed against hotspot 7 which is a 40 bp region from -332 to -292 relative to the TSS of p21. This region contains 17 overlapping 19-bp saRNA target sites after a short polyadenosine repetitive sequence was excluded (Fig. 8). Seventeen saRNAs with standard structure (19 nt symmetric duplex region with 3' dTdT overhangs) corresponding to each of the 17 targets were synthesized and named, according to their target positions relative to the TSS, as P21-297, P21-298, P21-299, P21-325, and so on. All the duplex sequences are listed in Table 1. Each of the duplexes was transfected into Ku-7-luc2-GFP (bladder urothelial carcinoma) cells individually, and 72 hours after transfection, p21 mRNA expression levels were detected. As shown in Fig. 9, multiple duplexes (i.e. P21-321, P21-322, and P21-331) are strong activators for p21, resulting in about 4- to 6-fold changes in p21 expression level, while the other duplexes did not significantly upregulate p21 expression. These data showed that the gene activation effect of a saRNA highly depends on the target location and/or their sequence thereof and a small shift of the target position (for example, one bp shift) can make a functional saRNA unfunctional.

### Example 4: Duplex saRNAs with 3' natural overhang or 2-nucleotide uracil overhang

In addition, the potency of a specific saRNA can be further improved by optimizing the design of the saRNA structure. Supporting data are as follows:

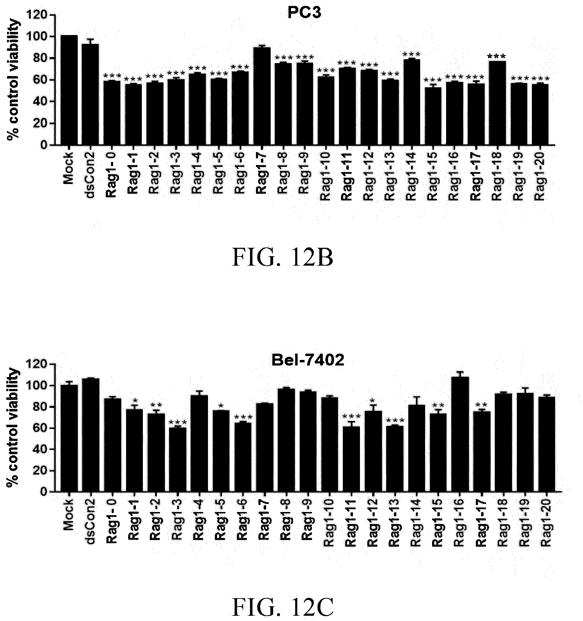

P21-322 (referred to as Rag1-0 hereinafter) was selected for further development with novel sequences and structures to improve its drug-like properties. First, duplex saRNA with different overhangs were tested including replacing dTdT overhang on each strand by a natural nucleotide overhang (i.e. homologous to the corresponding nucleotides on the target) or a UU overhang, resulting in two new saRNA variants (named as Rag1-1 and Rag1-2 respectively) (Fig. 10). Each of the two duplex saRNAs was transfected into three different types of cell lines, including Ku-7-luc2-GFP, PC3 (prostate cancer), and Bel-7402 (hepatocellular carcinoma) cells. 72 hours after transfection, p21 mRNA expression levels were evaluated. As shown in Figs. 11A-11C, in Ku-7-luc2-GFP cells, the p21 induction effect of Rag1-1 and Rag1-2 was slightly higher than that of Rag1-0; and in PC3 and Bel-7402 cells, the p21 induction effect of Rag1-2 is stronger than that of Rag1-0. The cell viability data as measured by the CCK-8 assay showed that the suppressing effects of both Rag1-1 and Rag1-2 against Bel7402 cell proliferation were enhanced compared with that of Rag1-0 (Fig. 12). These data indicated that saRNA duplexes with different overhang structures, i.e. natural nucleotide overhangs and UU overhangs, were not functionally inferior to Rag1-0, and had a better effect in inducing p21 expression and suppressing cell proliferation than Rag1-0 for certain cell types. This also suggested that in certain cancer cell types, the natural nucleotide overhang and the UU overhang structure improved the activity of a saRNA in gene induction and cell growth suppression. Duplex saRNAs with a natural overhang are 100% complementary with the target sequence, and its entire sequence is derived from its target site, which differs from dTdT and UU overhangs. This design provided a better target specificity and complementarity compared with saRNAs containing unnatural or mispaired overhangs.

### Example 5: Effects of duplex saRNA sizes on saRNA's activity in inducing p21

In order to determine whether duplex saRNA length can influence saRNA activity in inducing p21 expression, 5 novel duplexes were designed by extending the Rag1-0 target, one nucleotide at a time from 19 nt to 24 nt toward the direction of the p21 TSS and named Rag1-3, Rag1-4, Rag1-5, Rag1-6, and Rag1-7, which are of 22, 23, 24, 25 and 26 nucleotides in size after adding dTdT overhangs to the 3' end of each strand in the duplexes, (Fig. 10). As shown in Fig. 11A-11C, Rag1-3, which was 22 nucleotides in length, exhibited stronger activity in inducing p21 gene expression and inhibiting cell proliferation in Ku-7-luc2-GFP cells compared with Rag1-0 (Fig. 12). However, duplexes longer than 22 nucleotides lost their activity, suggesting that the duplex length of 21 to 22 nucleotides was preferable for gene induction.

Further, on the basis of P21-323 (also named as Rag1-8), a variant (Rag1-9) containing natural nucleotide overhangs and variants of 22, 23, 24, 25, and 26 nucleotides in length with 3' dTdT overhangs (named as Rag1-10, Rag1-11, Rag1-12, Rag1-13, and Rag1-14, respectively) were designed and synthesized (Fig. 10). As shown in Fig. 11A-11C, Rag1-9, which is 21 nucleotides in length, and bears natural overhangs did not show improved activity, while Rag1-10, which is 22 nucleotides in length, exhibited higher activity compared to all other duplexes in the cell lines tested, and, therefore, was a strong p21 activator. Meanwhile, Rag1-11, which is 23 nucleotides in length, exhibited an activity in Ku-7-luc2-GFP and PC3 cells comparable to Rag1-10, but not in Bel-7402 cells (Fig. 11A-11C). Therefore, extending the length of a saRNA could transform an unfunctional one into a potent gene activator.

### Example 6: Duplex saRNAs with mispaired bases

The applicants designed and synthesized variants of Rag1-0, wherein the adenine nucleotide (A) of the sense strand corresponding to the first nucleotide counted from the 5' terminus of the antisense strand was mutated into a uracil and cytosine, but the antisense strand was kept unchanged, to obtain two new saRNA duplexes Rag1-15 and Rag1-16, respectively, which contained a single mispaired base pair at the last basepair of the duplex. In addition, the nucleotides of the sense strand corresponding to the first and second nucleotide counted from the 5' terminus of the antisense strand were mutated into an adenosine nucleotide and a uracil nucleotide respectively to obtain a saRNA variant, Rag1-17, which contains two mispaired bases at the 3' terminus of the duplex (Fig. 10). These saRNAs were transfected into Ku-7-luc2-GFP, PC3, or Bel-7402 cells and the result showed that all three saRNA variants had better or at least equivalent activity in inducing p21 expression compared with Rag1-0. However, saRNA variant Rag1-16 that contained a mispaired cytosine was the strongest activator in all three cell lines (Fig. 11A-11C).

### Example 7: Duplex saRNAs with asymmetrical structures

Asymmetrically structured duplexes were also synthesized, including Rag1-18 (having a 3' blunt-end instead of an overhang at the 3' terminus of the sense strand), Rag1-20 (having a 3-nucleotide overhang at the 3' terminus of the antisense strand due to the removal of the first nucleotide counted from the 5' terminus of the sense strand), and Rag1-19 (containing a combination of the modifications in Rag1-18 and Rag1-20, i.e., an asymmetric duplex structure characteristic of a 5' end 3-nucleotide overhang and a 3' blunt end which was formed by removing the first nucleotide at the 5' terminus and the dTdT overhang at the 3' terminus of the sense strand) (Fig. 10). As shown in Fig. 11A-11C, Rag1-18 failed to activate p21 in all three cell lines, whereas Rag1-20 was capable of inducing p21 expression in all three cell lines to a similar extent as Rag1-0. Compared to Rag1-18, the combination of the two modifications in Rag1-19 led to the recovery of p21 inducing activity lost in Rag1-18. This data supports that these two structural optimizations does not impair gene induction activity while potentially offering an advantage of reduced off-target effects from the sense strand. An extra advantage is the reduced cost for oligonucleotide synthesis due to shortened nucleotides.

### Example 8: saRNA optimization by different combinations of structural features

1. As shown in Fig. 13, Rag1-21, Rag1-22, Rag1-23, and Rag1-24 were derivatives of Rag1-10.
   (1) Rag1-21 contains UU overhangs and has the following structural formula: sense strand (S₂₀ + 3U₂) and antisense strand (AS₂₀ + 3U₂).
   (2) Rag1-22 contains 2-nt natural nucleotide overhangs, and its structural combination formula is: sense strand (S₂₀ + 3NO₂) and antisense strand (AS₂₀ + 3NO₂).
   (3) Rag1-23 is a variant of Rag1-21 and contains a mismatch at the first base counted from the 3' terminus of the duplex which was created by mutating the adenine of the sense strand at that position into a cytosine. The structural combination formula is: sense strand (S₂₀ + 3U₂), antisense strand (AS₂₀ + 3U₂), and duplex (MM_{(3'1S:A,C)}).
   (4) Rag1-24 is a variant of Rag1-23, wherein the first nucleotide at the 5' terminus of the sense strand was deleted, and the length of the overhang of the antisense strand was thus increased. The structural combination formula is: sense strand (S₁₉ + 3U₂), antisense strand (AS₂₀ + 3U₂NO₂), and duplex (MM_{(3'1S:A,C)}).
2. Rag1-25 and Rag1-26 were variants of Rag1-16, wherein overhangs of the sense strand and the antisense strand were replaced by a UU overhang and a natural overhang respectively, wherein the first nucleotide at the 3' terminus of the sense strand was mutated to a cytosine to create a mismatch.
   (1) The structural combination formula of Rag1-25 is: sense strand (S₁₉ + 3U₂), antisense strand (AS₁₉ + 3U₂), and duplex (MM_{(3'1S:A,C)}).
   (2) The structural combination formula of Rag1-26 is: sense strand (S₁₉ + 3NO₂), antisense strand (AS₁₉ + 3NO₂), and duplex (MM_{(3'1S:A,C)}).
3. Rag1-27 and Rag1-28 were variants of Rag1-20, and in both variants, the first nucleotide at the 3' terminus of the sense strand was mutated to a cytosine to create a mismatch and the first nucleotide at the 5' terminus was deleted, resulting in a 3-nt overhang in the antisense strand. The sense and antisense strands are 20 and 21 nucleotides in length respectively.
   (1) Rag1-27 contains UU overhangs and its structural combination formula is: sense strand (S₁₈ + 3U₂), antisense strand (AS₁₉ + 3U₂), and duplex (MM_{(3'1S:A,C)}).
   (2) Rag1-28 contains 2-nt natural nucleotide overhangs and its structural combination formula is: sense strand (S₁₈ + 3NO₂), antisense strand (AS₁₉ + 3NO₂), and duplex (MM_{(3'1S:A,C)}).

   These saRNA variants (Rag1-21 to Rag1-28) were transfected into Ku-7-luc2-GFP, UM-UC-3 (bladder transitional cell carcinoma), T24 (bladder cancer), J82 (bladder transitional cell carcinoma), or Bel-7402 cell lines, and 72 hours after transfection, p21 gene expression was evaluated. As shown in Fig. 14A-14E, all duplexes induced p21 expression to different extents, of which, Rag1-21, Rag1-23, and Rag1-26 performed better than other variants. Meanwhile, the proliferative capabilities of the transfected cells were analyzed by CCK-8 assay, and the results showed that these saRNA variants exhibited varying degrees of growth inhibition in all cells with Rag1-26 performing the best in Ku-7-luc2-GFP, UM-UC-3, and T24 cells (Fig. 15A-15E). These data were indicative of the benefits provided by the cytosine mismatch at the 3' end of the duplexes and the natural overhang structure.
3. On the basis of Rag1-26, novel variants Rag1-29 to Rag1-44 were designed (Table 1, Fig. 13), and these variants contained the different combinations of the following sequence and structural tweaks:
   strand length variations:
      i. a sense strand of 17 to 22 nucleotides in length;
      ii. an antisense strand of 20 to 22 nucleotides in length;
   duplex structure variations:
      iii. 5' blunt end;
      iv. 3' blunt end;
      v. mispairing;
   overhang feature variations:
      vi. a 1- to 3-nucleotide overhang at the 3' terminus of the antisense strand;
      vii. a 1- to 2-nucleotide overhang at the 5' terminus of the antisense strand;
      viii. a 2-nucleotide (natural nucleotide or UU) overhang at the 3' terminus of the sense strand.

   (1) Rag1-29 had an asymmetric structure with a 5' blunt end which was achieved by removing the overhang of the antisense strand, the purpose of which was to determine whether the overhang of the antisense strand was necessary for p21 induction, and the structural combination formula is: sense strand (S₂₀ + 3NO₂), antisense strand (AS₂₀), and duplex (B₅ + MM_{(3'1S:A,C)}).
   (2) Rag1-30 had a sense strand with its first 5' terminus nucleotide truncated and a UU overhang at the 3' terminus, and an antisense strand with a 3-nt natural nucleotides overhang at the 3' terminus. The structural combination formula is: sense strand (S₁₈ + 3U₂), antisense strand (AS₁₈ + 3NO₃), and duplex (MM_{(3'1S:A,C)}).
   (3) Rag1-31 was similar to Rag1-30 but had an asymmetric structure with a blunt end formed by removing the overhang of the sense strand. The structural combination formula is: sense strand (S₁₈), antisense strand (AS₁₈ + 3NO₃), and duplex (MM_{(3'1S:A,C)}).
   (4) Rag1-32 was similar to Rag1-29 but had a UU overhang at the 3' terminus of the antisense strand, instead of a natural overhang. The structural combination formula is: sense strand (S₂₀ + 3NO₂), antisense strand (AS₂₀ + 3NO₂), and duplex (MM_{(3'1S:A,C)}).
   (5) Rag1-33 was similar to Rag1-32, except that the mispaired cytosine nucleotide in the sense strand was replaced by a guanine nucleotide to test the effect of G:U "wobble" base pairing. The structural combination formula is: sense strand (S₂₀ + 3NO₂), antisense strand (AS₂₀ + 3NO₂), and duplex (MM_{(3'1S:A,G)}).
   (6) Rag1-34 was similar to Rag1-32 but had a nucleotide deletion at the 5' terminus of the sense strand. The structural combination formula is: sense strand (S₁₉ + 3NO₂), antisense strand (AS₁₉ + 3NO₃), and duplex (MM_{(3'1S:A,C)}).
   (7) Rag1-35 was a blunt-end derivative of Rag1-32, wherein the overhang of the sense strand was removed. The structural combination formula is: sense strand (S₂₀), antisense strand (AS₂₀ + 3NO₂) and duplex (B3 + MM_{(3'1S:A,C)}).
   (8) Rag1-36 was similar to Rag1-35, and had an asymmetric structure with a blunt end, formed by deleting a nucleotide at the 5' terminus of the sense strand. The structural combination formula is: sense strand (S₁₉), antisense strand (AS₁₉ + 3NO₃), and duplex (B3 + MM_{(3'1S:A,C)}).
   (9) Rag1-37 was similar to Rag1-36, except that the mispaired cytosine nucleotide was deleted from the sense strand, resulting in a single-nucleotide overhang at the 5' terminus of the antisense strand. The structural combination formula is: sense strand (S₁₈), antisense strand (5NO₁ + AS₁₈ + 3NO₃), and duplex (OH_{AS5'}).
   (10) Rag1-38 was an unconventional derivative of Rag 1-26, wherein the antisense strand possessed a UU overhang at its 5' terminus to match the 3' overhang of the sense strand and a single-nucleotide overhang at the 3' terminus. The structural combination formula is: sense strand (S₁₈ + 3U₂), antisense strand (5U₂ + AS₁₈ + 3NO₁), and duplex (OH_{AS5'} + MM_{(3'1S:A,C)}).
   (11) Rag1-39 was a derivative of Rag1-33, wherein the mispaired cytosine nucleotide of the sense strand was replaced by a guanine nucleotide, and the duplex length was reduced by one base pair. The structural combination formula is: sense strand (S₁₈ + 3NO₂), antisense strand (AS₁₈ + 3NO₃), and duplex (MM_{(3'1S:A,G)}).
   (12) Rag1-40 was a derivative of Rag1-26, wherein an asymmetric structure with a blunt end was introduced by removing the overhang of the sense strand. The structural combination formula is: sense strand (S₁₉), antisense strand (AS₁₉ + 3NO₂), and duplex (B3 + MM_{(3'1S:A,C)}).
   (13) Rag1-41 was similar to Rag1-40, except that the mispaired cytosine nucleotide of the sense strand was replaced by a guanine nucleotide. The structural combination formula is: sense strand (S₁₉), antisense strand (AS₁₉ + 3NO₂), and duplex (B3 + MM_{(3'1S:A,G)}).
   (14) Rag1-42 was also similar to Rag1-40, except that the mispaired nucleotide was deleted from the sense strand, leading to a single-nucleotide overhang at the 5' terminus of the antisense strand. The structural combination formula is: sense strand (S₁₈), antisense strand (5NO₁ + AS₁₈ + 3NO₂), and duplex (OH_{AS5'}).
   (15) Rag1-43 was also similar to Rag1-40, except that the duplex length was reduced by one base pair. The structural combination formula is: sense strand (S₁₈), antisense strand (AS₁₈ + 3NO₂), and duplex (B3 + MM_{(3'1S:A,C)}).
   (16) Rag1-44 was similar to Rag1-37, except that the duplex length was reduced by one base pair. The structural combination formula is: sense strand (S₁₇), antisense strand (5NO₁ + AS₁₈ + 3NO₃), and duplex (OH_{AS5'}).

The aforementioned duplex saRNAs (Rag1-29 to Rag1-44) were transfected into Ku-7-luc2-GFP, UM-UC-3, T24, J82, or PC3 cell lines, respectively, and 72 hours after transfection, p21 mRNA expression was evaluated. As shown in Fig. 16A-16E, Rag1-29 and Rag1-38 almost completely lost their activity in inducing p21 expression in all cell lines. These two duplexes did not have an overhang of at least two nucleotides on their antisense strands, indicating this feature is indispensable for p21 expression induction. In contrast, a 3' overhang on the sense strand was totally dispensable, since Rag1-31, Rag1-35, Rag1-36, Rag1-40, Rag1-41, and Rag1-43 possessed strong activities. In addition, the presence of the nucleotide mispairing at the 3' terminus of a duplex was critical for maximizing activity, because the removal of the mispaired nucleotide at the 3' terminus of the sense strand of Rag1-37, Rag1-42, and Rag1-44 led to a decrease in p21 induction activity compared to other strong activators. Replacement of the cytosine mismatch on the sense strand by a guanine to form a G-U mispairing with the uracil on the antisense strand (for example, in Rag1-39 and Rag1-41) had an adverse impact on the activity of the saRNA with the exception for Rag1-33. A plausible explanation is that a positive influence resulted from the longer length of its duplex region (20 nucleotides, compared with 18 and 19 nucleotides of Rag1-39 and Rag1-40, respectively) offset the negative impact of the G-U mispairing. Nonetheless, this further supports a preference for cytosine mispairing over other nucleotides for enhanced gene induction activity of a saRNA.

The cell viability of each cell line (Fig. 17A-17E) was assayed by the CCK-8 method. It was discovered by a comprehensive analysis in combination with the gene-inducing activity (Fig. 16A-16E) that duplexes Rag1-30, Rag1-32, Rag1-35, and Rag1-40 all had a maximum gene-inducing activity and a strong suppressing effect against the proliferation of the cancer cells. Although Rag1-35 and Rag1-40 were variants of Rag1-23 and Rag1-26, respectively, and both have an asymmetric structure with a blunt end, a cytosine mispairing, and a natural nucleotide overhang, Rag1-40 showed significantly improved activity in gene induction and tumor cell growth suppression due to 100% complementarity of the full length of the antisense strand of its duplex with its target.

The duplex of Rag1-36 was a potent activator for p21 expression, but lost part or all of the cell proliferation-suppressing activity in the tested cell lines. It was the only duplex whose gene-inducing activity did not correlate to its cell proliferation-suppressing activity among all tested saRNA variants. This unique property could be related to the sequence and/or structural feature of Rag1-36 and have utility in a situation where only the induction of the target gene expression, but not inhibition of cell proliferation, is desired.

### Example 9: Validation of optimized combinations of structural features

saRNAs of conventional structures (i.e. RAG-431-0, RAG-553-0, RAG-688-0 and RAG-693-0) targeting the p21 gene promoter and three types of variants thereof were designed and synthesized. Their structural combination formulas are as follows (Fig. 18):
RAG-431-1: sense strand (S₁₉), antisense strand (AS₁₉ + 3NO₂), and duplex (B3 + MM_{(3'1S:U,C)})
RAG-431-3: sense strand (S₁₉ + 3T₂), antisense strand (AS₁₉), and duplex (B5 + MM_{(5'1AS:U,C)})
RAG-553-1: sense strand (S₁₉), antisense strand (AS₁₉ + 3NO₂), and duplex (B3 + MM_{(3'1S:G,C)})
RAG-553-3: sense strand (S₁₉ + 3T₂), antisense strand (AS₁₉), and duplex (B5 + MM_{(5'1AS:U,C)})
RAG-688-1: sense strand (S₁₉), antisense strand (AS₁₉ + 3NO₂), and duplex (B3 + MM_{(3'1S:A,C)})
RAG-688-3: sense strand (S₁₉ + 3T₂), antisense strand (AS₁₉), and duplex (B5 + MM_{(5'1AS:U,C)})
RAG-693-1: sense strand (S₁₉), antisense strand (AS₁₉ + 3NO₂), and duplex (B3 + MM_{(3'1S:U,C)})
RAG-693-3: sense strand (S₁₉ + 3T₂), antisense strand (AS₁₉), and duplex (B5 + MM_{(5'1AS:U,C)})

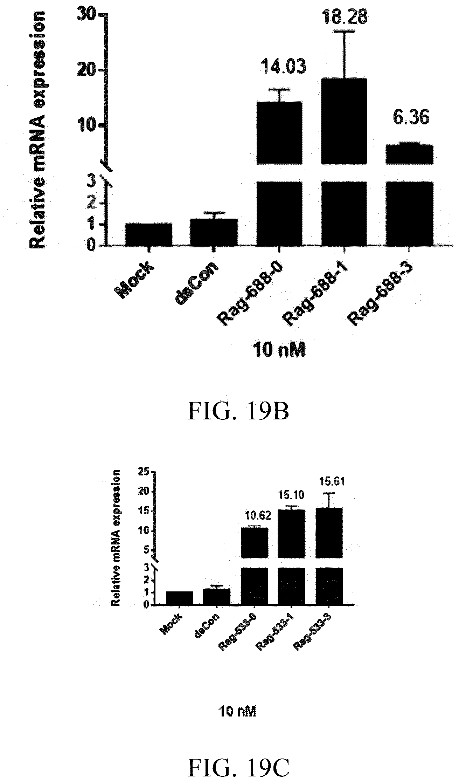

RAG-431-0 and the variants thereof were transfected into Ku-7-luc2-GFP cells, wherein RAG-431-0 induced a 9.37-fold change in p21 expression, and its variants of RAG-431-1 and RAG-431-3 induced an 18.35-fold change and a 12.26-fold change in p21 expression, respectively (Fig. 19).

RAG-553-0 and the variants thereof were transfected into Ku-7-luc2-GFP cells, wherein RAG-553-0 induced a 10.62-fold change in p21 expression, and its variants of RAG-553-1 and RAG-553-3 induced a 15.10-fold change and a 15.61-fold change in p21 expression, respectively (Fig. 19).

RAG-688-0 and the variants thereof were transfected into Ku-7-luc2-GFP cells, wherein RAG-688-0 induced a 14.03-fold change in p21 expression, and its variants of RAG-688-1 and RAG-688-3 induced an 18.28-fold change and a 6.36-fold change in p21 expression, respectively (Fig. 19).

RAG-693-0 and the variants thereof were transfected into Ku-7-luc2-GFP cells, wherein RAG-693-0 induced a 7.04-fold change in p21 expression, and its variants of RAG-693-1 and RAG-693-3 induced a 10.54-fold change and a 6.85-fold change in p21 expression, respectively (Fig. 19).

saRNAs (KLF4-0) targeting the KLF4 gene promoter and its variant KLF4-1 with structures similar to that of Rag1-40, were also designed and synthesized, and the structural combination formula for KLF4-1 is: sense strand (S₁₉), antisense strand (AS₁₉ + 3NO₂), and duplex (B3+MM_{(3'1S:A,C)}) (Fig. 20). These saRNAs were transfected into PC3, Ku-7-luc2-GFP, and Bel-7402 cells, wherein KLF4-0 induced a 2.64-, 1.57-, and 1.31-fold change in KLF4 expression, respectively in those cells, while the variant KLF4-1 exhibited a higher activity in inducing KLF4 expression than did KLF4-0, and induced a 3.42-, 1.77-, and 1.43-fold change, respectively, in the PC3, Ku-7-luc2-GFP, and Bel-7402 cells (Fig. 21A-21C).

saRNAs (NKX3-1-0) targeting the NKX3-1 gene promoter and its variants NKX3-1-1, NKX3-1-2, and NKX3-1-3 were also designed and synthesized. The structures of these variants were similar to that of Rag1-40. The structural combination formula of NKX3-1-1 is: sense strand (S₁₉), antisense strand (AS₁₉ + 3NO₂) and duplex (B3 + MM_{(3'1S:A,C)}); the structural combination formula of NKX3-1-2 is: sense strand (S₁₉), antisense strand (AS₁₉ + 3NO₂), and duplex (B3 + MM_{(3'1S:A,G)}); and the structural combination formula of NKX3-1-3 is: sense strand (S₁₉), antisense strand (AS₁₉ + 3NO₂), and duplex (B3 + MM_{(3'1S:A,U)}) (Fig. 20). These saRNAs were transfected into PC3 and Bel-7402 cells, wherein NKX3-1-0 induced a 2.7- and 1.67-fold change in NKX3-1 expression respectively in the two cell types, while the activities of all the variants (NKX3-1-1, NKX3-1-2, and NKX3-1-3) in inducing NKX3-1 expression were higher than that of NKX3-1-0 (Fig. 22A-22B).

saRNAs (VEGF-0) targeting the VEGFA gene promoter and its variant VEGF-1 with structures similar to that of Rag1-40, were also designed and synthesized, and the structural combination formula of VEGF-1 is: sense strand (S₁₉), antisense strand (AS₁₉ + 3NO₂), and duplex (B3 + MM_{(3'1S:A,C)}) (Fig. 20). These saRNAs were transfected into HeLa, COS-1, or ARPE-19 cells, wherein VEGF-0 induced a 1.22-, 1.11-, and 1.51-fold change in VEGFA expression, respectively in the 3 types of cells, while the activity of the variant VEGF-1 in inducing VEGFA expression was higher than that of VEGF-0 (Fig. 23A-23C).

From the above analysis, it was concluded that saRNAs with a structure similar to Rag1-40 were superior than saRNAs with conventional structure in terms of gene-activating activity and the enhancement was independent of the sequence of the saRNAs. Specifically, the 3' overhang of the antisense strand could be a natural nucleotide overhang (such as RAG-431-1, RAG-553-1, RAG-688-1, RAG-693-1, NKX3-1-1, NKX3-1-2, and NKX3-1-3) or a UU overhang (such as KLF4-1 and VEGF-1). The mutated nucleotides of the sense strand can be C (such as RAG-431-1, RAG-431-3, RAG-688-1, RAG-688-3, RAG-693-1, NKX3-1-1, KLF4-1, VEGF-1), A (RAG-553-1 and RAG-553-3), G (NKX3-1-2), and U (such as NKX3-1-3).

### Example 10: Inhibition of the growth of subcutaneously human xenograft tumors in mice by p21 saRNA Rag1-40

Formulation of saRNA: In vivo-jetPEI (201-10G, Polyplus-transfection, France) was used to deliver saRNA, and the saRNA was formulated according to the preparation method provided by the manufacturer. Briefly, the saRNA was first diluted in 10% glucose solution to obtain solution A; a required amount of in vivo-jetPEI was diluted in the 10% glucose solution to obtain solution B; then equal volumes of solution A and solution B were combined and mixed well to give a formulation with a nitrogen-to-phosphorus (N:P) ratio of 8 and a final concentration of glucose at 5%. The mixture was incubated at room temperature for 15 minutes before injection into mice.

HepG2 human hepatocellular carcinoma cells at the logarithmic phase were harvested and counted to make a suspension of 3.5 × 10⁷ cells per milliliter (mL). The cell suspension (0.2 mL/mouse) was then subcutaneously inoculated into the right armpit of BALB/c nude mice. When tumors grew to about 100 mm³, the tumor-bearing mice were randomly divided into two groups: a Vehicle (5% glucose) group and a Rag1-40-treated group, with 7 mice per group, and the saRNA formulation or vehicle control was injected into the tumors at a dose of 1 mg·kg-1 saRNA on day 1, day 4, day 7 and day 10. The long and short diameters of the tumors were measured every two days with a Vernier caliper starting from the first administration (day 1), and the volumes of the tumors were calculated according to a formula: V = (L × W²)/2, wherein L represents the longest diameter of a tumor, and W represents a diameter parallel with the tumor surface and perpendicular to the long diameter. A tumor growth curve during administration, as well as weight and morphology of the tumors after harvest, were recorded.

As shown in Fig. 24, Rag1-40 treated tumors exhibited a slower growth from day 4 than vehicle treated tumors and appeared to shrink from day 7 of treatment. On day 10 of treatment, the mean volume of Rag1-40 treated tumors increased by 12.8% compared to the volume at the beginning of treatment, whereas the mean volume of the tumors in the Vehicle group increased by 119.8%. The difference in the final tumor volume between the two groups was statistically significant (P < 0.001), indicating that the saRNA capable of activating p21 expression had a significant *in vivo* antitumor effect.

### Incorporation by reference

All disclosures of each patent literature and scientific literature cited herein are incorporated herein by reference for all purposes.

### Equivalence

The present invention can be implemented in other specific forms without departing from its fundamental characteristics. Therefore, the aforementioned examples shall be considered as illustrative rather than restrictive to the present invention described herein. The scope of the present invention is represented by the appended claims rather than the above specification and is intended to cover all changes falling into the meanings and scopes of equivalents of the claims.

## Claims

1. A small activating RNA, wherein the small activating RNA is composed of a first oligonucleotide strand containing 17 to 30 nucleotides and a second oligonucleotide strand containing 17 to 30 nucleotides, a sequence of at least 15 nucleotides in length in the first oligonucleotide strand is complementary to the second oligonucleotide strand, the first oligonucleotide strand or the second oligonucleotide strand has more than 75% homology or complementarity with any continuous fragment of 15 to 30 nucleotides in length in the promoter of a target gene, one end of a duplex formed by the first oligonucleotide strand and the second oligonucleotide strand is a blunt end, and the other end has an overhang with 1 to 4 nucleotides formed at the terminus of the first oligonucleotide strand or the second oligonucleotide strand.

2. The small activating RNA of claim 1, wherein one end of the duplex formed by the first oligonucleotide strand and the second oligonucleotide strand is a blunt end, and the other end has an overhang with 2 or 3 nucleotides formed at the terminus of the first oligonucleotide strand or the second oligonucleotide strand.

3. The small activating RNA of claim 1 or 2, wherein the nucleotides of the overhang are selected from thymines, uridines or natural nucleotides.

4. The small activating RNA of claim 3, wherein the overhang is selected from dTdTdT, dTdT, UUU, UU, or 2 or 3 continuous natural nucleotides.

5. The small activating RNA of any one of claims 1 to 4, wherein a 5' terminus of the first or second oligonucleotide strand is at the blunt end of the duplex, and there are 1 to 3 nucleotides of the first to third nucleotides from the 5' terminus in the first or second oligonucleotide strand are mispaired with nucleotides at the corresponding positions in the other strand.

6. The small activating RNA of claim 5, wherein the mispaired nucleotides are cytosines.

7. The small activating RNA of any one of claims 1 to 6, wherein the length of the duplex formed by the first oligonucleotide strand and the second oligonucleotide strand, excluding the overhang, is 17 to 24 nucleotides.

8. The small activating RNA of claim 7, wherein the length of the duplex formed by the first oligonucleotide strand and the second oligonucleotide strand, excluding the overhang, is 18 to 20 nucleotides.

9. The use of the small activating RNA of any one of claims 1 to 8 in the preparation of a formulation for activating or up-regulating the expression of a target gene in a cell.

10. The use of claim 9, wherein the small activating RNA is introduced into the cell directly.

11. The use of claim 10, wherein the cell is a mammalian cell, and preferably a human cell.

12. The application of claim 11, wherein the cell is present in a human body.

13. The use of claim 12, wherein the human body suffers from a disease caused by the defect and/or deficiency of target gene expression, and the small activating molecule is administrated in an effective amount to treat the disease.

14. The small activating RNA of any one of claims 1 to 8, wherein the target gene is selected from the group consisting of human p21, KLF4, NKX3-1, and VEGFA.

15. The small activating RNA of claim 14, wherein the small activating RNA activates or upregulates the expression of p21 gene by at least 10%.

16. A composition, comprising the small activating RNA of claim 14 or 15 and a pharmaceutically acceptable carrier.

17. The composition of claim 16, wherein the pharmaceutically acceptable carrier is a liposome, a macromolecular polymer, or a polypeptide.

18. The use of the small activating RNA of claim 14 or 15 or the composition of claim 16 or 17 in the preparation of a formulation for activating or upregulating the expression of target gene, and preferably in the preparation of a formulation for treating a tumor or a benign proliferative lesion, wherein the tumor is more preferably selected from bladder cancer, prostate cancer, hepatoma and colorectal cancer.


SEQUENCE LISTING

<110>  Sino-US Institute of RNA Technology

<120>  NOVEL SMALL ACTIVATING RNA

<130>  065786.11000/1US1

<150>  PCT/CN2019/082149
<151>  2019-04-10

<150>  CN201810317366.X
<151>  2018-04-10

<160>  583

<170>  PatentIn version 3.5

<210>  1
<211>  21
<212>  DNA
<213>  artificial sequence

<220>
<223>  Small Activating RNA

<400>  1
acuacugagu gacaguagat t                                                21


<210>  2
<211>  21
<212>  DNA
<213>  artificial sequence

<220>
<223>  Small Activating RNA

<400>  2
ucuacuguca cucaguagut t                                                21


<210>  3
<211>  21
<212>  DNA
<213>  artificial sequence

<220>
<223>  Small Activating RNA

<400>  3
cuggagagug ccaacucaut t                                                21


<210>  4
<211>  21
<212>  DNA
<213>  artificial sequence

<220>
<223>  Small Activating RNA

<400>  4

augagauggc acucuccagt t                                                21

<210>  5
<211>  21
<212>  DNA
<213>  artificial sequence

<220>
<223>  Small Activating RNA

<400>  5
uggagagugc caacucauut t                                                21

<210>  6
<211>  21
<212>  DNA
<213>  artificial sequence

<220>
<223>  Small Activating RNA

<400>  6
aaugaguugg cacucuccat t                                                21

<210>  7
<211>  21
<212>  DNA
<213>  artificial sequence

<220>
<223>  Small Activating RNA

<400>  7
ggagagugcc aacucauuct t                                                21

<210>  8
<211>  21
<212>  DNA
<213>  artificial sequence

<220>
<223>  Small Activating RNA

<400>  8
gaaugaguug gcacucucct t                                                21

<210>  9
<211>  21
<212>  DNA
<213>  artificial sequence

<220>
<223>  Small Activating RNA

<400>  9
gagagugcca acucauucut t                                                21

<210>  10
<211>  21
<212>  DNA
<213>  artificial sequence

<220>
<223>  Small Activating RNA

<400>  10
agaaugaguu ggcacucuct t                                                21


<210>  11
<211>  21
<212>  DNA
<213>  artificial sequence

<220>
<223>  Small Activating RNA

<400>  11
agagugccaa cucauucuct t                                                21


<210>  12
<211>  21
<212>  DNA
<213>  artificial sequence

<220>
<223>  Small Activating RNA

<400>  12
gagaaugagu uggcacucut t                                                21


<210>  13
<211>  21
<212>  DNA
<213>  artificial sequence

<220>
<223>  Small Activating RNA

<400>  13
gagugccaac ucauucucct t                                                21


<210>  14
<211>  21
<212>  DNA
<213>  artificial sequence

<220>
<223>  Small Activating RNA

<400>  14
ggagaaugag uuggcacuct t                                                21


<210>  15
<211>  21
<212>  DNA

<213>  artificial sequence

<220>
<223>  Small Activating RNA

<400>  15
agugccaacu cauucuccat t                                                21


<210>  16
<211>  21
<212>  DNA
<213>  artificial sequence

<220>
<223>  Small Activating RNA

<400>  16
uggagaauga guuggcacut t                                                21


<210>  17
<211>  21
<212>  DNA
<213>  artificial sequence

<220>
<223>  Small Activating RNA

<400>  17
gugccaacuc auucuccaat t                                                21


<210>  18
<211>  21
<212>  DNA
<213>  artificial sequence

<220>
<223>  Small Activating RNA

<400>  18
uuggagaaug aguuggcact t                                                21


<210>  19
<211>  21
<212>  DNA
<213>  artificial sequence

<220>
<223>  Small Activating RNA

<400>  19
ugccaacuca uucuccaagt t                                                21


<210>  20
<211>  21
<212>  DNA
<213>  artificial sequence

<220>

<223>  Small Activating RNA

<400>  20
cuuggagaau gaguuggcat t                                                21


<210>  21
<211>  21
<212>  DNA
<213>  artificial sequence

<220>
<223>  Small Activating RNA

<400>  21
gccaacucau ucuccaagut t                                                21


<210>  22
<211>  21
<212>  DNA
<213>  artificial sequence

<220>
<223>  Small Activating RNA

<400>  22
acuuggagaa ugaguuggct t                                                21


<210>  23
<211>  21
<212>  DNA
<213>  artificial sequence

<220>
<223>  Small Activating RNA

<400>  23
ccaacucauu cuccaaguat t                                                21


<210>  24
<211>  21
<212>  DNA
<213>  artificial sequence

<220>
<223>  Small Activating RNA

<400>  24
uacuuggaga augaguuggt t                                                21


<210>  25
<211>  21
<212>  DNA
<213>  artificial sequence

<220>
<223>  Small Activating RNA

<400>  25

caacucauuc uccaaguaat t                                                21

<210>  26
<211>  21
<212>  DNA
<213>  artificial sequence

<220>
<223>  Small Activating RNA

<400>  26
uuacuuggag aaugaguugt t                                                21

<210>  27
<211>  21
<212>  DNA
<213>  artificial sequence

<220>
<223>  Small Activating RNA

<400>  27
aaaagccaga uuuguggcut t                                                21

<210>  28
<211>  21
<212>  DNA
<213>  artificial sequence

<220>
<223>  Small Activating RNA

<400>  28
agccacaaau cuggcuuuut t                                                21

<210>  29
<211>  21
<212>  DNA
<213>  artificial sequence

<220>
<223>  Small Activating RNA

<400>  29
aaagccagau uuguggcuct t                                                21

<210>  30
<211>  21
<212>  DNA
<213>  artificial sequence

<220>
<223>  Small Activating RNA

<400>  30
gagccacaaa ucuggcuuut t                                                21

<210>  31
<211>  21
<212>  DNA
<213>  artificial sequence

<220>
<223>  Small Activating RNA

<400>  31
aagccagauu uguggcucat t                                                21


<210>  32
<211>  21
<212>  DNA
<213>  artificial sequence

<220>
<223>  Small Activating RNA

<400>  32
ugagccacaa aucuggcuut t                                                21


<210>  33
<211>  21
<212>  DNA
<213>  artificial sequence

<220>
<223>  Small Activating RNA

<400>  33
agccagauuu guggcucact t                                                21


<210>  34
<211>  21
<212>  DNA
<213>  artificial sequence

<220>
<223>  Small Activating RNA

<400>  34
gugagccaca aaucuggcut t                                                21


<210>  35
<211>  21
<212>  DNA
<213>  artificial sequence

<220>
<223>  Small Activating RNA

<400>  35
gccagauuug uggcucacut t                                                21


<210>  36
<211>  21
<212>  DNA

<213>  artificial sequence

<220>
<223>  Small Activating RNA

<400>  36
agugagccac aaaucuggct t                                                21


<210>  37
<211>  21
<212>  DNA
<213>  artificial sequence

<220>
<223>  Small Activating RNA

<400>  37
ccaacucauu cuccaaguaa a                                                21


<210>  38
<211>  21
<212>  DNA
<213>  artificial sequence

<220>
<223>  Small Activating RNA

<400>  38
uacuuggaga augaguuggc a                                                21


<210>  39
<211>  21
<212>  DNA
<213>  artificial sequence

<220>
<223>  Small Activating RNA

<400>  39
ccaacucauu cuccaaguau u                                                21


<210>  40
<211>  21
<212>  DNA
<213>  artificial sequence

<220>
<223>  Small Activating RNA

<400>  40
uacuuggaga augaguuggu u                                                21


<210>  41
<211>  22
<212>  DNA
<213>  artificial sequence

<220>

<223>  Small Activating RNA

<400>  41
ccaacucauu cuccaaguaa tt                                                22


<210>  42
<211>  22
<212>  DNA
<213>  artificial sequence

<220>
<223>  Small Activating RNA

<400>  42
uuacuuggag aaugaguugg tt                                                22


<210>  43
<211>  23
<212>  DNA
<213>  artificial sequence

<220>
<223>  Small Activating RNA

<400>  43
ccaacucauu cuccaaguaa att                                               23


<210>  44
<211>  23
<212>  DNA
<213>  artificial sequence

<220>
<223>  Small Activating RNA

<400>  44
uuuacuugga gaaugaguug gtt                                               23


<210>  45
<211>  24
<212>  DNA
<213>  artificial sequence

<220>
<223>  Small Activating RNA

<400>  45
ccaacucauu cuccaaguaa aatt                                              24


<210>  46
<211>  24
<212>  DNA
<213>  artificial sequence

<220>
<223>  Small Activating RNA

<400>  46

uuuuacuugg agaaugaguu ggtt                                                24

<210>  47
<211>  25
<212>  DNA
<213>  artificial sequence

<220>
<223>  Small Activating RNA

<400>  47
ccaacucauu cuccaaguaa aaatt                                               25

<210>  48
<211>  25
<212>  DNA
<213>  artificial sequence

<220>
<223>  Small Activating RNA

<400>  48
uuuuuacuug gagaaugagu uggtt                                               25

<210>  49
<211>  26
<212>  DNA
<213>  artificial sequence

<220>
<223>  Small Activating RNA

<400>  49
ccaacucauu cuccaaguaa aaaatt                                              26

<210>  50
<211>  26
<212>  DNA
<213>  artificial sequence

<220>
<223>  Small Activating RNA

<400>  50
uuuuuuacuu ggagaaugag uuggtt                                              26

<210>  51
<211>  21
<212>  DNA
<213>  artificial sequence

<220>
<223>  Small Activating RNA

<400>  51
gccaacucau ucuccaagua a                                                   21

<210>  52
<211>  21
<212>  DNA
<213>  artificial sequence

<220>
<223>  Small Activating RNA

<400>  52
acuuggagaa ugaguuggca c                                                21


<210>  53
<211>  22
<212>  DNA
<213>  artificial sequence

<220>
<223>  Small Activating RNA

<400>  53
gccaacucau ucuccaagua tt                                               22


<210>  54
<211>  22
<212>  DNA
<213>  artificial sequence

<220>
<223>  Small Activating RNA

<400>  54
uacuuggaga augaguuggc tt                                               22


<210>  55
<211>  23
<212>  DNA
<213>  artificial sequence

<220>
<223>  Small Activating RNA

<400>  55
gccaacucau ucuccaagua att                                              23


<210>  56
<211>  23
<212>  DNA
<213>  artificial sequence

<220>
<223>  Small Activating RNA

<400>  56
uuacuuggag aaugaguugg ctt                                              23


<210>  57
<211>  24
<212>  DNA

<213>  artificial sequence

<220>
<223>  Small Activating RNA

<400>  57
gccaacucau ucuccaagua aatt                                              24


<210>  58
<211>  24
<212>  DNA
<213>  artificial sequence

<220>
<223>  Small Activating RNA

<400>  58
uuuacuugga gaaugaguug gctt                                              24


<210>  59
<211>  25
<212>  DNA
<213>  artificial sequence

<220>
<223>  Small Activating RNA

<400>  59
gccaacucau ucuccaagua aaatt                                             25


<210>  60
<211>  25
<212>  DNA
<213>  artificial sequence

<220>
<223>  Small Activating RNA

<400>  60
uuuuacuugg agaaugaguu ggctt                                             25


<210>  61
<211>  26
<212>  DNA
<213>  artificial sequence

<220>
<223>  Small Activating RNA

<400>  61
gccaacucau ucuccaagua aaaatt                                            26


<210>  62
<211>  26
<212>  DNA
<213>  artificial sequence

<220>

<223>  Small Activating RNA

<400>  62
uuuuuacuug gagaaugagu uggctt                                          26


<210>  63
<211>  21
<212>  DNA
<213>  artificial sequence

<220>
<223>  Small Activating RNA

<400>  63
ccaacucauu cuccaaguut t                                               21


<210>  64
<211>  21
<212>  DNA
<213>  artificial sequence

<220>
<223>  Small Activating RNA

<400>  64
ccaacucauu cuccaaguct t                                               21


<210>  65
<211>  21
<212>  DNA
<213>  artificial sequence

<220>
<223>  Small Activating RNA

<400>  65
ccaacucauu cuccaagaut t                                               21


<210>  66
<211>  19
<212>  DNA
<213>  artificial sequence

<220>
<223>  Small Activating RNA

<400>  66
ccaacucauu cuccaagua                                                  19


<210>  67
<211>  18
<212>  DNA
<213>  artificial sequence

<220>
<223>  Small Activating RNA

<400>  67

caacucauuc uccaagua                                                    18


<210>  68
<211>  20
<212>  DNA
<213>  artificial sequence

<220>
<223>  Small Activating RNA

<400>  68
caacucauuc uccaaguatt                                                  20


<210>  69
<211>  22
<212>  DNA
<213>  artificial sequence

<220>
<223>  Small Activating RNA

<400>  69
gccaacucau ucuccaagua uu                                               22


<210>  70
<211>  22
<212>  DNA
<213>  artificial sequence

<220>
<223>  Small Activating RNA

<400>  70
uacuuggaga augaguuggc uu                                               22


<210>  71
<211>  22
<212>  DNA
<213>  artificial sequence

<220>
<223>  Small Activating RNA

<400>  71
gccaacucau ucuccaagua aa                                               22


<210>  72
<211>  22
<212>  DNA
<213>  artificial sequence

<220>
<223>  Small Activating RNA

<400>  72
uacuuggaga augaguuggc ac                                               22

<210>  73
<211>  22
<212>  DNA
<213>  artificial sequence

<220>
<223>  Small Activating RNA

<400>  73
gccaacucau ucuccaaguc uu                                                22


<210>  74
<211>  20
<212>  DNA
<213>  artificial sequence

<220>
<223>  Small Activating RNA

<400>  74
caacucauuc uccaagucuu                                                   20


<210>  75
<211>  21
<212>  DNA
<213>  artificial sequence

<220>
<223>  Small Activating RNA

<400>  75
ccaacucauu cuccaagucu u                                                 21


<210>  76
<211>  21
<212>  DNA
<213>  artificial sequence

<220>
<223>  Small Activating RNA

<400>  76
ccaacucauu cuccaaguca a                                                 21


<210>  77
<211>  20
<212>  DNA
<213>  artificial sequence

<220>
<223>  Small Activating RNA

<400>  77
caacucauuc uccaagucaa                                                   20


<210>  78
<211>  22
<212>  DNA

<213>  artificial sequence

<220>
<223>  Small Activating RNA

<400>  78
gccaacucau ucuccaaguc aa                                              22


<210>  79
<211>  20
<212>  DNA
<213>  artificial sequence

<220>
<223>  Small Activating RNA

<400>  79
uacuuggaga augaguuggc                                                 20


<210>  80
<211>  18
<212>  DNA
<213>  artificial sequence

<220>
<223>  Small Activating RNA

<400>  80
caacucauuc uccaaguc                                                   18


<210>  81
<211>  22
<212>  DNA
<213>  artificial sequence

<220>
<223>  Small Activating RNA

<400>  81
gccaacucau ucuccaagug aa                                              22


<210>  82
<211>  20
<212>  DNA
<213>  artificial sequence

<220>
<223>  Small Activating RNA

<400>  82
gccaacucau ucuccaaguc                                                 20


<210>  83
<211>  19
<212>  DNA
<213>  artificial sequence

<220>

<223>  Small Activating RNA

<400>  83
ccaacucauu cuccaaguc                                                    19


<210>  84
<211>  18
<212>  DNA
<213>  artificial sequence

<220>
<223>  Small Activating RNA

<400>  84
ccaacucauu cuccaagu                                                     18


<210>  85
<211>  21
<212>  DNA
<213>  artificial sequence

<220>
<223>  Small Activating RNA

<400>  85
uuuacuugga gaaugaguug g                                                 21


<210>  86
<211>  20
<212>  DNA
<213>  artificial sequence

<220>
<223>  Small Activating RNA

<400>  86
caacucauuc uccaagugaa                                                   20


<210>  87
<211>  19
<212>  DNA
<213>  artificial sequence

<220>
<223>  Small Activating RNA

<400>  87
ccaacucauu cuccaagug                                                    19


<210>  88
<211>  17
<212>  DNA
<213>  artificial sequence

<220>
<223>  Small Activating RNA

<400>  88

caacucauuc uccaagu                                                    17

<210>  89
<211>  21
<212>  DNA
<213>  artificial sequence

<220>
<223>  Synthesized primer for PCR

<400>  89
atcaccatct tccaggagcg a                                                21

<210>  90
<211>  21
<212>  DNA
<213>  artificial sequence

<220>
<223>  Synthesized primer for PCR

<400>  90
ttctccatgg tggtgaagac g                                                21

<210>  91
<211>  20
<212>  DNA
<213>  artificial sequence

<220>
<223>  Synthesized primer for PCR

<400>  91
ggaagaccat gtggacctgt                                                  20

<210>  92
<211>  20
<212>  DNA
<213>  artificial sequence

<220>
<223>  Synthesized primer for PCR

<400>  92
ggattagggc ttcctcttgg                                                  20

<210>  93
<211>  92
<212>  DNA
<213>  Homo sapiens

<400>  93
ggctatgtgg ggagtattca ggagacagac aactcactcg tcaaatcctc cccttcctgg      60

ccaacaaagc tgctgcaacc acagggattt ct                                    92

<210>  94

<211>  86
<212>  DNA
<213>  Homo sapiens

<400>  94
ggtagtctct ccaattccct ccttcccgga agcatgtgac aatcaacaac tttgtatact      60

taagttcagt ggacctcaat ttcctc                                          86


<210>  95
<211>  35
<212>  DNA
<213>  Homo sapiens

<400>  95
ctttgttggg gtgtctaggt gctccaggtg cttct                                35


<210>  96
<211>  51
<212>  DNA
<213>  Homo sapiens

<400>  96
ttctgggaga ggtgacctag tgagggatca gtgggaatag aggtgatatt g              51


<210>  97
<211>  30
<212>  DNA
<213>  Homo sapiens

<400>  97
aggtgatatt gtggggcttt tctggaaatt                                      30


<210>  98
<211>  38
<212>  DNA
<213>  Homo sapiens

<400>  98
attaatgtca tcctcctgat cttttcagct gcattggg                             38


<210>  99
<211>  40
<212>  DNA
<213>  Homo sapiens

<400>  99
tctaacagtg ctgtgtcctc ctggagagtg ccaactcatt                           40


<210>  100
<211>  66
<212>  DNA
<213>  Homo sapiens

<400>  100
gtgccaactc attctccaag taaaaaaagc cagatttgtg gctcacttcg tggggaaatg      60

tgtcca                                                                    66

<210>  101
<211>  21
<212>  DNA
<213>  artificial sequence

<220>
<223>  Small Activating RNA

<400>  101
ucguggggaa auguguccat t                                                   21

<210>  102
<211>  21
<212>  DNA
<213>  artificial sequence

<220>
<223>  Small Activating RNA

<400>  102
uggacacauu uccccacgat t                                                   21

<210>  103
<211>  21
<212>  DNA
<213>  artificial sequence

<220>
<223>  Small Activating RNA

<400>  103
uucgugggga aaugugucct t                                                   21

<210>  104
<211>  21
<212>  DNA
<213>  artificial sequence

<220>
<223>  Small Activating RNA

<400>  104
ggacacauuu ccccacgaat t                                                   21

<210>  105
<211>  21
<212>  DNA
<213>  artificial sequence

<220>
<223>  Small Activating RNA

<400>  105
   cuucgugggg aaauguguct t                                                  21

<210>  106
<211>  21
<212>  DNA
<213>  artificial sequence

<220>
<223>  Small Activating RNA

<400>  106
gacacauuuc cccacgaagt t                                                21


<210>  107
<211>  21
<212>  DNA
<213>  artificial sequence

<220>
<223>  Small Activating RNA

<400>  107
acuucguggg gaaaugugut t                                                21


<210>  108
<211>  21
<212>  DNA
<213>  artificial sequence

<220>
<223>  Small Activating RNA

<400>  108
acacauuucc ccacgaagut t                                                21


<210>  109
<211>  21
<212>  DNA
<213>  artificial sequence

<220>
<223>  Small Activating RNA

<400>  109
cacuucgugg ggaaaugugt t                                                21


<210>  110
<211>  21
<212>  DNA
<213>  artificial sequence

<220>
<223>  Small Activating RNA

<400>  110
cacauuuccc cacgaagugt t                                                21


<210>  111
<211>  21
<212>  DNA

<213>  artificial sequence

<220>
<223>  Small Activating RNA

<400>  111
ucacuucgug gggaaaugut t                                                21


<210>  112
<211>  21
<212>  DNA
<213>  artificial sequence

<220>
<223>  Small Activating RNA

<400>  112
acauuucccc acgaagugat t                                                21


<210>  113
<211>  21
<212>  DNA
<213>  artificial sequence

<220>
<223>  Small Activating RNA

<400>  113
cucacuucgu ggggaaaugt t                                                21


<210>  114
<211>  21
<212>  DNA
<213>  artificial sequence

<220>
<223>  Small Activating RNA

<400>  114
cauuucccca cgaagugagt t                                                21


<210>  115
<211>  21
<212>  DNA
<213>  artificial sequence

<220>
<223>  Small Activating RNA

<400>  115
gcucacuucg uggggaaaut t                                                21


<210>  116
<211>  21
<212>  DNA
<213>  artificial sequence

<220>

<223>  Small Activating RNA

<400>  116
auuuccccac gaagugagct t                                                21


<210>  117
<211>  21
<212>  DNA
<213>  artificial sequence

<220>
<223>  Small Activating RNA

<400>  117
ggcucacuuc guggggaaat t                                                21


<210>  118
<211>  21
<212>  DNA
<213>  artificial sequence

<220>
<223>  Small Activating RNA

<400>  118
uuuccccacg aagugagcct t                                                21


<210>  119
<211>  21
<212>  DNA
<213>  artificial sequence

<220>
<223>  Small Activating RNA

<400>  119
uggcucacuu cguggggaat t                                                21


<210>  120
<211>  21
<212>  DNA
<213>  artificial sequence

<220>
<223>  Small Activating RNA

<400>  120
uuccccacga agugagccat t                                                21


<210>  121
<211>  21
<212>  DNA
<213>  artificial sequence

<220>
<223>  Small Activating RNA

<400>  121

guggcucacu ucguggggat t                                                21

<210>  122
<211>  21
<212>  DNA
<213>  artificial sequence

<220>
<223>  Small Activating RNA

<400>  122
uccccacgaa gugagccact t                                                21

<210>  123
<211>  21
<212>  DNA
<213>  artificial sequence

<220>
<223>  Small Activating RNA

<400>  123
uguggcucac uucguggggt t                                                21

<210>  124
<211>  21
<212>  DNA
<213>  artificial sequence

<220>
<223>  Small Activating RNA

<400>  124
ccccacgaag ugagccacat t                                                21

<210>  125
<211>  21
<212>  DNA
<213>  artificial sequence

<220>
<223>  Small Activating RNA

<400>  125
uuuguggcuc acuucguggt t                                                21

<210>  126
<211>  21
<212>  DNA
<213>  artificial sequence

<220>
<223>  Small Activating RNA

<400>  126
ccacgaagug agccacaaat t                                                21

<210>  127
<211>  21
<212>  DNA
<213>  artificial sequence

<220>
<223>  Small Activating RNA

<400>  127
auuuguggcu cacuucgugt t                                                21


<210>  128
<211>  21
<212>  DNA
<213>  artificial sequence

<220>
<223>  Small Activating RNA

<400>  128
cacgaaguga gccacaaaut t                                                21


<210>  129
<211>  21
<212>  DNA
<213>  artificial sequence

<220>
<223>  Small Activating RNA

<400>  129
gauuuguggc ucacuucgut t                                                21


<210>  130
<211>  21
<212>  DNA
<213>  artificial sequence

<220>
<223>  Small Activating RNA

<400>  130
acgaagugag ccacaaauct t                                                21


<210>  131
<211>  21
<212>  DNA
<213>  artificial sequence

<220>
<223>  Small Activating RNA

<400>  131
agauuugugg cucacuucgt t                                                21


<210>  132
<211>  21
<212>  DNA

<213>  artificial sequence

<220>
<223>  Small Activating RNA

<400>  132
cgaagugagc cacaaaucut t                                                21


<210>  133
<211>  21
<212>  DNA
<213>  artificial sequence

<220>
<223>  Small Activating RNA

<400>  133
cagauuugug gcucacuuct t                                                21


<210>  134
<211>  21
<212>  DNA
<213>  artificial sequence

<220>
<223>  Small Activating RNA

<400>  134
gaagugagcc acaaaucugt t                                                21


<210>  135
<211>  21
<212>  DNA
<213>  artificial sequence

<220>
<223>  Small Activating RNA

<400>  135
ccagauuugu ggcucacuut t                                                21


<210>  136
<211>  21
<212>  DNA
<213>  artificial sequence

<220>
<223>  Small Activating RNA

<400>  136
aagugagcca caaaucuggt t                                                21


<210>  137
<211>  21
<212>  DNA
<213>  artificial sequence

<220>

<223>  Small Activating RNA

<400>  137
gccagauuug uggcucacut t                                                21


<210>  138
<211>  21
<212>  DNA
<213>  artificial sequence

<220>
<223>  Small Activating RNA

<400>  138
agugagccac aaaucuggct t                                                21


<210>  139
<211>  21
<212>  DNA
<213>  artificial sequence

<220>
<223>  Small Activating RNA

<400>  139
agccagauuu guggcucact t                                                21


<210>  140
<211>  21
<212>  DNA
<213>  artificial sequence

<220>
<223>  Small Activating RNA

<400>  140
gugagccaca aaucuggcut t                                                21


<210>  141
<211>  21
<212>  DNA
<213>  artificial sequence

<220>
<223>  Small Activating RNA

<400>  141
aagccagauu uguggcucat t                                                21


<210>  142
<211>  21
<212>  DNA
<213>  artificial sequence

<220>
<223>  Small Activating RNA

<400>  142

ugagccacaa aucuggcuut t                                                21

<210>  143
<211>  21
<212>  DNA
<213>  artificial sequence

<220>
<223>  Small Activating RNA

<400>  143
aaagccagau uuguggcuct t                                                21

<210>  144
<211>  21
<212>  DNA
<213>  artificial sequence

<220>
<223>  Small Activating RNA

<400>  144
gagccacaaa ucuggcuuut t                                                21

<210>  145
<211>  21
<212>  DNA
<213>  artificial sequence

<220>
<223>  Small Activating RNA

<400>  145
aaaagccaga uuuguggcut t                                                21

<210>  146
<211>  21
<212>  DNA
<213>  artificial sequence

<220>
<223>  Small Activating RNA

<400>  146
agccacaaau cuggcuuuut t                                                21

<210>  147
<211>  21
<212>  DNA
<213>  artificial sequence

<220>
<223>  Small Activating RNA

<400>  147
caacucauuc uccaaguaat t                                                21

<210>  148
<211>  21
<212>  DNA
<213>  artificial sequence

<220>
<223>  Small Activating RNA

<400>  148
uuacuuggag aaugaguugt t                                                 21


<210>  149
<211>  21
<212>  DNA
<213>  artificial sequence

<220>
<223>  Small Activating RNA

<400>  149
ccaacucauu cuccaaguat t                                                 21


<210>  150
<211>  21
<212>  DNA
<213>  artificial sequence

<220>
<223>  Small Activating RNA

<400>  150
uacuuggaga augaguuggt t                                                 21


<210>  151
<211>  21
<212>  DNA
<213>  artificial sequence

<220>
<223>  Small Activating RNA

<400>  151
gccaacucau ucuccaagut t                                                 21


<210>  152
<211>  21
<212>  DNA
<213>  artificial sequence

<220>
<223>  Small Activating RNA

<400>  152
acuuggagaa ugaguuggct t                                                 21


<210>  153
<211>  21
<212>  DNA

<213>  artificial sequence

<220>
<223>  Small Activating RNA

<400>  153
ugccaacuca uucuccaagt t                                                21


<210>  154
<211>  21
<212>  DNA
<213>  artificial sequence

<220>
<223>  Small Activating RNA

<400>  154
cuuggagaau gaguuggcat t                                                21


<210>  155
<211>  21
<212>  DNA
<213>  artificial sequence

<220>
<223>  Small Activating RNA

<400>  155
gugccaacuc auucuccaat t                                                21


<210>  156
<211>  21
<212>  DNA
<213>  artificial sequence

<220>
<223>  Small Activating RNA

<400>  156
uuggagaaug aguuggcact t                                                21


<210>  157
<211>  21
<212>  DNA
<213>  artificial sequence

<220>
<223>  Small Activating RNA

<400>  157
uggagagugc caacucauut t                                                21


<210>  158
<211>  21
<212>  DNA
<213>  artificial sequence

<220>

<223>  Small Activating RNA

<400>  158
aaugaguugg cacucuccat t                                                21


<210>  159
<211>  21
<212>  DNA
<213>  artificial sequence

<220>
<223>  Small Activating RNA

<400>  159
cuggagagug ccaacucaut t                                                21


<210>  160
<211>  21
<212>  DNA
<213>  artificial sequence

<220>
<223>  Small Activating RNA

<400>  160
augaguuggc acucuccagt t                                                21


<210>  161
<211>  21
<212>  DNA
<213>  artificial sequence

<220>
<223>  Small Activating RNA

<400>  161
ccuggagagu gccaacucat t                                                21


<210>  162
<211>  21
<212>  DNA
<213>  artificial sequence

<220>
<223>  Small Activating RNA

<400>  162
ugaguuggca cucuccaggt t                                                21


<210>  163
<211>  21
<212>  DNA
<213>  artificial sequence

<220>
<223>  Small Activating RNA

<400>  163

uccuggagag ugccaacuct t                                                21

<210>  164
<211>  21
<212>  DNA
<213>  artificial sequence

<220>
<223>  Small Activating RNA

<400>  164
gaguuggcac ucuccaggat t                                                21

<210>  165
<211>  21
<212>  DNA
<213>  artificial sequence

<220>
<223>  Small Activating RNA

<400>  165
cuccuggaga gugccaacut t                                                21

<210>  166
<211>  21
<212>  DNA
<213>  artificial sequence

<220>
<223>  Small Activating RNA

<400>  166
aguuggcacu cuccaggagt t                                                21

<210>  167
<211>  21
<212>  DNA
<213>  artificial sequence

<220>
<223>  Small Activating RNA

<400>  167
ccuccuggag agugccaact t                                                21

<210>  168
<211>  21
<212>  DNA
<213>  artificial sequence

<220>
<223>  Small Activating RNA

<400>  168
guuggcacuc uccaggaggt t                                                21

<210>  169
<211>  21
<212>  DNA
<213>  artificial sequence

<220>
<223>  Small Activating RNA

<400>  169
uccuccugga gagugccaat t                                                21


<210>  170
<211>  21
<212>  DNA
<213>  artificial sequence

<220>
<223>  Small Activating RNA

<400>  170
uuggcacucu ccaggaggat t                                                21


<210>  171
<211>  21
<212>  DNA
<213>  artificial sequence

<220>
<223>  Small Activating RNA

<400>  171
guccuccugg agagugccat t                                                21


<210>  172
<211>  21
<212>  DNA
<213>  artificial sequence

<220>
<223>  Small Activating RNA

<400>  172
uggcacucuc caggaggact t                                                21


<210>  173
<211>  21
<212>  DNA
<213>  artificial sequence

<220>
<223>  Small Activating RNA

<400>  173
uguguccucc uggagagugt t                                                21


<210>  174
<211>  21
<212>  DNA

<213>  artificial sequence

<220>
<223>  Small Activating RNA

<400>  174
cacucuccag gaggacacat t                                                21


<210>  175
<211>  21
<212>  DNA
<213>  artificial sequence

<220>
<223>  Small Activating RNA

<400>  175
cuguguccuc cuggagagut t                                                21


<210>  176
<211>  21
<212>  DNA
<213>  artificial sequence

<220>
<223>  Small Activating RNA

<400>  176
acucuccagg aggacacagt t                                                21


<210>  177
<211>  21
<212>  DNA
<213>  artificial sequence

<220>
<223>  Small Activating RNA

<400>  177
gcuguguccu ccuggagagt t                                                21


<210>  178
<211>  21
<212>  DNA
<213>  artificial sequence

<220>
<223>  Small Activating RNA

<400>  178
cucuccagga ggacacagct t                                                21


<210>  179
<211>  21
<212>  DNA
<213>  artificial sequence

<220>

<223>  Small Activating RNA

<400>  179
   ugcuguguccuccuggagat t                                                    21

<210>  180
<211>  21
<212>  DNA
<213>  artificial sequence

<220>
<223>  Small Activating RNA

<400>  180
ucuccaggag gacacagcat t                                                21

<210>  181
<211>  21
<212>  DNA
<213>  artificial sequence

<220>
<223>  Small Activating RNA

<400>  181
gugcuguguc cuccuggagt t                                                21

<210>  182
<211>  21
<212>  DNA
<213>  artificial sequence

<220>
<223>  Small Activating RNA

<400>  182
cuccaggagg acacagcact t                                                21

<210>  183
<211>  21
<212>  DNA
<213>  artificial sequence

<220>
<223>  Small Activating RNA

<400>  183
agugcugugu ccuccuggat t                                                21

<210>  184
<211>  21
<212>  DNA
<213>  artificial sequence

<220>
<223>  Small Activating RNA

<400>  184

uccaggagga cacagcacut t                                                21

<210>  185
<211>  21
<212>  DNA
<213>  artificial sequence

<220>
<223>  Small Activating RNA

<400>  185
acagugcugu guccuccugt t                                                21

<210>  186
<211>  21
<212>  DNA
<213>  artificial sequence

<220>
<223>  Small Activating RNA

<400>  186
caggaggaca cagcacugut t                                                21

<210>  187
<211>  21
<212>  DNA
<213>  artificial sequence

<220>
<223>  Small Activating RNA

<400>  187
aacagugcug uguccuccut t                                                21

<210>  188
<211>  21
<212>  DNA
<213>  artificial sequence

<220>
<223>  Small Activating RNA

<400>  188
aggaggacac agcacuguut t                                                21

<210>  189
<211>  21
<212>  DNA
<213>  artificial sequence

<220>
<223>  Small Activating RNA

<400>  189
uaacagugcu guguccucct t                                                21

<210>  190
<211>  21
<212>  DNA
<213>  artificial sequence

<220>
<223>  Small Activating RNA

<400>  190
ggaggacaca gcacuguuat t                                                21


<210>  191
<211>  21
<212>  DNA
<213>  artificial sequence

<220>
<223>  Small Activating RNA

<400>  191
cuaacagugc uguguccuct t                                                21


<210>  192
<211>  21
<212>  DNA
<213>  artificial sequence

<220>
<223>  Small Activating RNA

<400>  192
gaggacacag cacuguuagt t                                                21


<210>  193
<211>  21
<212>  DNA
<213>  artificial sequence

<220>
<223>  Small Activating RNA

<400>  193
ucuaacagug cuguguccut t                                                21


<210>  194
<211>  21
<212>  DNA
<213>  artificial sequence

<220>
<223>  Small Activating RNA

<400>  194
aggacacagc acuguuagat t                                                21


<210>  195
<211>  21
<212>  DNA

<213>  artificial sequence

<220>
<223>  Small Activating RNA

<400>  195
ucuuuucagc ugcauugggt t                                                21


<210>  196
<211>  21
<212>  DNA
<213>  artificial sequence

<220>
<223>  Small Activating RNA

<400>  196
cccaaugcag cugaaaagat t                                                21


<210>  197
<211>  21
<212>  DNA
<213>  artificial sequence

<220>
<223>  Small Activating RNA

<400>  197
aucuuuucag cugcauuggt t                                                21


<210>  198
<211>  21
<212>  DNA
<213>  artificial sequence

<220>
<223>  Small Activating RNA

<400>  198
ccaaugcagc ugaaaagaut t                                                21


<210>  199
<211>  21
<212>  DNA
<213>  artificial sequence

<220>
<223>  Small Activating RNA

<400>  199
gaucuuuuca gcugcauugt t                                                21


<210>  200
<211>  21
<212>  DNA
<213>  artificial sequence

<220>

<223>  Small Activating RNA

<400>  200
caaugcagcu gaaaagauct t                                                21


<210>  201
<211>  21
<212>  DNA
<213>  artificial sequence

<220>
<223>  Small Activating RNA

<400>  201
ugaucuuuuc agcugcauut t                                                21


<210>  202
<211>  21
<212>  DNA
<213>  artificial sequence

<220>
<223>  Small Activating RNA

<400>  202
aaugcagcug aaaagaucat t                                                21


<210>  203
<211>  21
<212>  DNA
<213>  artificial sequence

<220>
<223>  Small Activating RNA

<400>  203
cugaucuuuu cagcugcaut t                                                21


<210>  204
<211>  21
<212>  DNA
<213>  artificial sequence

<220>
<223>  Small Activating RNA

<400>  204
augcagcuga aaagaucagt t                                                21


<210>  205
<211>  21
<212>  DNA
<213>  artificial sequence

<220>
<223>  Small Activating RNA

<400>  205

ccugaucuuu ucagcugcat t                                                21

<210>  206
<211>  21
<212>  DNA
<213>  artificial sequence

<220>
<223>  Small Activating RNA

<400>  206
ugcagcugaa aagaucaggt t                                                21

<210>  207
<211>  21
<212>  DNA
<213>  artificial sequence

<220>
<223>  Small Activating RNA

<400>  207
uccugaucuu uucagcugct t                                                21

<210>  208
<211>  21
<212>  DNA
<213>  artificial sequence

<220>
<223>  Small Activating RNA

<400>  208
gcagcugaaa agaucaggat t                                                21

<210>  209
<211>  21
<212>  DNA
<213>  artificial sequence

<220>
<223>  Small Activating RNA

<400>  209
cuccugaucu uuucagcugt t                                                21

<210>  210
<211>  21
<212>  DNA
<213>  artificial sequence

<220>
<223>  Small Activating RNA

<400>  210
cagcugaaaa gaucaggagt t                                                21

<210>  211
<211>  21
<212>  DNA
<213>  artificial sequence

<220>
<223>  Small Activating RNA

<400>  211
ccuccugauc uuuucagcut t                                                21


<210>  212
<211>  21
<212>  DNA
<213>  artificial sequence

<220>
<223>  Small Activating RNA

<400>  212
agcugaaaag aucaggaggt t                                                21


<210>  213
<211>  21
<212>  DNA
<213>  artificial sequence

<220>
<223>  Small Activating RNA

<400>  213
uccuccugau cuuuucagct t                                                21


<210>  214
<211>  21
<212>  DNA
<213>  artificial sequence

<220>
<223>  Small Activating RNA

<400>  214
gcugaaaaga ucaggaggat t                                                21


<210>  215
<211>  21
<212>  DNA
<213>  artificial sequence

<220>
<223>  Small Activating RNA

<400>  215
auccuccuga ucuuuucagt t                                                21


<210>  216
<211>  21
<212>  DNA

<213>  artificial sequence

<220>
<223>  Small Activating RNA

<400>  216
cugaaaagau caggaggaut t                                                21


<210>  217
<211>  21
<212>  DNA
<213>  artificial sequence

<220>
<223>  Small Activating RNA

<400>  217
cauccuccug aucuuuucat t                                                21


<210>  218
<211>  21
<212>  DNA
<213>  artificial sequence

<220>
<223>  Small Activating RNA

<400>  218
ugaaaagauc aggaggaugt t                                                21


<210>  219
<211>  21
<212>  DNA
<213>  artificial sequence

<220>
<223>  Small Activating RNA

<400>  219
ucauccuccu gaucuuuuct t                                                21


<210>  220
<211>  21
<212>  DNA
<213>  artificial sequence

<220>
<223>  Small Activating RNA

<400>  220
gaaaagauca ggaggaugat t                                                21


<210>  221
<211>  21
<212>  DNA
<213>  artificial sequence

<220>

<223>  Small Activating RNA

<400>  221
gucauccucc ugaucuuuut t                                                21


<210>  222
<211>  21
<212>  DNA
<213>  artificial sequence

<220>
<223>  Small Activating RNA

<400>  222
aaaagaucag gaggaugact t                                                21


<210>  223
<211>  21
<212>  DNA
<213>  artificial sequence

<220>
<223>  Small Activating RNA

<400>  223
ugucauccuc cugaucuuut t                                                21


<210>  224
<211>  21
<212>  DNA
<213>  artificial sequence

<220>
<223>  Small Activating RNA

<400>  224
aaagaucagg aggaugacat t                                                21


<210>  225
<211>  21
<212>  DNA
<213>  artificial sequence

<220>
<223>  Small Activating RNA

<400>  225
augucauccu ccugaucuut t                                                21


<210>  226
<211>  21
<212>  DNA
<213>  artificial sequence

<220>
<223>  Small Activating RNA

<400>  226

aagaucagga ggaugacaut t                                                21

<210>  227
<211>  21
<212>  DNA
<213>  artificial sequence

<220>
<223>  Small Activating RNA

<400>  227
aaugucaucc uccugaucut t                                                21

<210>  228
<211>  21
<212>  DNA
<213>  artificial sequence

<220>
<223>  Small Activating RNA

<400>  228
agaucaggag gaugacauut t                                                21

<210>  229
<211>  21
<212>  DNA
<213>  artificial sequence

<220>
<223>  Small Activating RNA

<400>  229
uaaugucauc cuccugauct t                                                21

<210>  230
<211>  21
<212>  DNA
<213>  artificial sequence

<220>
<223>  Small Activating RNA

<400>  230
gaucaggagg augacauuat t                                                21

<210>  231
<211>  21
<212>  DNA
<213>  artificial sequence

<220>
<223>  Small Activating RNA

<400>  231
uuaaugucau ccuccugaut t                                                21

<210>  232
<211>  21
<212>  DNA
<213>  artificial sequence

<220>
<223>  Small Activating RNA

<400>  232
aucaggagga ugacauuaat t                                                21


<210>  233
<211>  21
<212>  DNA
<213>  artificial sequence

<220>
<223>  Small Activating RNA

<400>  233
auuaauguca uccuccugat t                                                21


<210>  234
<211>  21
<212>  DNA
<213>  artificial sequence

<220>
<223>  Small Activating RNA

<400>  234
ucaggaggau gacauuaaut t                                                21


<210>  235
<211>  21
<212>  DNA
<213>  artificial sequence

<220>
<223>  Small Activating RNA

<400>  235
uggggcuuuu cuggaaauut t                                                21


<210>  236
<211>  21
<212>  DNA
<213>  artificial sequence

<220>
<223>  Small Activating RNA

<400>  236
aauuuccaga aaagccccat t                                                21


<210>  237
<211>  21
<212>  DNA

<213>  artificial sequence

<220>
<223>  Small Activating RNA

<400>  237
guggggcuuu ucuggaaaut t                                                21


<210>  238
<211>  21
<212>  DNA
<213>  artificial sequence

<220>
<223>  Small Activating RNA

<400>  238
auuuccagaa aagccccact t                                                21


<210>  239
<211>  21
<212>  DNA
<213>  artificial sequence

<220>
<223>  Small Activating RNA

<400>  239
   uguggggcuu uucuggaaat t                                                21


<210>  240
<211>  21
<212>  DNA
<213>  artificial sequence

<220>
<223>  Small Activating RNA

<400>  240
uuuccagaaa agccccacat t                                                21


<210>  241
<211>  21
<212>  DNA
<213>  artificial sequence

<220>
<223>  Small Activating RNA

<400>  241
uuguggggcu uuucuggaat t                                                21


<210>  242
<211>  21
<212>  DNA
<213>  artificial sequence

<220>

<223>  Small Activating RNA

<400>  242
uuccagaaaa gccccacaat t                                                21


<210>  243
<211>  21
<212>  DNA
<213>  artificial sequence

<220>
<223>  Small Activating RNA

<400>  243
auuguggggc uuuucuggat t                                                21


<210>  244
<211>  21
<212>  DNA
<213>  artificial sequence

<220>
<223>  Small Activating RNA

<400>  244
uccagaaaag ccccacaaut t                                                21


<210>  245
<211>  21
<212>  DNA
<213>  artificial sequence

<220>
<223>  Small Activating RNA

<400>  245
uauugugggg cuuuucuggt t                                                21


<210>  246
<211>  21
<212>  DNA
<213>  artificial sequence

<220>
<223>  Small Activating RNA

<400>  246
ccagaaaagc cccacaauat t                                                21


<210>  247
<211>  21
<212>  DNA
<213>  artificial sequence

<220>
<223>  Small Activating RNA

<400>  247

auauuguggg gcuuuucugt t                                                21

<210>  248
<211>  21
<212>  DNA
<213>  artificial sequence

<220>
<223>  Small Activating RNA

<400>  248
cagaaaagcc ccacaauaut t                                                21

<210>  249
<211>  21
<212>  DNA
<213>  artificial sequence

<220>
<223>  Small Activating RNA

<400>  249
gauauugugg ggcuuuucut t                                                21

<210>  250
<211>  21
<212>  DNA
<213>  artificial sequence

<220>
<223>  Small Activating RNA

<400>  250
agaaaagccc cacaauauct t                                                21

<210>  251
<211>  21
<212>  DNA
<213>  artificial sequence

<220>
<223>  Small Activating RNA

<400>  251
ugauauugug gggcuuuuct t                                                21

<210>  252
<211>  21
<212>  DNA
<213>  artificial sequence

<220>
<223>  Small Activating RNA

<400>  252
gaaaagcccc acaauaucat t                                                21

<210>  253
<211>  21
<212>  DNA
<213>  artificial sequence

<220>
<223>  Small Activating RNA

<400>  253
gugauauugu ggggcuuuut t                                                21


<210>  254
<211>  21
<212>  DNA
<213>  artificial sequence

<220>
<223>  Small Activating RNA

<400>  254
aaaagcccca caauaucact t                                                21


<210>  255
<211>  21
<212>  DNA
<213>  artificial sequence

<220>
<223>  Small Activating RNA

<400>  255
ggugauauug uggggcuuut t                                                21


<210>  256
<211>  21
<212>  DNA
<213>  artificial sequence

<220>
<223>  Small Activating RNA

<400>  256
aaagccccac aauaucacct t                                                21


<210>  257
<211>  21
<212>  DNA
<213>  artificial sequence

<220>
<223>  Small Activating RNA

<400>  257
aggugauauu guggggcuut t                                                21


<210>  258
<211>  21
<212>  DNA

<213>  artificial sequence

<220>
<223>  Small Activating RNA

<400>  258
aagccccaca auaucaccut t                                                21


<210>  259
<211>  21
<212>  DNA
<213>  artificial sequence

<220>
<223>  Small Activating RNA

<400>  259
gggaauagag gugauauugt t                                                21


<210>  260
<211>  21
<212>  DNA
<213>  artificial sequence

<220>
<223>  Small Activating RNA

<400>  260
caauaucacc ucuauuccct t                                                21


<210>  261
<211>  21
<212>  DNA
<213>  artificial sequence

<220>
<223>  Small Activating RNA

<400>  261
ugggaauaga ggugauauut t                                                21


<210>  262
<211>  21
<212>  DNA
<213>  artificial sequence

<220>
<223>  Small Activating RNA

<400>  262
aauaucaccu cuauucccat t                                                21


<210>  263
<211>  21
<212>  DNA
<213>  artificial sequence

<220>

<223>  Small Activating RNA

<400>  263
gugggaauag aggugauaut t                                                21


<210>  264
<211>  21
<212>  DNA
<213>  artificial sequence

<220>
<223>  Small Activating RNA

<400>  264
auaucaccuc uauucccact t                                                21


<210>  265
<211>  21
<212>  DNA
<213>  artificial sequence

<220>
<223>  Small Activating RNA

<400>  265
agugggaaua gaggugauat t                                                21


<210>  266
<211>  21
<212>  DNA
<213>  artificial sequence

<220>
<223>  Small Activating RNA

<400>  266
uaucaccucu auucccacut t                                                21


<210>  267
<211>  21
<212>  DNA
<213>  artificial sequence

<220>
<223>  Small Activating RNA

<400>  267
cagugggaau agaggugaut t                                                21


<210>  268
<211>  21
<212>  DNA
<213>  artificial sequence

<220>
<223>  Small Activating RNA

<400>  268

aucaccucua uucccacugt t                                                21

<210>  269
<211>  21
<212>  DNA
<213>  artificial sequence

<220>
<223>  Small Activating RNA

<400>  269
ucagugggaa uagaggugat t                                                21

<210>  270
<211>  21
<212>  DNA
<213>  artificial sequence

<220>
<223>  Small Activating RNA

<400>  270
ucaccucuau ucccacugat t                                                21

<210>  271
<211>  21
<212>  DNA
<213>  artificial sequence

<220>
<223>  Small Activating RNA

<400>  271
aucaguggga auagaggugt t                                                21

<210>  272
<211>  21
<212>  DNA
<213>  artificial sequence

<220>
<223>  Small Activating RNA

<400>  272
caccucuauu cccacugaut t                                                21

<210>  273
<211>  21
<212>  DNA
<213>  artificial sequence

<220>
<223>  Small Activating RNA

<400>  273
gaucaguggg aauagaggut t                                                21

<210>  274
<211>  21
<212>  DNA
<213>  artificial sequence

<220>
<223>  Small Activating RNA

<400>  274
accucuauuc ccacugauct t                                                21


<210>  275
<211>  21
<212>  DNA
<213>  artificial sequence

<220>
<223>  Small Activating RNA

<400>  275
ggaucagugg gaauagaggt t                                                21


<210>  276
<211>  21
<212>  DNA
<213>  artificial sequence

<220>
<223>  Small Activating RNA

<400>  276
ccucuauucc cacugaucct t                                                21


<210>  277
<211>  21
<212>  DNA
<213>  artificial sequence

<220>
<223>  Small Activating RNA

<400>  277
gggaucagug ggaauagagt t                                                21


<210>  278
<211>  21
<212>  DNA
<213>  artificial sequence

<220>
<223>  Small Activating RNA

<400>  278
cucuauuccc acugauccct t                                                21


<210>  279
<211>  21
<212>  DNA

<213>  artificial sequence

<220>
<223>  Small Activating RNA

<400>  279
agggaucagu gggaauagat t                                                21


<210>  280
<211>  21
<212>  DNA
<213>  artificial sequence

<220>
<223>  Small Activating RNA

<400>  280
ucuauuccca cugaucccut t                                                21


<210>  281
<211>  21
<212>  DNA
<213>  artificial sequence

<220>
<223>  Small Activating RNA

<400>  281
gagggaucag ugggaauagt t                                                21


<210>  282
<211>  21
<212>  DNA
<213>  artificial sequence

<220>
<223>  Small Activating RNA

<400>  282
cuauucccac ugaucccuct t                                                21


<210>  283
<211>  21
<212>  DNA
<213>  artificial sequence

<220>
<223>  Small Activating RNA

<400>  283
ugagggauca gugggaauat t                                                21


<210>  284
<211>  21
<212>  DNA
<213>  artificial sequence

<220>

<223>  Small Activating RNA

<400>  284
uauucccacu gaucccucat t                                                21


<210>  285
<211>  21
<212>  DNA
<213>  artificial sequence

<220>
<223>  Small Activating RNA

<400>  285
gugagggauc agugggaaut t                                                21


<210>  286
<211>  21
<212>  DNA
<213>  artificial sequence

<220>
<223>  Small Activating RNA

<400>  286
auucccacug aucccucact t                                                21


<210>  287
<211>  21
<212>  DNA
<213>  artificial sequence

<220>
<223>  Small Activating RNA

<400>  287
agugagggau cagugggaat t                                                21


<210>  288
<211>  21
<212>  DNA
<213>  artificial sequence

<220>
<223>  Small Activating RNA

<400>  288
uucccacuga ucccucacut t                                                21


<210>  289
<211>  21
<212>  DNA
<213>  artificial sequence

<220>
<223>  Small Activating RNA

<400>  289

uagugaggga ucagugggat t                                                21

<210>  290
<211>  21
<212>  DNA
<213>  artificial sequence

<220>
<223>  Small Activating RNA

<400>  290
ucccacugau cccucacuat t                                                21

<210>  291
<211>  21
<212>  DNA
<213>  artificial sequence

<220>
<223>  Small Activating RNA

<400>  291
cuagugaggg aucagugggt t                                                21

<210>  292
<211>  21
<212>  DNA
<213>  artificial sequence

<220>
<223>  Small Activating RNA

<400>  292
cccacugauc ccucacuagt t                                                21

<210>  293
<211>  21
<212>  DNA
<213>  artificial sequence

<220>
<223>  Small Activating RNA

<400>  293
accuagugag ggaucagugt t                                                21

<210>  294
<211>  21
<212>  DNA
<213>  artificial sequence

<220>
<223>  Small Activating RNA

<400>  294
cacugauccc ucacuaggut t                                                21

<210>  295
<211>  21
<212>  DNA
<213>  artificial sequence

<220>
<223>  Small Activating RNA

<400>  295
gaccuaguga gggaucagut t                                                21


<210>  296
<211>  21
<212>  DNA
<213>  artificial sequence

<220>
<223>  Small Activating RNA

<400>  296
acugaucccu cacuagguct t                                                21


<210>  297
<211>  21
<212>  DNA
<213>  artificial sequence

<220>
<223>  Small Activating RNA

<400>  297
ugaccuagug agggaucagt t                                                21


<210>  298
<211>  21
<212>  DNA
<213>  artificial sequence

<220>
<223>  Small Activating RNA

<400>  298
cugaucccuc acuaggucat t                                                21


<210>  299
<211>  21
<212>  DNA
<213>  artificial sequence

<220>
<223>  Small Activating RNA

<400>  299
gugaccuagu gagggaucat t                                                21


<210>  300
<211>  21
<212>  DNA

<213>  artificial sequence

<220>
<223>  Small Activating RNA

<400>  300
ugaucccuca cuaggucact t                                                21


<210>  301
<211>  21
<212>  DNA
<213>  artificial sequence

<220>
<223>  Small Activating RNA

<400>  301
ggugaccuag ugagggauct t                                                21


<210>  302
<211>  21
<212>  DNA
<213>  artificial sequence

<220>
<223>  Small Activating RNA

<400>  302
gaucccucac uaggucacct t                                                21


<210>  303
<211>  21
<212>  DNA
<213>  artificial sequence

<220>
<223>  Small Activating RNA

<400>  303
aggugaccua gugagggaut t                                                21


<210>  304
<211>  21
<212>  DNA
<213>  artificial sequence

<220>
<223>  Small Activating RNA

<400>  304
aucccucacu aggucaccut t                                                21


<210>  305
<211>  21
<212>  DNA
<213>  artificial sequence

<220>

<223>  Small Activating RNA

<400>  305
gaggugaccu agugagggat t                                                21


<210>  306
<211>  21
<212>  DNA
<213>  artificial sequence

<220>
<223>  Small Activating RNA

<400>  306
ucccucacua ggucaccuct t                                                21


<210>  307
<211>  21
<212>  DNA
<213>  artificial sequence

<220>
<223>  Small Activating RNA

<400>  307
ggagagguga ccuagugagt t                                                21


<210>  308
<211>  21
<212>  DNA
<213>  artificial sequence

<220>
<223>  Small Activating RNA

<400>  308
cucacuaggu caccucucct t                                                21


<210>  309
<211>  21
<212>  DNA
<213>  artificial sequence

<220>
<223>  Small Activating RNA

<400>  309
gggagaggug accuagugat t                                                21


<210>  310
<211>  21
<212>  DNA
<213>  artificial sequence

<220>
<223>  Small Activating RNA

<400>  310

ucacuagguc accucuccct t                                                21


<210>  311
<211>  21
<212>  DNA
<213>  artificial sequence

<220>
<223>  Small Activating RNA

<400>  311
ugggagaggu gaccuagugt t                                                21


<210>  312
<211>  21
<212>  DNA
<213>  artificial sequence

<220>
<223>  Small Activating RNA

<400>  312
cacuagguca ccucucccat t                                                21


<210>  313
<211>  21
<212>  DNA
<213>  artificial sequence

<220>
<223>  Small Activating RNA

<400>  313
cugggagagg ugaccuagut t                                                21


<210>  314
<211>  21
<212>  DNA
<213>  artificial sequence

<220>
<223>  Small Activating RNA

<400>  314
acuaggucac cucucccagt t                                                21


<210>  315
<211>  21
<212>  DNA
<213>  artificial sequence

<220>
<223>  Small Activating RNA

<400>  315
ucugggagag gugaccuagt t                                                21

<210>  316
<211>  21
<212>  DNA
<213>  artificial sequence

<220>
<223>  Small Activating RNA

<400>  316
cuaggucacc ucucccagat t                                                21


<210>  317
<211>  21
<212>  DNA
<213>  artificial sequence

<220>
<223>  Small Activating RNA

<400>  317
uucugggaga ggugaccuat t                                                21


<210>  318
<211>  21
<212>  DNA
<213>  artificial sequence

<220>
<223>  Small Activating RNA

<400>  318
uaggucaccu cucccagaat t                                                21


<210>  319
<211>  21
<212>  DNA
<213>  artificial sequence

<220>
<223>  Small Activating RNA

<400>  319
aggugcucca ggugcuucut t                                                21


<210>  320
<211>  21
<212>  DNA
<213>  artificial sequence

<220>
<223>  Small Activating RNA

<400>  320
agaagcaccu ggagcaccut t                                                21


<210>  321
<211>  21
<212>  DNA

<213>  artificial sequence

<220>
<223>  Small Activating RNA

<400>  321
uaggugcucc aggugcuuct t                                                21


<210>  322
<211>  21
<212>  DNA
<213>  artificial sequence

<220>
<223>  Small Activating RNA

<400>  322
gaagcaccug gagcaccuat t                                                21


<210>  323
<211>  21
<212>  DNA
<213>  artificial sequence

<220>
<223>  Small Activating RNA

<400>  323
ugucuaggug cuccaggugt t                                                21


<210>  324
<211>  21
<212>  DNA
<213>  artificial sequence

<220>
<223>  Small Activating RNA

<400>  324
caccuggagc accuagacat t                                                21


<210>  325
<211>  21
<212>  DNA
<213>  artificial sequence

<220>
<223>  Small Activating RNA

<400>  325
ggugucuagg ugcuccaggt t                                                21


<210>  326
<211>  21
<212>  DNA
<213>  artificial sequence

<220>

<223>  Small Activating RNA

<400>  326
ccuggagcac cuagacacct t                                                21


<210>  327
<211>  21
<212>  DNA
<213>  artificial sequence

<220>
<223>  Small Activating RNA

<400>  327
gggugucuag gugcuccagt t                                                21


<210>  328
<211>  21
<212>  DNA
<213>  artificial sequence

<220>
<223>  Small Activating RNA

<400>  328
cuggagcacc uagacaccct t                                                21


<210>  329
<211>  21
<212>  DNA
<213>  artificial sequence

<220>
<223>  Small Activating RNA

<400>  329
ggggugucua ggugcuccat t                                                21


<210>  330
<211>  21
<212>  DNA
<213>  artificial sequence

<220>
<223>  Small Activating RNA

<400>  330
uggagcaccu agacacccct t                                                21


<210>  331
<211>  21
<212>  DNA
<213>  artificial sequence

<220>
<223>  Small Activating RNA

<400>  331

uggggugucu aggugcucct t                                                21

<210>  332
<211>  21
<212>  DNA
<213>  artificial sequence

<220>
<223>  Small Activating RNA

<400>  332
ggagcaccua gacaccccat t                                                21

<210>  333
<211>  21
<212>  DNA
<213>  artificial sequence

<220>
<223>  Small Activating RNA

<400>  333
   uuggggugug uaggugcuct t                                              21

<210>  334
<211>  21
<212>  DNA
<213>  artificial sequence

<220>
<223>  Small Activating RNA

<400>  334
gagcaccuag acaccccaat t                                                21

<210>  335
<211>  21
<212>  DNA
<213>  artificial sequence

<220>
<223>  Small Activating RNA

<400>  335
uuguuggggu gucuaggugt t                                                21

<210>  336
<211>  21
<212>  DNA
<213>  artificial sequence

<220>
<223>  Small Activating RNA

<400>  336
caccuagaca ccccaacaat t                                                21

<210>  337
<211>  21
<212>  DNA
<213>  artificial sequence

<220>
<223>  Small Activating RNA

<400>  337
uuuguugggg ugucuaggut t                                                21


<210>  338
<211>  21
<212>  DNA
<213>  artificial sequence

<220>
<223>  Small Activating RNA

<400>  338
accuagacac cccaacaaat t                                                21


<210>  339
<211>  21
<212>  DNA
<213>  artificial sequence

<220>
<223>  Small Activating RNA

<400>  339
cuuuguuggg gugucuaggt t                                                21


<210>  340
<211>  21
<212>  DNA
<213>  artificial sequence

<220>
<223>  Small Activating RNA

<400>  340
ccuagacacc ccaacaaagt t                                                21


<210>  341
<211>  21
<212>  DNA
<213>  artificial sequence

<220>
<223>  Small Activating RNA

<400>  341
aguggaccuc aauuuccuct t                                                21


<210>  342
<211>  21
<212>  DNA

<213>  artificial sequence

<220>
<223>  Small Activating RNA

<400>  342
gaggaaauug agguccacut t                                                21


<210>  343
<211>  21
<212>  DNA
<213>  artificial sequence

<220>
<223>  Small Activating RNA

<400>  343
caguggaccu caauuuccut t                                                21


<210>  344
<211>  21
<212>  DNA
<213>  artificial sequence

<220>
<223>  Small Activating RNA

<400>  344
aggaaauuga gguccacugt t                                                21


<210>  345
<211>  21
<212>  DNA
<213>  artificial sequence

<220>
<223>  Small Activating RNA

<400>  345
ucaguggacc ucaauuucct t                                                21


<210>  346
<211>  21
<212>  DNA
<213>  artificial sequence

<220>
<223>  Small Activating RNA

<400>  346
ggaaauugag guccacugat t                                                21


<210>  347
<211>  21
<212>  DNA
<213>  artificial sequence

<220>

<223>  Small Activating RNA

<400>  347
uucaguggac cucaauuuct t                                                21


<210>  348
<211>  21
<212>  DNA
<213>  artificial sequence

<220>
<223>  Small Activating RNA

<400>  348
gaaauugagg uccacugaat t                                                21


<210>  349
<211>  21
<212>  DNA
<213>  artificial sequence

<220>
<223>  Small Activating RNA

<400>  349
guucagugga ccucaauuut t                                                21


<210>  350
<211>  21
<212>  DNA
<213>  artificial sequence

<220>
<223>  Small Activating RNA

<400>  350
aaauugaggu ccacugaact t                                                21


<210>  351
<211>  21
<212>  DNA
<213>  artificial sequence

<220>
<223>  Small Activating RNA

<400>  351
aguucagugg accucaauut t                                                21


<210>  352
<211>  21
<212>  DNA
<213>  artificial sequence

<220>
<223>  Small Activating RNA

<400>  352

aauugagguc cacugaacut t                                                21

<210>  353
<211>  21
<212>  DNA
<213>  artificial sequence

<220>
<223>  Small Activating RNA

<400>  353
aaguucagug gaccucaaut t                                                21

<210>  354
<211>  21
<212>  DNA
<213>  artificial sequence

<220>
<223>  Small Activating RNA

<400>  354
auugaggucc acugaacuut t                                                21

<210>  355
<211>  21
<212>  DNA
<213>  artificial sequence

<220>
<223>  Small Activating RNA

<400>  355
uaaguucagu ggaccucaat t                                                21

<210>  356
<211>  21
<212>  DNA
<213>  artificial sequence

<220>
<223>  Small Activating RNA

<400>  356
uugaggucca cugaacuuat t                                                21

<210>  357
<211>  21
<212>  DNA
<213>  artificial sequence

<220>
<223>  Small Activating RNA

<400>  357
uuaaguucag uggaccucat t                                                21

<210>  358
<211>  21
<212>  DNA
<213>  artificial sequence

<220>
<223>  Small Activating RNA

<400>  358
ugagguccac ugaacuuaat t                                                21


<210>  359
<211>  21
<212>  DNA
<213>  artificial sequence

<220>
<223>  Small Activating RNA

<400>  359
cuuaaguuca guggaccuct t                                                21


<210>  360
<211>  21
<212>  DNA
<213>  artificial sequence

<220>
<223>  Small Activating RNA

<400>  360
gagguccacu gaacuuaagt t                                                21


<210>  361
<211>  21
<212>  DNA
<213>  artificial sequence

<220>
<223>  Small Activating RNA

<400>  361
acuuaaguuc aguggaccut t                                                21


<210>  362
<211>  21
<212>  DNA
<213>  artificial sequence

<220>
<223>  Small Activating RNA

<400>  362
agguccacug aacuuaagut t                                                21


<210>  363
<211>  21
<212>  DNA

<213>  artificial sequence

<220>
<223>  Small Activating RNA

<400>  363
uacuuaaguu caguggacct t                                                21


<210>  364
<211>  21
<212>  DNA
<213>  artificial sequence

<220>
<223>  Small Activating RNA

<400>  364
gguccacuga acuuaaguat t                                                21


<210>  365
<211>  21
<212>  DNA
<213>  artificial sequence

<220>
<223>  Small Activating RNA

<400>  365
auacuuaagu ucaguggact t                                                21


<210>  366
<211>  21
<212>  DNA
<213>  artificial sequence

<220>
<223>  Small Activating RNA

<400>  366
guccacugaa cuuaaguaut t                                                21


<210>  367
<211>  21
<212>  DNA
<213>  artificial sequence

<220>
<223>  Small Activating RNA

<400>  367
gugacaauca acaacuuugt t                                                21


<210>  368
<211>  21
<212>  DNA
<213>  artificial sequence

<220>

<223>  Small Activating RNA

<400>  368
caaaguuguu gauugucact t                                                21


<210>  369
<211>  21
<212>  DNA
<213>  artificial sequence

<220>
<223>  Small Activating RNA

<400>  369
caugugacaa ucaacaacut t                                                21


<210>  370
<211>  21
<212>  DNA
<213>  artificial sequence

<220>
<223>  Small Activating RNA

<400>  370
aguuguugau ugucacaugt t                                                21


<210>  371
<211>  21
<212>  DNA
<213>  artificial sequence

<220>
<223>  Small Activating RNA

<400>  371
gcaugugaca aucaacaact t                                                21


<210>  372
<211>  21
<212>  DNA
<213>  artificial sequence

<220>
<223>  Small Activating RNA

<400>  372
guuguugauu gucacaugct t                                                21


<210>  373
<211>  21
<212>  DNA
<213>  artificial sequence

<220>
<223>  Small Activating RNA

<400>  373

agcaugugac aaucaacaat t                                                21

<210>  374
<211>  21
<212>  DNA
<213>  artificial sequence

<220>
<223>  Small Activating RNA

<400>  374
uuguugauug ucacaugcut t                                                21

<210>  375
<211>  21
<212>  DNA
<213>  artificial sequence

<220>
<223>  Small Activating RNA

<400>  375
aagcauguga caaucaacat t                                                21

<210>  376
<211>  21
<212>  DNA
<213>  artificial sequence

<220>
<223>  Small Activating RNA

<400>  376
uguugauugu cacaugcuut t                                                21

<210>  377
<211>  21
<212>  DNA
<213>  artificial sequence

<220>
<223>  Small Activating RNA

<400>  377
gaagcaugug acaaucaact t                                                21

<210>  378
<211>  21
<212>  DNA
<213>  artificial sequence

<220>
<223>  Small Activating RNA

<400>  378
guugauuguc acaugcuuct t                                                21

<210>  379
<211>  21
<212>  DNA
<213>  artificial sequence

<220>
<223>  Small Activating RNA

<400>  379
ggaagcaugu gacaaucaat t                                                21


<210>  380
<211>  21
<212>  DNA
<213>  artificial sequence

<220>
<223>  Small Activating RNA

<400>  380
uugauuguca caugcuucct t                                                21


<210>  381
<211>  21
<212>  DNA
<213>  artificial sequence

<220>
<223>  Small Activating RNA

<400>  381
cggaagcaug ugacaaucat t                                                21


<210>  382
<211>  21
<212>  DNA
<213>  artificial sequence

<220>
<223>  Small Activating RNA

<400>  382
ugauugucac augcuuccgt t                                                21


<210>  383
<211>  21
<212>  DNA
<213>  artificial sequence

<220>
<223>  Small Activating RNA

<400>  383
ccggaagcau gugacaauct t                                                21


<210>  384
<211>  21
<212>  DNA

<213>  artificial sequence

<220>
<223>  Small Activating RNA

<400>  384
gauugucaca ugcuuccggt t                                                21


<210>  385
<211>  21
<212>  DNA
<213>  artificial sequence

<220>
<223>  Small Activating RNA

<400>  385
cccggaagca ugugacaaut t                                                21


<210>  386
<211>  21
<212>  DNA
<213>  artificial sequence

<220>
<223>  Small Activating RNA

<400>  386
auugucacau gcuuccgggt t                                                21


<210>  387
<211>  21
<212>  DNA
<213>  artificial sequence

<220>
<223>  Small Activating RNA

<400>  387
ucccggaagc augugacaat t                                                21


<210>  388
<211>  21
<212>  DNA
<213>  artificial sequence

<220>
<223>  Small Activating RNA

<400>  388
uugucacaug cuuccgggat t                                                21


<210>  389
<211>  21
<212>  DNA
<213>  artificial sequence

<220>

<223>  Small Activating RNA

<400>  389
uucccggaag caugugacat t                                                21


<210>  390
<211>  21
<212>  DNA
<213>  artificial sequence

<220>
<223>  Small Activating RNA

<400>  390
ugucacaugc uuccgggaat t                                                21


<210>  391
<211>  21
<212>  DNA
<213>  artificial sequence

<220>
<223>  Small Activating RNA

<400>  391
cuucccggaa gcaugugact t                                                21


<210>  392
<211>  21
<212>  DNA
<213>  artificial sequence

<220>
<223>  Small Activating RNA

<400>  392
gucacaugcu uccgggaagt t                                                21


<210>  393
<211>  21
<212>  DNA
<213>  artificial sequence

<220>
<223>  Small Activating RNA

<400>  393
ccuucccgga agcaugugat t                                                21


<210>  394
<211>  21
<212>  DNA
<213>  artificial sequence

<220>
<223>  Small Activating RNA

<400>  394

ucacaugcuu ccgggaaggt t                                                21

<210>  395
<211>  21
<212>  DNA
<213>  artificial sequence

<220>
<223>  Small Activating RNA

<400>  395
uccuucccgg aagcaugugt t                                                21

<210>  396
<211>  21
<212>  DNA
<213>  artificial sequence

<220>
<223>  Small Activating RNA

<400>  396
cacaugcuuc cgggaaggat t                                                21

<210>  397
<211>  21
<212>  DNA
<213>  artificial sequence

<220>
<223>  Small Activating RNA

<400>  397
cuccuucccg gaagcaugut t                                                21

<210>  398
<211>  21
<212>  DNA
<213>  artificial sequence

<220>
<223>  Small Activating RNA

<400>  398
acaugcuucc gggaaggagt t                                                21

<210>  399
<211>  21
<212>  DNA
<213>  artificial sequence

<220>
<223>  Small Activating RNA

<400>  399
ccuccuuccc ggaagcaugt t                                                21

<210>  400
<211>  21
<212>  DNA
<213>  artificial sequence

<220>
<223>  Small Activating RNA

<400>  400
caugcuuccg ggaaggaggt t                                                21


<210>  401
<211>  21
<212>  DNA
<213>  artificial sequence

<220>
<223>  Small Activating RNA

<400>  401
ccucccuucc cggaagcaut t                                                21


<210>  402
<211>  21
<212>  DNA
<213>  artificial sequence

<220>
<223>  Small Activating RNA

<400>  402
augcuuccgg gaaggagggt t                                                21


<210>  403
<211>  21
<212>  DNA
<213>  artificial sequence

<220>
<223>  Small Activating RNA

<400>  403
ucccuccuuc ccggaagcat t                                                21


<210>  404
<211>  21
<212>  DNA
<213>  artificial sequence

<220>
<223>  Small Activating RNA

<400>  404
ugcuuccggg aaggagggat t                                                21


<210>  405
<211>  21
<212>  DNA

<213>  artificial sequence

<220>
<223>  Small Activating RNA

<400>  405
auucccuccu ucccggaagt t                                                21


<210>  406
<211>  21
<212>  DNA
<213>  artificial sequence

<220>
<223>  Small Activating RNA

<400>  406
cuucccggaa ggagggaaut t                                                21


<210>  407
<211>  21
<212>  DNA
<213>  artificial sequence

<220>
<223>  Small Activating RNA

<400>  407
aauucccucc uucccggaat t                                                21


<210>  408
<211>  21
<212>  DNA
<213>  artificial sequence

<220>
<223>  Small Activating RNA

<400>  408
uuccgggaag gagggaauut t                                                21


<210>  409
<211>  21
<212>  DNA
<213>  artificial sequence

<220>
<223>  Small Activating RNA

<400>  409
ucuccaauuc ccuccuucct t                                                21


<210>  410
<211>  21
<212>  DNA
<213>  artificial sequence

<220>

<223>  Small Activating RNA

<400>  410
ggaaggaggg aauuggagat t                                                21


<210>  411
<211>  21
<212>  DNA
<213>  artificial sequence

<220>
<223>  Small Activating RNA

<400>  411
cucuccaauu cccuccuuct t                                                21


<210>  412
<211>  21
<212>  DNA
<213>  artificial sequence

<220>
<223>  Small Activating RNA

<400>  412
gaaggaggga auuggagagt t                                                21


<210>  413
<211>  21
<212>  DNA
<213>  artificial sequence

<220>
<223>  Small Activating RNA

<400>  413
ucucuccaau ucccuccuut t                                                21


<210>  414
<211>  21
<212>  DNA
<213>  artificial sequence

<220>
<223>  Small Activating RNA

<400>  414
aaggagggaa uuggagagat t                                                21


<210>  415
<211>  21
<212>  DNA
<213>  artificial sequence

<220>
<223>  Small Activating RNA

<400>  415

gucucuccaa uucccuccut t                                                21

<210>  416
<211>  21
<212>  DNA
<213>  artificial sequence

<220>
<223>  Small Activating RNA

<400>  416
aggagggaau uggagagact t                                                21

<210>  417
<211>  21
<212>  DNA
<213>  artificial sequence

<220>
<223>  Small Activating RNA

<400>  417
agucucucca auucccucct t                                                21

<210>  418
<211>  21
<212>  DNA
<213>  artificial sequence

<220>
<223>  Small Activating RNA

<400>  418
ggagggaauu ggagagacut t                                                21

<210>  419
<211>  21
<212>  DNA
<213>  artificial sequence

<220>
<223>  Small Activating RNA

<400>  419
uagucucucc aauucccuct t                                                21

<210>  420
<211>  21
<212>  DNA
<213>  artificial sequence

<220>
<223>  Small Activating RNA

<400>  420
gagggaauug gagagacuat t                                                21

<210>  421
<211>  21
<212>  DNA
<213>  artificial sequence

<220>
<223>  Small Activating RNA

<400>  421
guagucucuc caauucccut t                                                21


<210>  422
<211>  21
<212>  DNA
<213>  artificial sequence

<220>
<223>  Small Activating RNA

<400>  422
agggaauugg agagacuact t                                                21


<210>  423
<211>  21
<212>  DNA
<213>  artificial sequence

<220>
<223>  Small Activating RNA

<400>  423
gguagucucu ccaauuccct t                                                21


<210>  424
<211>  21
<212>  DNA
<213>  artificial sequence

<220>
<223>  Small Activating RNA

<400>  424
gggaauugga gagacuacct t                                                21


<210>  425
<211>  21
<212>  DNA
<213>  artificial sequence

<220>
<223>  Small Activating RNA

<400>  425
ugcaaccaca gggauuucut t                                                21


<210>  426
<211>  21
<212>  DNA

<213>  artificial sequence

<220>
<223>  Small Activating RNA

<400>  426
agaaaucccu gugguugcat t                                                21


<210>  427
<211>  21
<212>  DNA
<213>  artificial sequence

<220>
<223>  Small Activating RNA

<400>  427
cugcaaccac agggauuuct t                                                21


<210>  428
<211>  21
<212>  DNA
<213>  artificial sequence

<220>
<223>  Small Activating RNA

<400>  428
gaaaucccug ugguugcagt t                                                21


<210>  429
<211>  21
<212>  DNA
<213>  artificial sequence

<220>
<223>  Small Activating RNA

<400>  429
gcugcaacca cagggauuut t                                                21


<210>  430
<211>  21
<212>  DNA
<213>  artificial sequence

<220>
<223>  Small Activating RNA

<400>  430
aaaucccugu gguugcagct t                                                21


<210>  431
<211>  21
<212>  DNA
<213>  artificial sequence

<220>

<223>  Small Activating RNA

<400>  431
ugcugcaacc acagggauut t                                                21


<210>  432
<211>  21
<212>  DNA
<213>  artificial sequence

<220>
<223>  Small Activating RNA

<400>  432
aaucccugug guugcagcat t                                                21


<210>  433
<211>  21
<212>  DNA
<213>  artificial sequence

<220>
<223>  Small Activating RNA

<400>  433
cugcugcaac cacagggaut t                                                21


<210>  434
<211>  21
<212>  DNA
<213>  artificial sequence

<220>
<223>  Small Activating RNA

<400>  434
aucccugugg uugcagcagt t                                                21


<210>  435
<211>  21
<212>  DNA
<213>  artificial sequence

<220>
<223>  Small Activating RNA

<400>  435
gcugcugcaa ccacagggat t                                                21


<210>  436
<211>  21
<212>  DNA
<213>  artificial sequence

<220>
<223>  Small Activating RNA

<400>  436

ucccuguggu ugcagcagct t                                                21

<210>  437
<211>  21
<212>  DNA
<213>  artificial sequence

<220>
<223>  Small Activating RNA

<400>  437
agcugcugca accacagggt t                                                21

<210>  438
<211>  21
<212>  DNA
<213>  artificial sequence

<220>
<223>  Small Activating RNA

<400>  438
cccugugguu gcagcagcut t                                                21

<210>  439
<211>  21
<212>  DNA
<213>  artificial sequence

<220>
<223>  Small Activating RNA

<400>  439
aaagcugcug caaccacagt t                                                21

<210>  440
<211>  21
<212>  DNA
<213>  artificial sequence

<220>
<223>  Small Activating RNA

<400>  440
cugugguugc agcagcuuut t                                                21

<210>  441
<211>  21
<212>  DNA
<213>  artificial sequence

<220>
<223>  Small Activating RNA

<400>  441
caaagcugcu gcaaccacat t                                                21

<210>  442
<211>  21
<212>  DNA
<213>  artificial sequence

<220>
<223>  Small Activating RNA

<400>  442
ugugguugca gcagcuuugt t                                                21


<210>  443
<211>  21
<212>  DNA
<213>  artificial sequence

<220>
<223>  Small Activating RNA

<400>  443
acaaagcugc ugcaaccact t                                                21


<210>  444
<211>  21
<212>  DNA
<213>  artificial sequence

<220>
<223>  Small Activating RNA

<400>  444
gugguugcag cagcuuugut t                                                21


<210>  445
<211>  21
<212>  DNA
<213>  artificial sequence

<220>
<223>  Small Activating RNA

<400>  445
aacaaagcug cugcaaccat t                                                21


<210>  446
<211>  21
<212>  DNA
<213>  artificial sequence

<220>
<223>  Small Activating RNA

<400>  446
ugguugcagc agcuuuguut t                                                21


<210>  447
<211>  21
<212>  DNA

<213>  artificial sequence

<220>
<223>  Small Activating RNA

<400>  447
caacaaagcu gcugcaacct t                                                21


<210>  448
<211>  21
<212>  DNA
<213>  artificial sequence

<220>
<223>  Small Activating RNA

<400>  448
gguugcagca gcuuuguugt t                                                21


<210>  449
<211>  21
<212>  DNA
<213>  artificial sequence

<220>
<223>  Small Activating RNA

<400>  449
ccaacaaagc ugcugcaact t                                                21


<210>  450
<211>  21
<212>  DNA
<213>  artificial sequence

<220>
<223>  Small Activating RNA

<400>  450
guugcagcag cuuuguuggt t                                                21


<210>  451
<211>  21
<212>  DNA
<213>  artificial sequence

<220>
<223>  Small Activating RNA

<400>  451
gccaacaaag cugcugcaat t                                                21


<210>  452
<211>  21
<212>  DNA
<213>  artificial sequence

<220>

<223>  Small Activating RNA

<400>  452
uugcagcagc uuuguuggct t                                                21


<210>  453
<211>  21
<212>  DNA
<213>  artificial sequence

<220>
<223>  Small Activating RNA

<400>  453
ggccaacaaa gcugcugcat t                                                21


<210>  454
<211>  21
<212>  DNA
<213>  artificial sequence

<220>
<223>  Small Activating RNA

<400>  454
ugcagcagcu uuguuggcct t                                                21


<210>  455
<211>  21
<212>  DNA
<213>  artificial sequence

<220>
<223>  Small Activating RNA

<400>  455
uggccaacaa agcugcugct t                                                21


<210>  456
<211>  21
<212>  DNA
<213>  artificial sequence

<220>
<223>  Small Activating RNA

<400>  456
gcagcagcuu uguuggccat t                                                21


<210>  457
<211>  21
<212>  DNA
<213>  artificial sequence

<220>
<223>  Small Activating RNA

<400>  457

cuggccaaca aagcugcugt t                                                21

<210>  458
<211>  21
<212>  DNA
<213>  artificial sequence

<220>
<223>  Small Activating RNA

<400>  458
cagcagcuuu guuggccagt t                                                21

<210>  459
<211>  21
<212>  DNA
<213>  artificial sequence

<220>
<223>  Small Activating RNA

<400>  459
ccuggccaac aaagcugcut t                                                21

<210>  460
<211>  21
<212>  DNA
<213>  artificial sequence

<220>
<223>  Small Activating RNA

<400>  460
agcagcuuug uuggccaggt t                                                21

<210>  461
<211>  21
<212>  DNA
<213>  artificial sequence

<220>
<223>  Small Activating RNA

<400>  461
uuccuggcca acaaagcugt t                                                21

<210>  462
<211>  21
<212>  DNA
<213>  artificial sequence

<220>
<223>  Small Activating RNA

<400>  462
cagcuuuguu ggccaggaat t                                                21

<210>  463
<211>  21
<212>  DNA
<213>  artificial sequence

<220>
<223>  Small Activating RNA

<400>  463
cuuccuggcc aacaaagcut t                                                21


<210>  464
<211>  21
<212>  DNA
<213>  artificial sequence

<220>
<223>  Small Activating RNA

<400>  464
agcuuuguug gccaggaagt t                                                21


<210>  465
<211>  21
<212>  DNA
<213>  artificial sequence

<220>
<223>  Small Activating RNA

<400>  465
cccuuccugg ccaacaaagt t                                                21


<210>  466
<211>  21
<212>  DNA
<213>  artificial sequence

<220>
<223>  Small Activating RNA

<400>  466
cuuuguuggc caggaagggt t                                                21


<210>  467
<211>  21
<212>  DNA
<213>  artificial sequence

<220>
<223>  Small Activating RNA

<400>  467
ccccuuccug gccaacaaat t                                                21


<210>  468
<211>  21
<212>  DNA

<213>  artificial sequence

<220>
<223>  Small Activating RNA

<400>  468
uuuguuggcc aggaaggggt t                                                21


<210>  469
<211>  21
<212>  DNA
<213>  artificial sequence

<220>
<223>  Small Activating RNA

<400>  469
uccccuuccu ggccaacaat t                                                21


<210>  470
<211>  21
<212>  DNA
<213>  artificial sequence

<220>
<223>  Small Activating RNA

<400>  470
uuguuggcca ggaaggggat t                                                21


<210>  471
<211>  21
<212>  DNA
<213>  artificial sequence

<220>
<223>  Small Activating RNA

<400>  471
cuccccuucc uggccaacat t                                                21


<210>  472
<211>  21
<212>  DNA
<213>  artificial sequence

<220>
<223>  Small Activating RNA

<400>  472
uguuggccag gaaggggagt t                                                21


<210>  473
<211>  21
<212>  DNA
<213>  artificial sequence

<220>

<223>  Small Activating RNA

<400>  473
uccuccccuu ccuggccaat t                                                21


<210>  474
<211>  21
<212>  DNA
<213>  artificial sequence

<220>
<223>  Small Activating RNA

<400>  474
uuggccagga aggggaggat t                                                21


<210>  475
<211>  21
<212>  DNA
<213>  artificial sequence

<220>
<223>  Small Activating RNA

<400>  475
auccuccccu uccuggccat t                                                21


<210>  476
<211>  21
<212>  DNA
<213>  artificial sequence

<220>
<223>  Small Activating RNA

<400>  476
uggccaggaa ggggaggaut t                                                21


<210>  477
<211>  21
<212>  DNA
<213>  artificial sequence

<220>
<223>  Small Activating RNA

<400>  477
ucaaauccuc cccuuccugt t                                                21


<210>  478
<211>  21
<212>  DNA
<213>  artificial sequence

<220>
<223>  Small Activating RNA

<400>  478

caggaagggg aggauuugat t                                                21

<210>  479
<211>  21
<212>  DNA
<213>  artificial sequence

<220>
<223>  Small Activating RNA

<400>  479
gucaaauccu ccccuuccut t                                                21

<210>  480
<211>  21
<212>  DNA
<213>  artificial sequence

<220>
<223>  Small Activating RNA

<400>  480
aggaagggga ggauuugact t                                                21

<210>  481
<211>  21
<212>  DNA
<213>  artificial sequence

<220>
<223>  Small Activating RNA

<400>  481
cgucaaaucc uccccuucct t                                                21

<210>  482
<211>  21
<212>  DNA
<213>  artificial sequence

<220>
<223>  Small Activating RNA

<400>  482
ggaaggggag gauuugacgt t                                                21

<210>  483
<211>  21
<212>  DNA
<213>  artificial sequence

<220>
<223>  Small Activating RNA

<400>  483
ucgucaaauc cuccccuuct t                                                21

<210>  484
<211>  21
<212>  DNA
<213>  artificial sequence

<220>
<223>  Small Activating RNA

<400>  484
gaaggggagg auuugacgat t                                                21


<210>  485
<211>  21
<212>  DNA
<213>  artificial sequence

<220>
<223>  Small Activating RNA

<400>  485
cucgucaaau ccuccccuut t                                                21


<210>  486
<211>  21
<212>  DNA
<213>  artificial sequence

<220>
<223>  Small Activating RNA

<400>  486
aaggggagga uuugacgagt t                                                21


<210>  487
<211>  21
<212>  DNA
<213>  artificial sequence

<220>
<223>  Small Activating RNA

<400>  487
acucgucaaa uccuccccut t                                                21


<210>  488
<211>  21
<212>  DNA
<213>  artificial sequence

<220>
<223>  Small Activating RNA

<400>  488
aggggaggau uugacgagut t                                                21


<210>  489
<211>  21
<212>  DNA

<213>  artificial sequence

<220>
<223>  Small Activating RNA

<400>  489
ucacucguca aauccuccct t                                                21


<210>  490
<211>  21
<212>  DNA
<213>  artificial sequence

<220>
<223>  Small Activating RNA

<400>  490
gggaggauuu gacgagugat t                                                21


<210>  491
<211>  21
<212>  DNA
<213>  artificial sequence

<220>
<223>  Small Activating RNA

<400>  491
cucacucguc aaauccucct t                                                21


<210>  492
<211>  21
<212>  DNA
<213>  artificial sequence

<220>
<223>  Small Activating RNA

<400>  492
ggaggauuug acgagugagt t                                                21


<210>  493
<211>  21
<212>  DNA
<213>  artificial sequence

<220>
<223>  Small Activating RNA

<400>  493
acucacucgu caaauccuct t                                                21


<210>  494
<211>  21
<212>  DNA
<213>  artificial sequence

<220>

<223>  Small Activating RNA

<400>  494
gaggauuuga cgagugagut t                                                21


<210>  495
<211>  21
<212>  DNA
<213>  artificial sequence

<220>
<223>  Small Activating RNA

<400>  495
caacucacuc gucaaaucct t                                                21


<210>  496
<211>  21
<212>  DNA
<213>  artificial sequence

<220>
<223>  Small Activating RNA

<400>  496
ggauuugacg agugaguugt t                                                21


<210>  497
<211>  21
<212>  DNA
<213>  artificial sequence

<220>
<223>  Small Activating RNA

<400>  497
acaacucacu cgucaaauct t                                                21


<210>  498
<211>  21
<212>  DNA
<213>  artificial sequence

<220>
<223>  Small Activating RNA

<400>  498
gauuugacga gugaguugut t                                                21


<210>  499
<211>  21
<212>  DNA
<213>  artificial sequence

<220>
<223>  Small Activating RNA

<400>  499

gacaacucac ucgucaaaut t                                                21

<210>  500
<211>  21
<212>  DNA
<213>  artificial sequence

<220>
<223>  Small Activating RNA

<400>  500
auuugacgag ugaguuguct t                                                21

<210>  501
<211>  21
<212>  DNA
<213>  artificial sequence

<220>
<223>  Small Activating RNA

<400>  501
agacaacuca cucgucaaat t                                                21

<210>  502
<211>  21
<212>  DNA
<213>  artificial sequence

<220>
<223>  Small Activating RNA

<400>  502
uuugacgagu gaguugucut t                                                21

<210>  503
<211>  21
<212>  DNA
<213>  artificial sequence

<220>
<223>  Small Activating RNA

<400>  503
cagacaacuc acucgucaat t                                                21

<210>  504
<211>  21
<212>  DNA
<213>  artificial sequence

<220>
<223>  Small Activating RNA

<400>  504
uugacgagug aguugucugt t                                                21

<210>  505
<211>  21
<212>  DNA
<213>  artificial sequence

<220>
<223>  Small Activating RNA

<400>  505
acagacaacu cacucgucat t                                                21


<210>  506
<211>  21
<212>  DNA
<213>  artificial sequence

<220>
<223>  Small Activating RNA

<400>  506
ugacgaguga guugucugut t                                                21


<210>  507
<211>  21
<212>  DNA
<213>  artificial sequence

<220>
<223>  Small Activating RNA

<400>  507
gacagacaac ucacucguct t                                                21


<210>  508
<211>  21
<212>  DNA
<213>  artificial sequence

<220>
<223>  Small Activating RNA

<400>  508
gacgagugag uugucuguct t                                                21


<210>  509
<211>  21
<212>  DNA
<213>  artificial sequence

<220>
<223>  Small Activating RNA

<400>  509
agacagacaa cucacucgut t                                                21


<210>  510
<211>  21
<212>  DNA

<213>  artificial sequence

<220>
<223>  Small Activating RNA

<400>  510
acgagugagu ugucugucut t                                                21


<210>  511
<211>  21
<212>  DNA
<213>  artificial sequence

<220>
<223>  Small Activating RNA

<400>  511
gagacagaca acucacucgt t                                                21


<210>  512
<211>  21
<212>  DNA
<213>  artificial sequence

<220>
<223>  Small Activating RNA

<400>  512
cgagugaguu gucugucuct t                                                21


<210>  513
<211>  21
<212>  DNA
<213>  artificial sequence

<220>
<223>  Small Activating RNA

<400>  513
ggagacagac aacucacuct t                                                21


<210>  514
<211>  21
<212>  DNA
<213>  artificial sequence

<220>
<223>  Small Activating RNA

<400>  514
gagugaguug ucugucucct t                                                21


<210>  515
<211>  21
<212>  DNA
<213>  artificial sequence

<220>

<223>  Small Activating RNA

<400>  515
aggagacaga caacucacut t                                                21


<210>  516
<211>  21
<212>  DNA
<213>  artificial sequence

<220>
<223>  Small Activating RNA

<400>  516
agugaguugu cugucuccut t                                                21


<210>  517
<211>  21
<212>  DNA
<213>  artificial sequence

<220>
<223>  Small Activating RNA

<400>  517
caggagacag acaacucact t                                                21


<210>  518
<211>  21
<212>  DNA
<213>  artificial sequence

<220>
<223>  Small Activating RNA

<400>  518
gugaguuguc ugucuccugt t                                                21


<210>  519
<211>  21
<212>  DNA
<213>  artificial sequence

<220>
<223>  Small Activating RNA

<400>  519
ucaggagaca gacaacucat t                                                21


<210>  520
<211>  21
<212>  DNA
<213>  artificial sequence

<220>
<223>  Small Activating RNA

<400>  520

ugaguugucu gucuccugat t                                                21

<210>  521
<211>  21
<212>  DNA
<213>  artificial sequence

<220>
<223>  Small Activating RNA

<400>  521
uucaggagac agacaacuct t                                                21

<210>  522
<211>  21
<212>  DNA
<213>  artificial sequence

<220>
<223>  Small Activating RNA

<400>  522
gaguugucug ucuccugaat t                                                21

<210>  523
<211>  21
<212>  DNA
<213>  artificial sequence

<220>
<223>  Small Activating RNA

<400>  523
auucaggaga cagacaacut t                                                21

<210>  524
<211>  21
<212>  DNA
<213>  artificial sequence

<220>
<223>  Small Activating RNA

<400>  524
aguugucugu cuccugaaut t                                                21

<210>  525
<211>  21
<212>  DNA
<213>  artificial sequence

<220>
<223>  Small Activating RNA

<400>  525
uauucaggag acagacaact t                                                21

<210>  526
<211>  21
<212>  DNA
<213>  artificial sequence

<220>
<223>  Small Activating RNA

<400>  526
guugucuguc uccugaauat t                                                21


<210>  527
<211>  21
<212>  DNA
<213>  artificial sequence

<220>
<223>  Small Activating RNA

<400>  527
guauucagga gacagacaat t                                                21


<210>  528
<211>  21
<212>  DNA
<213>  artificial sequence

<220>
<223>  Small Activating RNA

<400>  528
uugucugucu ccugaauact t                                                21


<210>  529
<211>  21
<212>  DNA
<213>  artificial sequence

<220>
<223>  Small Activating RNA

<400>  529
aguauucagg agacagacat t                                                21


<210>  530
<211>  21
<212>  DNA
<213>  artificial sequence

<220>
<223>  Small Activating RNA

<400>  530
ugucugucuc cugaauacut t                                                21


<210>  531
<211>  21
<212>  DNA

<213>  artificial sequence

<220>
<223>  Small Activating RNA

<400>  531
gaguauucag gagacagact t                                                21


<210>  532
<211>  21
<212>  DNA
<213>  artificial sequence

<220>
<223>  Small Activating RNA

<400>  532
gucugucucc ugaauacuct t                                                21


<210>  533
<211>  21
<212>  DNA
<213>  artificial sequence

<220>
<223>  Small Activating RNA

<400>  533
ggaguauuca ggagacagat t                                                21


<210>  534
<211>  21
<212>  DNA
<213>  artificial sequence

<220>
<223>  Small Activating RNA

<400>  534
ucugucuccu gaauacucct t                                                21


<210>  535
<211>  21
<212>  DNA
<213>  artificial sequence

<220>
<223>  Small Activating RNA

<400>  535
gggaguauuc aggagacagt t                                                21


<210>  536
<211>  21
<212>  DNA
<213>  artificial sequence

<220>

<223>  Small Activating RNA

<400>  536
   cugucuccug aauacuccct t                                                21


<210>  537
<211>  21
<212>  DNA
<213>  artificial sequence

<220>
<223>  Small Activating RNA

<400>  537
ggggaguauu caggagacat t                                                 21


<210>  538
<211>  21
<212>  DNA
<213>  artificial sequence

<220>
<223>  Small Activating RNA

<400>  538
ugucuccuga auacucccct t                                                 21


<210>  539
<211>  21
<212>  DNA
<213>  artificial sequence

<220>
<223>  Small Activating RNA

<400>  539
uggggaguau ucaggagact t                                                 21


<210>  540
<211>  21
<212>  DNA
<213>  artificial sequence

<220>
<223>  Small Activating RNA

<400>  540
gucuccugaa uacuccccat t                                                 21


<210>  541
<211>  21
<212>  DNA
<213>  artificial sequence

<220>
<223>  Small Activating RNA

<400>  541
   guggggagua uucaggagat t                                              21

<210>  542
<211>  21
<212>  DNA
<213>  artificial sequence

<220>
<223>  Small Activating RNA

<400>  542
ucuccugaau acuccccact t                                               21

<210>  543
<211>  21
<212>  DNA
<213>  artificial sequence

<220>
<223>  Small Activating RNA

<400>  543
uguggggagu auucaggagt t                                               21

<210>  544
<211>  21
<212>  DNA
<213>  artificial sequence

<220>
<223>  Small Activating RNA

<400>  544
cuccugaaua cuccccacat t                                               21

<210>  545
<211>  21
<212>  DNA
<213>  artificial sequence

<220>
<223>  Small Activating RNA

<400>  545
   auguggggag uauucaggat t                                              21

<210>  546
<211>  21
<212>  DNA
<213>  artificial sequence

<220>
<223>  Small Activating RNA

<400>  546
uccugaauac uccccacaut t                                               21

<210>  547
<211>  21
<212>  DNA
<213>  artificial sequence

<220>
<223>  Small Activating RNA

<400>  547
uaugugggga guauucaggt t                                                21


<210>  548
<211>  21
<212>  DNA
<213>  artificial sequence

<220>
<223>  Small Activating RNA

<400>  548
ccugaauacu ccccacauat t                                                21


<210>  549
<211>  21
<212>  DNA
<213>  artificial sequence

<220>
<223>  Small Activating RNA

<400>  549
cuaugugggg aguauucagt t                                                21


<210>  550
<211>  21
<212>  DNA
<213>  artificial sequence

<220>
<223>  Small Activating RNA

<400>  550
cugaauacuc cccacauagt t                                                21


<210>  551
<211>  21
<212>  DNA
<213>  artificial sequence

<220>
<223>  Small Activating RNA

<400>  551
gcuaugugggg gaguauucat t                                                21


<210>  552
<211>  21
<212>  DNA

<213>  artificial sequence

<220>
<223>  Small Activating RNA

<400>  552
ugaauacucc ccacauagct t                                                21


<210>  553
<211>  21
<212>  DNA
<213>  artificial sequence

<220>
<223>  Small Activating RNA

<400>  553
ggcuaugugg ggaguauuct t                                                21


<210>  554
<211>  21
<212>  DNA
<213>  artificial sequence

<220>
<223>  Small Activating RNA

<400>  554
gaauacuccc cacauagcct t                                                21


<210>  555
<211>  21
<212>  DNA
<213>  artificial sequence

<220>
<223>  Small Activating RNA

<400>  555
gggcuaugug gggaguauut t                                                21


<210>  556
<211>  21
<212>  DNA
<213>  artificial sequence

<220>
<223>  Small Activating RNA

<400>  556
aauacucccc acauagccct t                                                21


<210>  557
<211>  19
<212>  DNA
<213>  artificial sequence

<220>

<223>  Small Activating RNA

<400>  557
ccuccugauc uuuucagcc                                                    19


<210>  558
<211>  21
<212>  DNA
<213>  artificial sequence

<220>
<223>  Small Activating RNA

<400>  558
agcugaaaag aucaggagga u                                                 21


<210>  559
<211>  19
<212>  DNA
<213>  artificial sequence

<220>
<223>  Small Activating RNA

<400>  559
ucugggagag gugaccuaa                                                    19


<210>  560
<211>  21
<212>  DNA
<213>  artificial sequence

<220>
<223>  Small Activating RNA

<400>  560
cuaggucacc ucucccagaa g                                                 21


<210>  561
<211>  19
<212>  DNA
<213>  artificial sequence

<220>
<223>  Small Activating RNA

<400>  561
cuaggucacc ucucccagc                                                    19


<210>  562
<211>  19
<212>  DNA
<213>  artificial sequence

<220>
<223>  Small Activating RNA

<400>  562

cccggaagca ugugacaac                                                    19

<210>  563
<211>  21
<212>  DNA
<213>  artificial sequence

<220>
<223>  Small Activating RNA

<400>  563
auugucacau gcuuccggga a                                                  21

<210>  564
<211>  21
<212>  DNA
<213>  artificial sequence

<220>
<223>  Small Activating RNA

<400>  564
gaacccaggg agccgacaat t                                                  21

<210>  565
<211>  21
<212>  DNA
<213>  artificial sequence

<220>
<223>  Small Activating RNA

<400>  565
uugucggcuc ccuggguuct t                                                  21

<210>  566
<211>  19
<212>  DNA
<213>  artificial sequence

<220>
<223>  Small Activating RNA

<400>  566
gaacccaggg agccgacac                                                    19

<210>  567
<211>  21
<212>  DNA
<213>  artificial sequence

<220>
<223>  Small Activating RNA

<400>  567
uugucggcuc ccuggguucu u                                                  21

<210>  568
<211>  21
<212>  DNA
<213>  artificial sequence

<220>
<223>  Small Activating RNA

<400>  568
gacgguccug aagagcuaat t                                                21


<210>  569
<211>  21
<212>  DNA
<213>  artificial sequence

<220>
<223>  Small Activating RNA

<400>  569
uuagcucuuc aggaccguct t                                                21


<210>  570
<211>  19
<212>  DNA
<213>  artificial sequence

<220>
<223>  Small Activating RNA

<400>  570
gacgguccug aagagcuac                                                   19


<210>  571
<211>  21
<212>  DNA
<213>  artificial sequence

<220>
<223>  Small Activating RNA

<400>  571
uuagcucuuc aggaccguca g                                                21


<210>  572
<211>  19
<212>  DNA
<213>  artificial sequence

<220>
<223>  Small Activating RNA

<400>  572
gacgguccug aagagcuag                                                   19


<210>  573
<211>  19
<212>  DNA

<213>  artificial sequence

<220>
<223>  Small Activating RNA

<400>  573
gacgguccug aagagcuau                                                    19


<210>  574
<211>  21
<212>  DNA
<213>  artificial sequence

<220>
<223>  Small Activating RNA

<400>  574
gcaacuccag ucccaaauat t                                                 21


<210>  575
<211>  21
<212>  DNA
<213>  artificial sequence

<220>
<223>  Small Activating RNA

<400>  575
uauuugggac uggaguugct t                                                 21


<210>  576
<211>  19
<212>  DNA
<213>  artificial sequence

<220>
<223>  Small Activating RNA

<400>  576
gcaacuccag ucccaaauc                                                    19


<210>  577
<211>  21
<212>  DNA
<213>  artificial sequence

<220>
<223>  Small Activating RNA

<400>  577
uauuugggac uggaguugcu u                                                 21


<210>  578
<211>  20
<212>  DNA
<213>  artificial sequence

<220>

<223>  Synthesized primer for PCR

<400>  578
ttcccatctc aaggcacacc                                                    20


<210>  579
<211>  20
<212>  DNA
<213>  artificial sequence

<220>
<223>  Synthesized primer for PCR

<400>  579
gcgaatttcc atccacagcc                                                    20


<210>  580
<211>  20
<212>  DNA
<213>  artificial sequence

<220>
<223>  Synthesized primer for PCR

<400>  580
aggcttcccc aaacccctaa                                                    20


<210>  581
<211>  20
<212>  DNA
<213>  artificial sequence

<220>
<223>  Synthesized primer for PCR

<400>  581
tccgtgagct tgaggttctt                                                    20


<210>  582
<211>  20
<212>  DNA
<213>  artificial sequence

<220>
<223>  Synthesized primer for PCR

<400>  582
ctttctgctg tcttgggtgc                                                    20


<210>  583
<211>  21
<212>  DNA
<213>  artificial sequence

<220>
<223>  Synthesized primer for PCR

<400>  583

ttcgtgatga ttctgccctc c 21